# EUROPEAN PATENT SPECIFICATION

(11) **EP 4 194 540 B1**
(45) Date of publication and mention of the grant of the patent: **02.12.2026**
(21) Application number: 21858304.5
(22) Date of filing: 17.08.2021
(51) Int. Cl.: C12M 1/34, C12Q 1/04, G01N 33/543, G01N 29/02

(54) **VIRUS TEST DEVICE, VIRUS TEST SYSTEM, VIRUS TEST METHOD, AND VIRUS TEST PROGRAM**
VIRUSTESTVORRICHTUNG, VIRUSTESTSYSTEM, VIRUSTESTVERFAHREN UND VIRUSTESTPROGRAMM
DISPOSITIF DE TEST DE VIRUS, SYSTÈME DE TEST DE VIRUS, PROCÉDÉ DE TEST DE VIRUS ET PROGRAMME DE TEST DE VIRUS

(30) Priority: 17.08.2020 JP 2020137683
(43) Date of publication of application: 14.06.2023
(73) Proprietor: Ball Wave Inc., Sendai-shi, Miyagi 980-8579 (JP)
(72) Inventor: YAMANAKA, Kazushi, Sendai-shi, Miyagi 980-8579 (JP); TAKEDA, Nobuo, Sendai-shi, Miyagi 980-8579 (JP); AKAO, Shingo, Sendai-shi, Miyagi 980-8579 (JP); TSUKAHARA, Yusuke, Sendai-shi, Miyagi 980-8579 (JP); OIZUMI, Toru, Sendai-shi, Miyagi 980-8579 (JP); IWAYA, Takamitsu, Sendai-shi, Miyagi 980-8579 (JP)
(74) Representative: Hoffmann Eitle
(86) International application number: PCT/JP2021/030045
(87) International publication number: WO 2022/039160

(56) References cited:
- WO-A1-2009/037977
- WO-A1-2019/014049
- WO-A1-2019/030360
- WO-A1-2019/151362
- JP-A- 2002 525 578
- JP-A- 2007 225 546
- JP-A- 2007 525 670
- JP-A- 2019 532 313
- US-A1- 2010 170 345
- US-B1- 6 321 588
- LUC GERVAIS ET AL: "Microfluidic Chips for Point-of-Care Immunodiagnostics", ADVANCED MATERIALS, VCH PUBLISHERS, DE, vol. 23, no. 24, 13 May 2011 (2011-05-13), pages H151 - H176, XP071810818, ISSN: 0935-9648, DOI: 10.1002/ADMA.201100464

## Description

### [Technical Field]

The present invention relates to a virus test device, a virus test system using the virus test device, a virus test method using the virus test system, and a virus test program which controls an operation of the virus test system as computer system. Specifically, the present invention pertains to the virus test device, the virus test system, the virus test method, and the virus test program which facilitate executions of in-situ monitoring.

### [Background Art]

Seven serotypes of human pathogenic coronaviruses have already known as illustrated in table 1, after a pandemic of Old East Asiatic flu (Russian flu) which is reported one million people have been killed from 1889 to 1891 in the world. In addition to the human coronaviruses illustrated in table. 1, many species of coronavirus, such as TGEV, PEDV and HEV which infect pig, FIPV and FECoV which infect cat, CCoV which infects dog, MHV which infects mouse, and IBV which infects chicken, are identified.

**[Table. 1]**

| | Human Coronavirus Serotypes | year of identification or prevalence |
|---|---|---|
| 1 | HCoV-OC43 | 1889 (re-identified in1962) |
| 2 | HCoV-229E | 1966 |
| 3 | SARS-CoV | 2002 |
| 4 | HCoV-NL63 | 2004 |
| 5 | HCoV-HKU1 | 2005 |
| 6 | MERS-CoV | 2012 |
| 7 | SARS-CoV-2 | 2019 |

On the other hand, in Japan, the number of deaths attributed to influenza virus were 12,000 people every year as an average from 1952 to 2009, and the average number of deaths associated with influenza virus were 25,000 people every year before 1939. Also, according to an official announcement of Ministry of Health, Labor and Welfare in January 2019, in a specific week, the number of infected persons with influenza virus were over 300,000 people a day. Although it is predicted that the battles between human and the viral infections continue to be repeated by different patterns, Dr. Omi Shigeru, Chairman of the New Coronavirus Infectious Diseases Control Subcommittee, said on July 6, 2020, "It is a large proposition how we make socioeconomic activities and infection preventive measures compatible." This large proposition will be solved by a development of means that the virus concentration in the air is measured at once in the natural or original position *--in-situ--,* or that persons who have tested positive for SARS-CoV-2 virus-infection are identified in situ. That is, the infection preventive measures compatible with the socioeconomic activities is realized, if suitable ventilation and disinfection are performed, and the in-situ screening process, which executes respiration monitoring at an entrance etc., and identifies Positively-Tested-Persons in real time, can be realized, with aid of in-situ measurements of the virus concentrations in airs sucked down from mass transportation systems, event sites and so on. However, in 2021, according to the current official decision of the Japanese administration, it is necessary to conduct an additional authorized virus test, such as a polymerase chain reaction (PCR) test or an antigen test, after the identification process of Positively-Tested-Person with in-situ measurement. In any way, an in-situ measurement equipment which can measure a concentration of SARS-CoV-2 virus simply in a real-time and can identify Positively-Tested-Person is not developed at the present time.

Until now, various biosensors using an antibody and aptamer (hereafter, referred to as "antibody", comprehensively), which binds specifically to viruses, such as to proteins and allergens in the viruses, are researched and developed. However, the earlier biosensors do not have the quick responses or test speeds which facilitate the in-situ monitoring. The speed of antigen-antibody reaction (AAR) used by biosensors is high by itself, because the AAR is based on a mechanism which recognizes mutually referenced molecules in a buffer solution, which imitates a water or a bio-environment. The recognizing mechanism is ascribable to fluctuations of molecular chains, which constitute the virus and antibody molecules. However, in the AAR in a liquid, the virus as the reactant needs to be supplied to the antibody by a diffusion process owing to a concentration gradient. The diffusion process in the liquid is based on random walks. Because the speeds of random walks are slow generally, the response time of the AAR in the liquid is several minutes or more (see Non-Patent literature (NPTL) 1). As a result, a problem of increased infection risk occurs, since a quick escape from a dangerous air environment cannot be achieved, and a management of air cleaning by a powerful ventilation tool will become too late. For executing the quick escape from the dangerous air environment and the quick management of air cleaning, a high-speed measurement at an order of seconds is required. That is, the earlier biosensors are not suitable for the in-situ monitoring, because the earlier biosensors always use liquids as reaction fields (see NPTL 1).

The earlier liquid-based biosensor needs a sensor cell (detection cell) in which a detection portion of the sensor is dipped in the liquid, a liquid storage tank which amasses a cell cleaning agent, a waste liquid storage tank which amasses a waste liquid after cleaning, and means for supplying the liquid, such as a flow channel and a pump. So, in the earlier biosensor, since the mechanism and structure are complicated and reductions in size and weight have limitations, it is difficult to develop a transportable equipment which is applicable to the in-situ measurement in transport facilities and theaters, etc. Equipment of earlier PCR-based diagnostics and the like for infectious diseases require larger size. Therefore, there is a problem that a compact measurement equipment, which facilitates easy and convenient operations of the in-situ monitoring on various practical sites, and can substitute the earlier large-sized equipment of PCR-based diagnostics and the like, is not realized.

Even an aerosol collection scheme, which extract the aerosol containing viruses from environmental air, is not suitable to the in-situ monitoring. In the earlier aerosol collection scheme, the viruses in the subject gas are transported into a specific liquid through bedewing process, by cooling of air (see Patent Literature (PTL) 1), or through babbling process in the liquid (see PTL 2), so that a virus structure can be recognized and detected, by using the AAR in the liquid. Then, for obviating the scourge due to the viral pandemic of infectious disease, or for bringing the end of the viral pandemic at earlier phases, the development of the in-situ virus-measurement equipment is an important social issue, and therefore, the in-situ virus-measurement equipment shall have a simple structure and features such that the miniaturization is possible and that the high response speed is realized.

NPL 3 discloses microfluidic chips for point-of-care immunodiagnostics with specific emphasis on corresponding features and motivations such as gaps and opportunities in medical diagnostics, the characteristics of different technologies including details on materials, surface treatment, sample processing, microfluidic elements, receptors, and analytes. In addition, PTL 3 and PTL 4 have been found to disclose related technological background.

### [Citation List]

### [Patent Literature]

[PTL 1] WO 2011/136344 A1
[PTL 2] JP-2011-152109 A
[PTL 3] US 6 321 588 B1
[PTL 4] US 2010/170345 A1

### [Non-Patent literature]

[NPL 1] N. Moll et.al., "Multipurpose Love acoustic wave immunosensor for bacteria, virus or proteins detection", ITBM-RBM, 29 (2008), pp. 155-161
[NPL 2] P. Fabian et.al., "Influenza virus in human exhaled breath: an observational study", PLoS ONE, 2008, Volume 3, p.e2691 (online)
[NPL 3] LUC GERVAIS ET AL: « Microfluidic Chips for Point-of-Care Immunodiagnostics", ADVANCED MATERIALS, VCH PUBLISHERS, DE, vol. 23, no. 24, 13 May 2011 (2011-05-13), pages H151-H176, XP071810818, ISSN: 0935-9648, DOI: 10.1002/ADMA-201100464

### [Summary of Invention]

### [Technical Problem]

To solve the above-described problem, an objective of the present invention is to provide a virus test device, which have a simple structure and a high response speed, facilitating miniaturization and execution of the in-situ monitoring, a virus test system using the virus test device, a virus test method using the virus test system and a virus test program driving and controlling the virus test system as a computer system.

### [Solution to Problem]

To achieve the objective described above, it is provided the subject-matter of the independent claims. Further preferred embodiments of the present invention are defined in the dependent claims.

### [Advantageous Effects of Invention]

According to the present invention, the virus test device, which have the simple structure and the high response speed, facilitating the miniaturization and execution of the in-situ monitoring, can be provided, and furthermore, the virus test system using the virus test device, the virus test method using the virus test device, and the virus test program which controls an operation of the virus test system as computer system can be provided.

### [Brief Description of Drawings]

FIG. 1 is a schematic diagram illustrating a rough structure of a virus test system pertaining to a fundamental embodiment addressing to a base concept of a technical idea of the present invention;
FIG. 2 is a schematic diagram illustrating a rough structure of a sensor of a virus test device pertaining to the fundamental embodiment;
FIG. 3 is a schematic diagram illustrating a rough structure of a sensor unit of the virus test device pertaining to the fundamental embodiment;
FIG. 4 is a bird's-eye view illustrating a rough structure of a handle-attached susceptor in the virus test device pertaining to the fundamental embodiment;
FIG. 5 is a schematic local sectional view illustrating the rough structure of the handle-attached susceptor in the virus test device pertaining to the fundamental embodiment taken from the left side;
FIG. 6 is a schematic local sectional view illustrating the rough structure of the handle-attached susceptor in the virus test device pertaining to the fundamental embodiment taken from a front side;
FIG. 7 is a schematic diagram illustrating a concrete constitution of the virus test device pertaining to a first modification of the fundamental embodiment;
FIG. 8 is a schematic diagram illustrating the other concrete constitution as the virus test device pertaining to a second modification of the fundamental embodiment;
FIG. 9 is a block diagram illustrating a rough structure of a hardware resource implementing a signal processor of the virus test system pertaining to the fundamental embodiment;
FIG. 10A is a schematic diagram illustrating a state of a spike-protein receptor-binding on a surface of the pseudo-receptor film in a phase that a liquid film does not exist on a surface of the pseudo-receptor film of a sensor cell (detection cell);
FIG. 10B is a schematic diagram illustrating another state of a spike-protein receptor-binding on the surface of the pseudo-receptor film in a phase that the pseudo-receptor film is covered by a liquid film at a time of the spike-protein receptor-binding;
FIG. 11A is a schematic diagram illustrating an example that an aerosol-under-test is ejected to the pseudo-receptor film with a high-speed air-flow from a nozzle;
FIG. 11B is a schematic diagram illustrating the other example that the aerosol-under-test is ejected to the pseudo-receptor film with the high-speed air-flow from the nozzle;
FIG. 12 is a flow chart describing an outline of the virus test method pertaining to the fundamental embodiment;
FIG. 13 is a schematic diagram illustrating a rough structure of a detection vessel for the virus test device pertaining to a first derived-embodiment;
FIG. 14 is a schematic diagram illustrating a rough structure of a detection vessel for the virus test device pertaining to the first derived-embodiment;
FIG. 15 is a flow chart describing an outline of the virus test method pertaining to a second derived-embodiment;
FIG. 16 is a schematic diagram illustrating a rough structure of a detection vessel for the virus test device pertaining to the second derived-embodiment;
FIG. 17 is a schematic diagram seeing the detection vessel for the virus test device illustrated in FIG. 16, taken from X-direction in FIG. 16;
FIG. 18A is a schematic diagram illustrating a rough structure of the virus test system pertaining to a third derived-embodiment;
FIG. 18B is a schematic diagram illustrating a rough structure of an improved example of the detection vessel pertaining to the third derived-embodiment;
FIG. 19 is a flow chart illustrating an outline of the virus test method pertaining to the third derived-embodiment;
FIG. 20 is a diagram illustrating a principle that an assay time is shorted in the third derived-embodiment;
FIG. 21 is a schematic diagram illustrating a rough structure of a nozzle of a virus test device pertaining to a fourth derived-embodiment;
FIG. 22A is a schematic diagram illustrating a rough structure of a detection vessel of a virus test device pertaining to a fifth derived-embodiment, in a state when a detector-substrate is removed;
FIG. 22B is a schematic diagram describing a location of the pseudo-receptor film when the detector-substrate is allocated in the inside of the detection vessel illustrated in FIG. 22A;
FIG. 22C is a schematic diagram describing another structure in which a height of an inner wall of a gas-circumventing chamber of the detection vessel illustrated in FIG. 22A set lower;
FIG. 23 is a schematic diagram illustrating a rough structure of a detection vessel of a virus test device pertaining to a sixth derived-embodiment;
FIG. 24 is a block diagram illustrating a rough structure of a hardware resource implementing a signal processor of the virus test device pertaining to the sixth derived-embodiment;
FIG. 25 is a flow chart describing an outline of the virus test method pertaining to the sixth derived-embodiment;
FIG. 26A is a cross-sectional view schematically illustrating a structure of a body of the detection vessel for the virus test device pertaining to the sixth derived-embodiment;
FIG. 26B is a cross-sectional view illustrating a structure of a susceptor which is inserted in a lower portion of the body of the detection vessel illustrated in FIG. 26A, and a schematic diagram describing an example of a mechanism for exchanging a sensor cell (detection cell) by detaching a portion of a susceptor, which is assigned as a part of a revolver;
FIG. 27 is a schematic diagram illustrating a rough structure of a sextuple-ball rotational-motion scheme (revolver), which automatically arranges selectively a specific sensor cell among sextuple sensor cells into the detection vessel, according to the virus test device pertaining to the sixth derived-embodiment;
FIG. 28 is a schematic diagram illustrating a rough structure of a virus test device pertaining to a seventh derived-embodiment of the present invention;
FIG. 29 is a schematic diagram illustrating a detail of an aerosol generator of the virus test device as one part of the virus test system illustrated in FIG. 28, and a peripheral structure of the aerosol generator;
FIG. 30 is a flow chart describing an outline of each step of the virus test method pertaining to the seventh derived-embodiment;
FIG. 31 is a diagram illustrating a response-curve of a virus sensor of the virus test device pertaining to the seventh derived-embodiment;
FIG. 32 is a diagram illustrating a calibration curve using the virus test method pertaining to the seventh derived-embodiment;
FIG. 33 is a flow chart describing an outline of each step when the sensitivity is calibrated in the virus test method pertaining to the seventh derived-embodiment;
FIG. 34 is a schematic diagram illustrating a detail of a detection vessel for the virus test device as one part of the virus test system pertaining to the eighth derived-embodiment, and a peripheral structure of the detection vessel;
FIG. 35A is a schematic diagram indicating states of target viruses and non-specific adsorption (NSA) materials at a timing of ejection of air-under-test (AUT) in detail;
FIG. 35B is a schematic diagram indicating states of target viruses and the NSA materials at a timing of ejection of purge gas in detail;
FIG. 36 is a schematic diagram illustrating a rough structure of a virus test device pertaining to another embodiment; and
FIG. 37 is a schematic diagram further illustrating a rough structure of a virus test device pertaining to still another embodiment.

### [Description of Embodiments]

Hereinafter, in a top section labeled as a "fundamental embodiment of the present invention", a brief overview of a basic technical idea of the present invention will be described, with reference to the drawings. Next, based upon the technical idea of the fundamental embodiment, first to eighth derived-embodiments of the present invention, which shall boost the basic technical idea, will be described with reference to the drawings. The same or similar reference numerals are assigned to the same or similar parts and elements throughout the description of the following drawings. However, it is to be noted that the drawings are schematic, and a relationship between the thickness and the plan view dimensions, a ratio of the thicknesses of the elements, etc., differs from an actual relationship and an actual ratio, etc., in the real products. Therefore, a specific thickness, a specific size, etc., shall be judged by considering the following explanation. Also, in the following drawings, it is to be naturally noted that the relationship and ratio between sizes of the elements may be different from each other.

Furthermore, the fundamental embodiment and the first to eighth derived-embodiments described below are typical examples representing structures and methods for implementing the technical idea of the present invention, and the technical idea of the present invention is not limited to the materials, shapes, structures, arrangements, and the like of configuration parts, which are recited in the following. The technical idea of the present invention can add various changes to the technical scopes prescribed by claims. Also, the "right and left" directions and the "up and down" directions recited in the following description are merely assigned to specific directions for convenience, so the technical idea of the present invention is not limited to the assigned directions in the following.

### (FUNDAMENTAL EMBODIMENT)

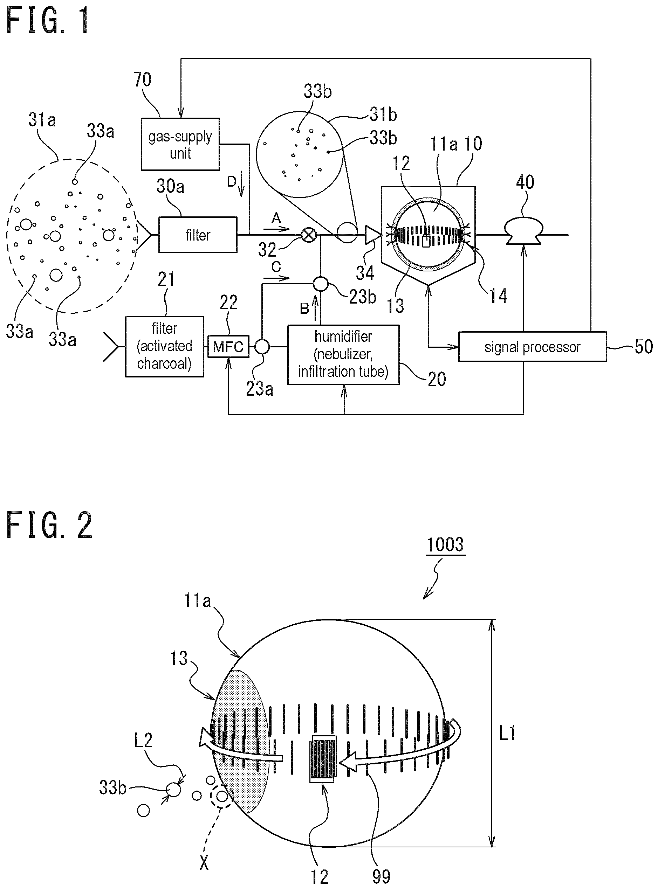

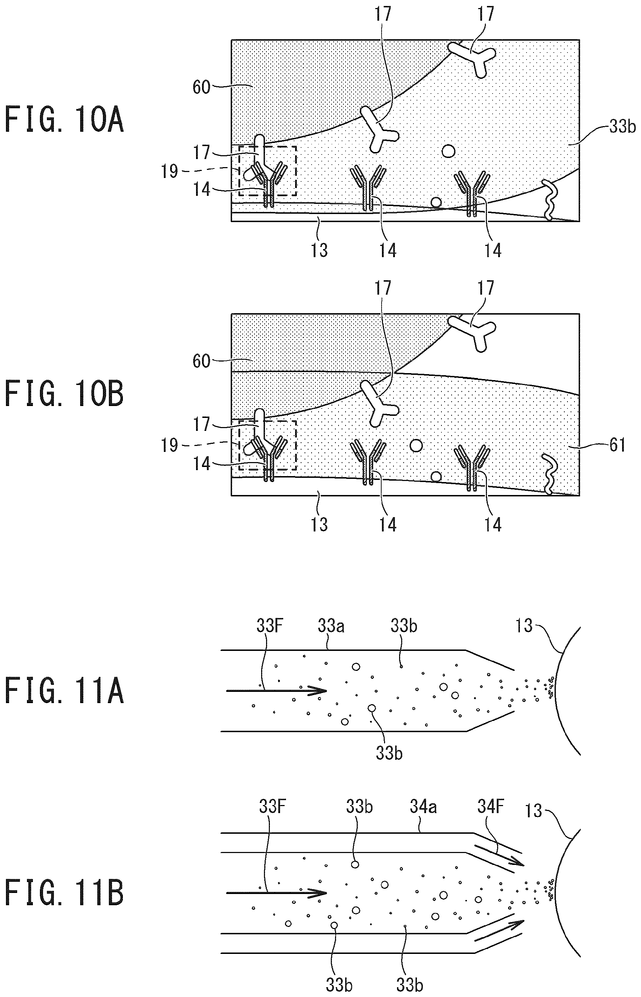

At first, the basic concept and the basic thinking schemes of the technical idea of the present invention will be described below. As illustrated in FIG. 1, the virus test system pertaining to the fundamental embodiment of the present invention encompasses a detector-substrate 11a, which laminates a pseudo-receptor film 13 at least on one part in a surface of the detector-substrate 11a, and a virus introducing-tube-A, which ingests and condenses an air-under-test (AUT) 31a. The virus introducing-tube-A ejects the AUT 31a to a surface of the pseudo-receptor film 13. The virus test system of the fundamental embodiment invention further encompasses a signal processor 50, which executes a difference-integral detection for judging the existence of a target virus bind to a surface of the pseudo-receptor film 13 by a spike-protein receptor-binding, under the examination functions of the detector-substrate 11a and the pseudo-receptor film 13. As illustrated in FIGs. 10A and 10B schematically, each of the pseudo-receptors 14 has a structure mimicking a protein called as "immunoglobulin", which has been produced by B-cell, for example. And, each of the pseudo-receptors 14 recognizes a predetermined virus, and binds specifically to protrusions of spike-glycoproteins of the predetermined virus, as host-cell receptors (receptors) at a surface of biological cell.

Focusing to the generation of the spike-protein receptor-bindings 19 illustrated in FIGs. 10A and 10B, in the virus test system pertaining to the fundamental embodiment, the existence of target viruses is detected by the binding of the pseudo-receptors 14 to the receptor-binding domain (RBD) of the spike-glycoprotein 17 in the target viruses 60, which is the similar reaction of generating "the spike-glycoprotein receptor" by the binding between the RBD of the spike-glycoprotein in the virus and the host-cell receptor at a surface of the biological cell. By the way, FIG. 10B is a diagram describing a model in which the RBD of the spike-glycoprotein 17 is bound to the pseudo-receptors 14 in a liquidus state such that a surface of the pseudo-receptor film 13 is covered by a liquid film 61. In contrast with FIG. 10B, FIG. 10A is a diagram describing a model in which the RBD of the spike-glycoprotein 17 in the target viruses 60 is bound to the pseudo-receptors 14, by an action with the liquid included in the aerosol 33b under test, in a dry state such that the pseudo-receptor film 13 is not covered by the liquid film 61. And then, in the instant specification, the reaction in which the spike-protein receptor-binding 19 is provided by the binding between the RBD of the spike-glycoprotein 17 in the target viruses 60 and the pseudo-receptors 14, like the biological cell, is called "specific-binding reaction".

In the virus test system of the fundamental embodiment, a structure mimicking a configuration of the general antibody, such as Immunoglobulin G (IgG) antibody, is considered as a candidate structure of the pseudo-receptors 14, which can be adopted as an examination target. In FIGs. 10A and 10B, a structure of the Y-shaped pseudo-receptors 14 mimicking a configuration of the Y-shaped antibody, which is provided by the binding of a pair of H-chain and L-chain, is described schematically as an example. A subject portion that the host-cell receptor on a surface of the biological cell mainly bind to the virus is a tip (called a "variable region"), at a site where the H-chain and the L-chain facing each other. Therefore, it is enough to prepare only a configuration of the subject portion as the structure of the pseudo-receptors 14 of the virus test device pertaining to the fundamental embodiment. For example, if it is a case where the target viruses 60 exemplary illustrated in FIGs. 10A and 10B is SARS-CoV-2 virus, it is well known that the RBD of the spike-glycoprotein 17 binds to an angiotensin-converting enzyme 2 (ACE 2) receptor on a surface of human cells with a high affinity. If it is assumed that the target viruses 60 is SARS-CoV-2 virus, a mimicked structure of the Variable domain of Heavy chain of Heavy chain (VHH) antibody, which is picked up only the variable region, among antibodies constituted only by the H-chain, is acceptable as the pseudo-receptors 14. The VHH-antibody is the variable region which is a piece of a characteristic antibody as a heavy-chain antibody, possessed by alpacas et. al. Then, VHH-antibody binds to antigens on the virus, etc., with a single-chain only, differed from a normal IgG-antibody.

As illustrated in FIG. 1, the virus test device as a part of the virus test system pertaining to the fundamental embodiment, as necessary, may further encompass a moist-air introducing-tube- B, which combines to a middle bifurcation node of the virus introducing-tube-A, and through which a cleaned moist-air is supplied to the pseudo-receptor film 13 laminated on the detector-substrate 11a through the virus introducing-tube-A. The virus test device may further encompass a dry-air bypass-tube-C, through which a cleaned dry-air is supplied to the pseudo-receptor film 13 laminated on the detector-substrate 11a through the virus introducing-tube-A, and a purge-gas introducing-tube-D, through which a disinfecting gas or a purge gas is supplied to suction pump 40, via the path through the virus introducing-tube-A and the detection vessel 10, and so on, where appropriate.

In general, the viruses are included in airborne droplets, micro-droplets (aerosol), droplet-nuclei, etc., of the AUT 31a. Although an airborne droplet is defined as a particle having a size of five micrometers or more, and a micro-droplet and droplet-nuclei are defined respectively as particles having the sizes of five micrometers or less, generally. However, since the size of aerosol is not clearly defined, the airborne droplets, micro-droplets and droplet-nuclei are collectively called "aerosol" in the instant specification. The AUT 31a of the virus test device pertaining to the fundamental embodiment is the air being the target to be examined, as the target being tested whether the virus exists or not. And, the AUT 31a includes an air which is extracted from a living space, a public building or a public conveyance, and a breath retrieved from the target person in a respiratory gas analysis or an expiratory test. The "cleaned moist-air" is an air which does not include the impurity particles and the virus, and an air which includes a water being a base of a reaction field of the specific-binding reaction or a vapor of buffer solution mimicking a bio-environment. Also, the "dry-air" is a desiccated air which does not include impurity particles and the virus.

In the virus test system pertaining to the fundamental embodiment, an interdigital electrode, which is allocated in a ball surface acoustic wave sensor (hereafter, referred to as the "ball SAW sensor") 1003 illustrated in FIG. 2, is assigned as the signal conditioner 12, as an example. Then, the detector-substrate 11a shall correspond to a homogeneous piezoelectric-crystal sphere, such as a crystal ball, in the ball SAW sensor 1003. The ball SAW sensor 1003 embracing the detector-substrate 11a, used as the virus test device of the fundamental embodiment, further includes a signal conditioner 12 and a pseudo-receptor film 13, etc., which are disposed on the detector-substrate 11a, as being clear from FIG. 2. However, the detector-substrate 11a is not limited to the piezoelectric-crystal sphere. That is, the detector-substrate 11a may be any substrate, on which the pseudo-receptor film 13 mountable at least one portion of a surface of the detector-substrate 11a, as long as the substrate is mountable the signal conditioner 12, which can convert physical signals representing the difference of surface states of the pseudo-receptor film 13 to electric signals, when the surface state of the pseudo-receptor film 13 metamorphoses. In the case of the ball SAW sensor 1003 illustrated in FIG. 2, the pseudo-receptor film 13, which is arranged at least on one part of the surface of the piezoelectric-crystal sphere as the detector-substrate 11a, is a sensitive film of a surface acoustic wave (SAW). Under a condition that the pseudo-receptors 14, which binds specifically to the virus, is arranged on the surface of the sensitive film, and the SAW propagates in the pseudo-receptor film 13. That is, the ball SAW sensor 1003 of the virus test device of the fundamental embodiment is, as illustrated in FIG. 2, standardizes on an architecture having the detector-substrate 11a, the signal conditioner 12 and the pseudo-receptor film 13 laminated on the detector-substrate 11a, and the pseudo-receptors 14 provided on the pseudo-receptor film 13. In the ball SAW sensor 1003 exemplified in FIG. 2, the signal conditioner 12, which is disposed in a predetermined area on the surface of the detector-substrate 11a, is an interdigital electrode.

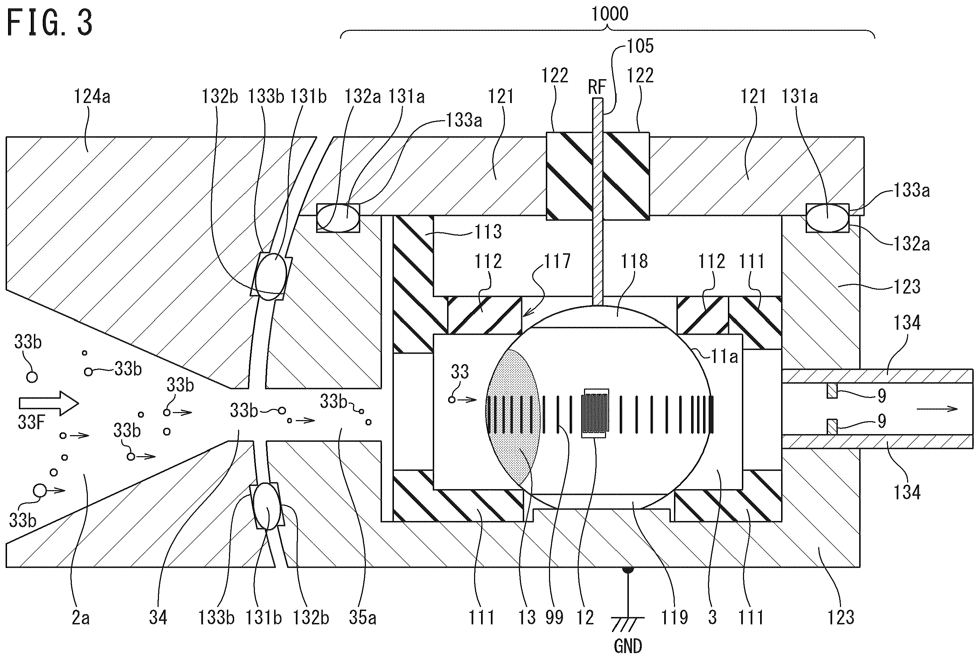

Namely, the signal conditioner 12 converts acoustic signals to electric signals, the acoustic signals represent variances of a mass of the pseudo-receptor film 13, after the pseudo-receptors 14 provided on the pseudo-receptor film 13 bind specifically to the target viruses. The electric signals, which are delivered from the signal conditioner 12 as delay-time responses of the SAW, are processed by the difference-integral detection through the signal processor 50. Here, the difference-integral detection measures an increase of an areal density by weight of the pseudo-receptor film 13, which is ascribable to an amount of the viruses bound to the pseudo-receptors 14. As illustrated in FIG. 1, the detection vessel 10, which retracts the detector-substrate 11a, lodges the ball SAW sensor 1003 in the closed space so that the signal conditioner 12 can feed the electric signals, which are required for the execution of the difference-integral detection. A "detection cell (sensor cell) 1000" in the virus test device of the fundamental embodiment, as illustrated in FIG. 3, encompasses the ball SAW sensor 1003 illustrated in FIG. 2, and the detection vessel 10 which lodges the ball SAW sensor 1003. Also, according to the scheme of the detection cell 1000 illustrated in FIG. 3, the AUT 31a is introduced to the detection vessel 10, such that the high-speed air-flow of the AUT 31a, in which the number of the target viruses is enriched, is ejected to the ball SAW sensor 1003 housed in the detection vessel 10. That is, the detection vessel 10 implements a hermetic space blocked from the atmosphere of outside, configured to convert the acoustic signals, which have information of the variations of the physical states owing to the target viruses existing in the AUT 31a, to the electric signals through the signal conditioner 12.

As illustrated in FIG. 3, the detection vessel 10, which lodges the ball SAW sensor 1003, implement an inlet-canal 35a at one side (the left side in FIG. 3) in the wall of the detection vessel 10, and an exhaust tube 134 at the other side (the right side in FIG. 3) being opposite to the inlet-canal 35a. The eject-side edge of the virus introducing-tube-A is connected to the inlet-canal 35a of the detection vessel 10 from the left side in FIG. 3. The suction pump 40 illustrated in FIG. 1 is connected to the exhaust tube 134 of the detection vessel 10 at the right side in FIG. 3. The detection vessel 10 has a hollow structure, and the ball SAW sensor 1003 is disposed in the inside of the detection vessel 10. The detector-substrate 11 is spherical, and as illustrated in FIG. 2, one partial area or an almost entire are of the surface of detector-substrate 11a is covered by the pseudo-receptor film 13.

The detector-substrate 11a as a three-dimensional substrate, implementing the ball SAW sensor 1003 of the virus test device according to the fundamental embodiment, is provided by a homogeneous spherical material on which a circular looping-belt for propagating the SAW is defined. The signal conditioner 12 encompasses the interdigital electrode which generates a collimated SAW beam. Then, the SAW propagates repeatedly along a circular orbital defined on the piezoelectric ball, while passing through the sensitive film as the pseudo-receptor film 13 provided on the circular orbital. In FIG. 2, positions of the wavefronts 99 of the SAW, implementing the collimated beam, are schematically illustrated by a set of vertical lines along the equator of the detector-substrate 11a. In the schematic configuration exemplified in FIG. 2, the pseudo-receptor film 13 is possible to be laminated on the almost entire surface of the circular looping-belt, which defines the circular orbital on the three-dimensional substrate. Since the pseudo-receptor film 13 has the pseudo-receptors 14 which bind specifically to a particular virus, it is possible to inspect whether the target virus is included in the AUT 31a, by selecting the specific structure of the pseudo-receptors 14.

The following crystal sphere is available as the piezoelectric ball as one partial area of the detector-substrate 11a of the virus test device according to the fundamental embodiment: a rock crystal, LANGASITE (La₃Ga₅SiO₁₄), lithium niobate (LiNbO₃), lithium tantalate (LiTaO₃), piezoelectric ceramic (PZT), bismuth germanium oxide (Bi₁₂GeO₂₀), and the like. A silica (SiOₓ) film, etc., are available as the underlying film of the pseudo-receptor film 13. The interdigital transducer (IDT), which is patterned as comb-shaped arrays of metallic chrome (Cr) electrodes, can be merged on the underlying film, as the signal conditioner 12. In the case of a single-crystalline sphere like a homogeneous piezoelectric ball, the orbital path of the SAW is limited by the specific orbital band having a constant width depending on a kind of a crystal material. The width of the orbital band may increase or decrease depending on an anisotropy of the crystal.

In the case of the ball SAW sensor 1003 of the virus test device according to the fundamental embodiment, a diffraction loss is not generated and a propagation loss is only generated by a material damping, when the SAW travels on a peripheral of the piezoelectric ball as the detector-substrate 11a. The collimated beam passes ultra-multiple times repeatedly through the pseudo-receptor film 13, configured to adsorb only the target viruses included in the AUT 31a. In FIG. 1, the AUT 31a is schematically illustrated by the surrounding broken line. Since the target viruses adsorbed by the pseudo-receptors 14 on the pseudo-receptor film 13 alters propagation characteristics of the SAW, which is measured by the ball SAW sensor 1003, the variations of the propagation characteristic, owing to the target viruses adsorbed on the pseudo-receptor film 13, are integrated at every roundtrip of the collimated SAW beam in the ultra-multiple roundtrips. Therefore, according to the virus test device of the fundamental embodiment, since the target viruses is effectively detected in real time, even if the number of the target viruses which are included in the AUT 31a is small, the precision of the in-situ measurement of the target viruses can be improved.

The aerosols-under-test 33b illustrated by the schematic diagram in FIG. 2 are tiny liquid particles which have been transmitted through the filter 30a among the coarse aerosols 33a, the size L2 of the aerosols 33b depends on functions of the filters 30a. Although not illustrated in FIG. 2, the sizes of the target viruses 60 which are included in the aerosols-under-test 33b will vary from several ten nanometers to several hundred nanometers, normally. On the other hand, in the normal case, it is preferable that the sizes L1 of the detector-substrate 11a are elected from one millimeter to five millimeters, for example three millimeters. However, the most suitable size L1 of the detector-substrate 11a can be determined, depending on the dimensional scale of the virus test device and the frequency of replacements with the detector-substrates 11a, according to the virus test device pertaining to the fundamental embodiment.

Various methods for manufacturing the pseudo-receptor films 13 are known, in each of which the plurality of pseudo-receptors 14 are provided on the surface the detector-substrate 11a, as illustrated in FIGs. 10A and 10B. For example, the methods for physically or chemically conjoin the plurality of pseudo-receptors 14 to carriers, such as in water-insoluble bead states or film-like states, etc., are acceptable. Normal covalent bonds and the like are considered as the chemical bonding, and a van der Waals force and the like are considered as the physical binding. As materials for the carriers, agarose or quartz, etc., may be adopted. For example, aldehyde group or N-hydroxy succinimide group (NHS group), etc. may be fixed on a surface of the carrier, and thereafter, amide bonds with the amino groups in the plurality of pseudo-receptors 14, may be applied to the surface of the carrier. Alternatively, protein-A or protein-G, etc. may be fixed on the surface of the carrier, and thereafter, a material, in which a plurality of pseudo-receptors 14 is affinity bonded to the protein-A or the protein-G, may be applied to the surface of the carrier. The affinity bonds can be reinforced, by adding the crosslinking agent when executing the process of the affinity bonds.

In addition, as one of the other methods for manufacturing the plurality of pseudo-receptors 14, a method of trapping the carrier into a reticular polymeric molecular compound is acceptable. For example, a method of trapping the carrier into nets, which are crosslinked to polyvinyl alcohol (PVA), etc., is acceptable. And, the ball SAW sensor 1003 as illustrated in FIG. 2 is realized by providing the pseudo-receptor film 13, which is implemented by the nets crosslinked to PVA, etc., at least on one partial area around the detector-substrate 11a. Then, the detection cell 10 of the virus test device, according to the fundamental embodiment as illustrated in FIG. 3, is constructed by accommodating the ball SAW sensor 1003 into the detection vessel 10.

Since the pseudo-receptor film 13 encompasses the plurality of pseudo-receptors 14 implemented by organic substance, it is necessary to control the states of the detection cell 1000 and the ball SAW sensor 1003 with great caution. Considering the pseudo-receptors 14 are made of protein, it is necessary to control the states of the detection cells 1000 and the ball SAW sensors 1003 under appropriate temperature conditions, appropriate pH-conditions, and the other appropriate environmental conditions, by which the pseudo-receptors 14 are not denatured. Especially, a kind of the pseudo-receptor 14 must be frozen at about -80 degrees centigrade so as not to be denatured, and another kind of the pseudo-receptor 14 may stably exist, keeping the normal state, in a refrigerated state at about four degrees centigrade, or alternatively, may stably exist at the ordinary temperature. Then, regarding the management and control of the state of the pseudo-receptors 14, the temperature conditions shall be appropriately elected individually, depending on kinds of the pseudo-receptors 14. Also, in the storage management of the states of the detection cell 1000 and the ball SAW sensor 1003, a method of applying the stabilizer on the surface of the pseudo-receptors 14, or a method of soaking the detection cell 1000 and the ball SAW sensor 1003 in a liquid of the stabilizer, etc., may be acceptable. For the storage management of the detection cell 1000 and the ball SAW sensor 1003, generally used materials, such as glycerol, trehalose, and sucrose, may be acceptable as the stabilizer.

For the storage management of the ball SAW sensor 1003, a method of providing a mask on the pseudo-receptors 14 is acceptable, such that normally providing the mask on the pseudo-receptors 14 at a regular unused period, and then, activating the pseudo-receptors 14, by removing the mask from the pseudo-receptors 14 at the use-period of the pseudo-receptors 14. After the use-period, the mask is provided on the pseudo-receptors 14 again, when the pseudo-receptors 14 is not used. For example, the pseudo-receptors 14 may be protected at the regular unused period by the mask of a material like a pseudo-antigen, and the mask of the pseudo-antigen may be removed by an acid, an alkali, a buffer solution, etc., when the use-period of the detection cell 1000 and the ball SAW sensor 1003 comes. After the use-period of the ball SAW sensor 1003 is finished, and the nonuse-period of the pseudo-receptors 14 comes again, it is preferable that the pseudo-receptors 14 is masked again, by the buffer solution, etc., which includes a large quantity of the pseudo-antigen. By the way, the mask is unnecessary if the pseudo-receptors 14 is stable without masking, as a matter of course.

The used detection cell 1000 and the used ball SAW sensor 1003 are reusable, by removing the target viruses bound to the pseudo-receptors 14. When the used detection cell 1000 and the used ball SAW sensor 1003 are reused, the impurities, other than the target viruses bound to the pseudo-receptor film 13, being attached to the pseudo-receptors 14, and furthermore, being attached to the other portions than the pseudo-receptors 14 shall be removed. When reusing the detection cell 1000 and the ball SAW sensor 1003, it is preferable to execute the disinfection treatment of the used detector-substrate 11a, before the used detection cell 1000 and the used ball SAW sensor 1003 are reused. As the disinfection or sterilization scheme, the heating process, the acid process, the alkali process, the high concentration alcohol process, the ultraviolet radiation process and the surfactant process, etc., is acceptable. However, in the disinfection treatment, it is necessary to satisfy the condition that the pseudo-receptors 14 made of protein is not denatured. And, it is preferable that the disinfection treatments shall be executed as necessary, against to the virus introducing-tube-A, the inside of the detection vessel 10 and the suction pump 40, etc., through which there is some possibility that the target viruses will pass. In the disinfection treatments, strong disinfection process and strong cleaning process which approaches to the limit of the withstand capability of the piping system and the detection vessel10 shall be applied.

In the above, although a method of repeating the uses of the used detector-substrates 11a, after the timings that the used detector-substrates 11a are taken out from the detection vessel 10, is described, a scheme for recovering the activity of the pseudo-receptors 14 in a steady state that the detector-substrate 11a is disposed in the detection vessel 10 could be employed. For example, by providing an intra disinfection mechanism in the detection vessel 10, the target viruses which bind to the pseudo-receptors 14 by the spike-protein receptor-binding 19, and the impurities, etc. other than the target viruses, which are attached to the pseudo-receptor film 13 and the other portion of the pseudo-receptor film 13, can be removed. That is, the target viruses bound to the pseudo-receptors 14 and the impurities, etc. can be removed with the intra disinfection mechanism, in an initial stage in the procedure by which the virus test method and the virus test program which will be described below, are executed.

As illustrated in FIG. 1, the virus introducing-tube-A of the virus test device according to the fundamental embodiment encompasses a filter 30a, an on-off valve 32, and a common concentrating-mechanism 34. The filter 30a has a function for removing the aerosols having sizes larger than a predetermined size, for example, four micrometers, among the aerosols having sizes spanning between 0.1 micrometer to 30 micrometers, which are included in the AUT 31a. The filter 30a is provided at an entrance side of the virus introducing-tube-A. The predetermined sizes vary depending on the species of viruses. And, the predetermined sizes also vary whether the transmission and spread of virus depend on aerial infection, aerosols infection, or droplet infection.

The moist-air introducing-tube- B of the virus test device according to the fundamental embodiment is connected to the middle of the virus introducing-tube-A, specifically, is connected to a bifurcation node between the on-off valve 32 and the common concentrating-mechanism 34. And, the moist-air introducing-tube- B encompasses a filter 21, a mass flow controller (MFC) 22, a humidifier 20, a humidification-input valve 23a and a humidification-output valve 23b. The humidification-input valve 23a and the humidification-output valve 23b is implemented by a three-way valve, and the humidifier 20 is implemented by a moisture-generating nebulizer (liquid atomization apparatus) and an infiltration tube, etc. The filter 21 includes an activated charcoal, etc., and has a function of generating the cleaned dry-air by cleaning up the impurities and moisture included in the environmental air. Here, it is preferable that the environmental air is an air which is extracted from a space different from an inspection room, or an outside air. However, the environmental air is specifically not limited to the air extracted from the space different from the inspection room or the outside air, and may be an air extracted from the same space as the inspection room. The humidifier 20 has a function for supplying a steam of the liquid, configured to achieve the specific-binding reaction, into the cleaned dry-air, and thereby, generating the cleaned moist-air.

The dry-air bypass-tube-C of the virus test device according to the fundamental embodiment is a bypass pathway to the humidifier 20 in the path of moist-air introducing-tube- B. The purge-gas introducing-tube-D of the virus test device according to the fundamental embodiment encompasses a gas-supply unit 70, the exit side of the purge-gas introducing-tube-D from the gas-supply unit 70 is connected to a middle point of the virus introducing-tube-A, between the filter 30a and the on-off valve 32. The gas-supply unit 70 supplies the disinfecting gas or the purge gas to the virus introducing-tube-A selectively, through the purge-gas introducing-tube-D. The disinfecting gas is, for example, used for removing the viruses, etc., attached to the inner walls of the tube and the apparatus, which are disposed in a path from the virus introducing-tube-A to the suction pump 40, after the period when checking whether the viruses exists or not in the air is finished. By the way, the purge gas is used for removing the non-specific adsorption (NSA) material other than the target viruses adsorbed on the surface of the detector-substrate 11a. In addition, the purge gas is used for exhausting the residual gas adsorbed at the inner-walls of the tube and the apparatus, before supplying the disinfecting gas. And, the purge gas is used for exhausting the gas adsorbed at the inner-walls of the tube and the apparatus, before confirming the variations of humidity in the tube and the apparatus, caused by the drive of humidifier 20.

In the virus test device of the fundamental embodiment, the aerosols passing through filter 30a turns to the aerosols-under-test 33b implemented by hydrous aerosols, in which the aerosols passing through filter 30a is mixed with the cleaned moist-air produced by the humidifier 20. In FIG. 1, a common concentrating-mechanism 34 is represented schematically by a triangle to illustrate a topology of an ejection nozzle. The common concentrating-mechanism 34 may have ever-shrinking ejecting nozzle shapes, as exemplified in FIG. 11A or FIG. 11B. However, it is not necessary that the shapes of the common concentrating-mechanism 34 are limited to the concrete geometries illustrated in FIG. 11A or FIG. 11B. And therefore, various configurations are available as the shapes of the common concentrating-mechanisms 34, if the structures include the shrinking tapered shape. The density of the aerosols-under-test 33b made by hydrous aerosols becomes higher in a clump of the aerosols-under-test 33b, by passing through the common concentrating-mechanism 34. And therefore, the number density per unit volume of the target viruses included in the aerosols-under-test 33b becomes more enriched. The collection of the aerosols-under-test 33b which is ejected from the common concentrating-mechanism 34 is, as illustrated in FIG. 1, evacuated by the suction pump 40 connected to the detection vessel 10, and is ejected from the common concentrating-mechanism 34 into the inside of the detection vessel 10 as the high-speed air-flow. The collection of the aerosols-under-test 33b are ejected to the pseudo-receptor film 13, which is disposed on at least partial area in the detector-substrate 11a, along with the high-speed air-flow. By the way, the nozzle structure of FIG. 11B is a dual-wall structure with an outer sheath flow-F having a function of the collimation-focusing action, surrounding an inner main flow implemented by the clump of the aerosols-under-test 33b. The sheath flow-F is implemented by a cleaned air. As the inner jet of aerosols is wrapped by the outer flow of the sheath flow-F, the jet of aerosols does not spread. So, the clump of the aerosols-under-test 33b made by hydrous aerosols can be collected efficiently to the surface of the pseudo-receptor film 13, which is housed in the ball SAW sensor 1003.

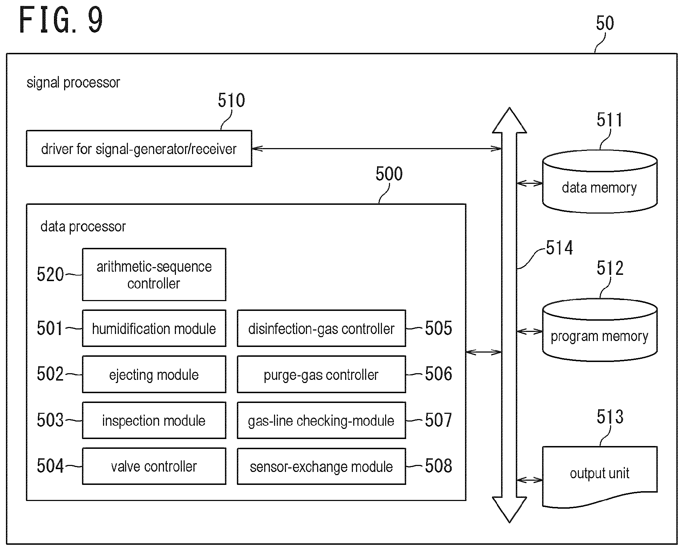

The signal processor 50 of the virus test system of the fundamental embodiment illustrated in FIG. 9 controls operations of the detection vessel 10, the humidifier 20, the MFC 22, the suction pump 40 and the gas-supply unit 70 illustrated in FIG. 1 respectively. Furthermore, the signal processor 50 controls operations of the open/close of the on-off valve 32, the humidification-input valve 23a, and the humidification-output valve 23b respectively. The cleaned dry-air passed through the filter 21 is supplied to the humidifier 20, after the flow rate is controlled by the MFC 22. Or alternatively, the cleaned dry-air passed through the filter 21 and the MFC 22 is supplied to the virus introducing-tube-A at the bifurcation node between the on-off valve 32 and the common concentrating-mechanism 34, which has the tapered nozzle shape, through the dry-air bypass-tube-C. The cleaned moist-air produced by the humidifier 20 is supplied to the virus introducing-tube-A at the bifurcation node between the on-off valve 32 and the common concentrating-mechanism 34. The moist-air introducing-tube- B and the dry-air bypass-tube-C are switched via the signal processor 50 alternatively.

As illustrated in FIG. 3, the detection vessel 10 implementing the detection cell 1000 of the virus test device according to the fundamental embodiment encompasses the inlet-canal 35a merged with the common concentrating-mechanism 34 at the one side of the detection vessel 10. The common concentrating-mechanism 34 condenses the number density per unit volume as to the clump of the aerosols-under-test 33b made of the hydrous aerosols. The detection vessel 10 implementing the detection cell 1000 is built by a rectangular hermetic chamber embracing a metallic encapsulating box 123 having a window at an upper central ceiling and a metallic lid 121 which closes the upper central ceiling of the encapsulating box 123. An O-ring groove 132a is engraved as U-groove for achieving the hermetic chamber at an uppermost edge of the encapsulating box 123, the O-ring groove 132a surrounds the upper portion of the encapsulating box 123. And the O-ring 131a is put into the O-ring groove 132a. Furthermore, an O-ring groove 133a is engraved as U-groove at an under surface of the lid 121 by a topology corresponding to the O-ring groove 132a. The exhaust tube 134 is provided at the other side (the right side in FIG. 3) opposite to the inlet-canal 35a of the encapsulating box 123, and an orifice 9 is provided in the inside of the exhaust tube 134. The one partial portion of the virus introducing-tube-A schematically (conceptionally) illustrated in FIG. 1 is implemented by the common concentrating-mechanism 34 and the inlet-canal 35a, which is merged with the common concentrating-mechanism 34. And, the assemblage of the aerosols-under-test 33b passed through the filter 30a in the configuration illustrated in FIG. 1 is ejected as the high-speed air-flow towards the pseudo-receptor film 13 of the ball SAW sensor 1003 encapsulated in the detection vessel 10.

The common concentrating-mechanism 34 generates the high-speed air-flow toward the pseudo-receptor film 13 by the geometry of the ejection nozzle having the tapered shrinking hole, which is provided inside a first-path end-plaque 124a as the termination of the virus introducing-tube-A. The common concentrating-mechanism 34 at the first-path end-plaque 124a and the inlet-canal 35a of the encapsulating box 123 are coupled hermetically with each other, by using mechanism of an O-ring 131b. Therefore, an O-ring groove 132b is engraved as the U-groove at the left side of the encapsulating box 123, so as to surround the inlet-canal 35a, and an O-ring 131b is put into the O-ring groove 132b. An O-ring groove 133b is engraved as the U-groove at the right side of the first-path end-plaque 124a, corresponding to the geometry of the O-ring groove 132b.

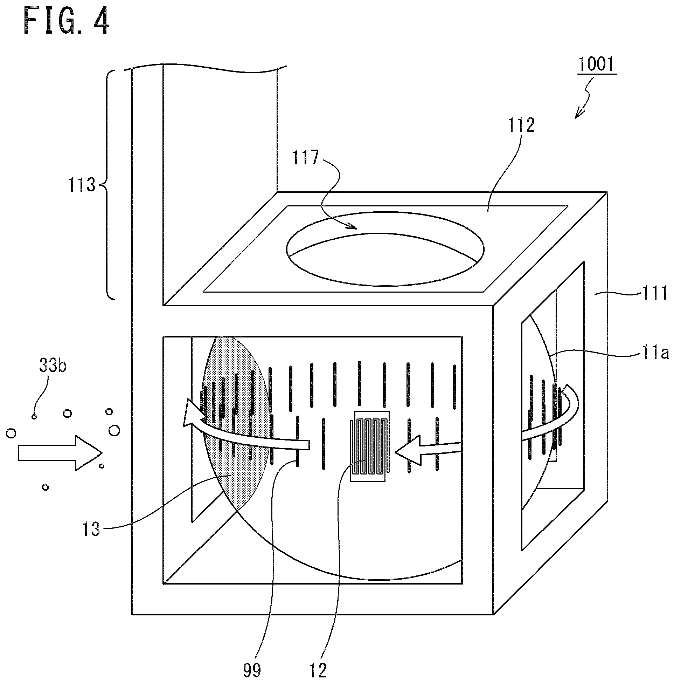

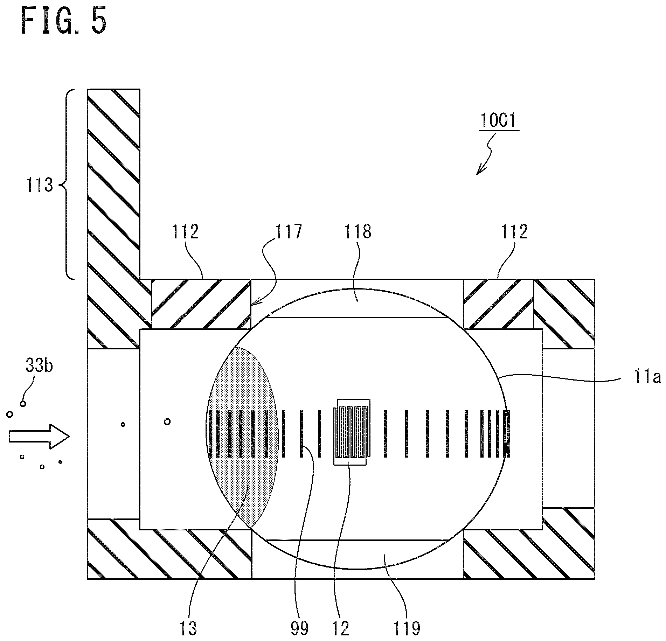

The ball SAW sensor 1003 is accommodated inside a handle-attached susceptor 1001 illustrated in FIG. 4 to FIG. 6. The susceptor facilitates the adjustment processes of the orientations of the north-pole and the south-pole of the ball SAW sensor 1003. The handle-attached susceptor 1001 encompasses a flame 111, a flame-cap 112 provided on the upper surface of the flame 111, and a handle 113, configured to hold the flame 111, etc. The flame 111 of the handle-attached susceptor 1001 is a cube or rectangular shaped skeleton flame made of resin, which is possible to accommodate the ball SAW sensor 1003. The flame 111 is built by quadruple vertical pillars. As illustrated at the left upper portion in FIG. 5, the handle 113, which will serve as the holding portion of susceptor 1001 when the replacements of the ball SAW sensors 1003 are executed, is provided as the integral structure so that the handle 113 continues to the flame 111 at one side of the upper surface of the ceiling of the flame 111. As understood from FIG. 4 to FIG. 6, the handle 113 may have a plate-like structure. As illustrated in FIG. 4, the flame-cap 112, which facilitates the openings of the upper portion of the flame 111, is provided as a removable element on the upper surface of the flame 111. A top contact-window 117 for the electrical connection to the north-pole electrode 118, which is located at the north-pole of the ball SAW sensor 1003, is provided at the middle of the upper portion of the flame-cap 112. Similarly, a bottom contact-window for the electrical connection to the south-pole electrode 119, which is located at the south-pole of the ball SAW sensor 1003, is provided at the middle of the bottom plane of the flame 111. However, the bottom contact-window provided at the bottom plane is not illustrated in the FIG. 4 in an assertive way, because the south-pole side of the detector-substrate 11a is connected to the bottom contact-window provided at the bottom plane. Since the detector-substrate 11a is spherical, the bottom contact-window, which is located at the middle of the bottom plane of the handle-attached susceptor 1001, is a circular recess for trapping the detector-substrate 11a so as not to roll over the detector-substrate 11a, having the similar effectiveness as the top contact-window 117.

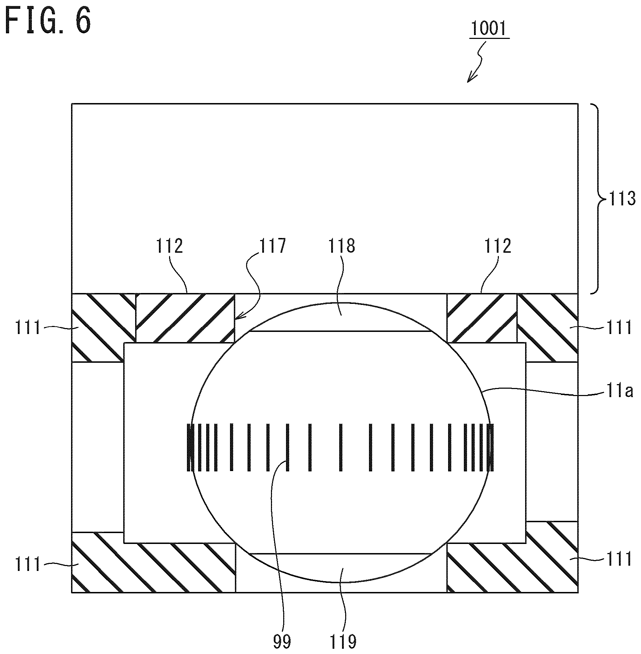

As understood from FIG.5 and FIG. 6, the ball SAW sensor 1003 is inserted into the handle-attached susceptor 1001, with precise and sophisticated alignments of a north-pole 118, a south-pole 119 and a belt of the equational zone on the detector-substrate 11a, in advance. And thereafter, the ball SAW sensor 1003 is fixed by the edge of the top contact-window 117 and by the edge of the bottom contact-window. Since the lid 121 of the detection vessel 10 illustrated in FIG. 4 has a structure easily removable from the encapsulating box 123, the taking in and out of the flame 111 from the upper portion of the encapsulating box 123 are executed easily, after detaching the lid 121 from the encapsulating box 123. That is, the accurate alignments of the north-pole 118, the south-pole 119 and the belt of the equational zone on the ball SAW sensor 1003 are required for assembling the detection cell 1000. The requirement of the accurate alignments is achieved by preparing a assembled structure, in which the ball SAW sensor 1003 is alignment-adjusted inside the flame 111 precisely and sophisticatedly with the aid of the handle-attached susceptor 1001 in advance, at the time of factory shipment as illustrated in FIG. 4 to FIG. 6. That is, in the case that the handle-attached susceptor 1001 is prepared in advance, because the taking in and out of the ball SAW sensor 1003 to the detection vessel 10 is executable with the assembled structure, the assemble process of the detection cell 1000 will become easy for users, even if the user of the virus test device according to the fundamental embodiment is a person who has no skill of the alignment adjustment.

Therefore, in the practical side of industry, it is better that a set of the handle-attached susceptor 1001 and the ball SAW sensor 1003, which will be accommodated in the handle-attached susceptor 1001, is shipped from the factory, as a commercial product of "the sensor unit", and the users are supposed to assemble the detection cell 1000 with the set of the handle-attached susceptor 1001 and the ball SAW sensor 1003. As understood from FIG. 3, the U-groove for alignment is engraved at the bottom plane of the lid 121 such that the U-groove can mate with the location of the handle 113. As illustrated in FIG. 3, if the U-groove for alignment is prepared at the bottom plane of the lid 121, the alignment of the locations of the aerosol jet ejected from the inlet-canal 35a and the pseudo-receptor film 13 can be executed easily, by fitting the uppermost edge of the handle 113 in the corresponding alignment groove with close tolerance. The handle-attached susceptors 1001 illustrated in FIG. 4 to FIG. 6 can be employed for manufacturing or regenerating, etc., the pseudo-receptor film 13. And therefore, the handle-attached susceptor 1001 achieves an effectiveness that the handling of the detector-substrate 11a in the above manufacturing or regenerating process becomes easy.

As illustrated in FIG. 3, an electrode-feedthrough 122 made of insulator is provided at the middle of the lid 121 of the detection vessel 10 of the virus test device according to the fundamental embodiment, and the rod-like external-electrode 105 pass through the middle of the electrode-feedthrough 122 toward the vertical direction. The tip of the lower portion of the external-electrode 105 is electrically connected to the north-pole 118 through the top contact-window 117. The metallic encapsulating box 123 is electrically set to a ground potential. As illustrated in FIG. 3, a projecting portion protruded at the middle of the bottom portion of the encapsulating box 123 is electrically connected to the south-pole electrode 119 through the bottom contact-window which is provided at the middle of the bottom plane of the flame 111. By the way, although the illustration is omitted, a Peltier element and a thermistor may be assembled of the encapsulating box 123. The Peltier element can be used as a tool for heating and cooling the detector-substrate 11a. The thermistor can replace the other temperature sensors, such as a thermocouple, and can be used as a tool for adjusting the temperature of the detector-substrate 11a. As described above, since the O-ring 131a as the sealing element is provided between the upper portion of the encapsulating box 123 and the lid 121, an architecture which can replace the handle-attached susceptor 1001 for exchanging the sensor units can be achieved. Therefore, the structure using mechanism of an O-ring 131a ensures the hermetical sealing, as the O-ring 131a prevents the leakage of gas after operation procedures which includes the processes of taking in and out of the handle-attached susceptor 1001.

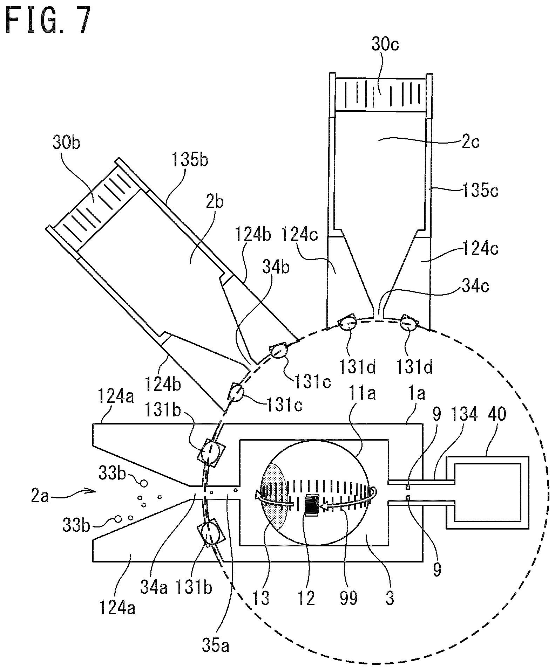

The conceptional architecture of the virus test device according to the virus test system pertaining to the fundamental embodiment illustrated in FIG.1 can be achieved more easily and compactly, by a specific scheme according to a first modification of the fundamental embodiment as illustrated in FIG. 7. That is, in FIG. 1, although the paths of the virus introducing-tube-A, the moist-air introducing-tube- B and the dry-air bypass-tube-C are selected alternatively by controlling the operations of the on-off valve 32, the humidification-input valve 23a and the humidification-output valve 23b, in the case of using the concrete configuration of the virus test device pertaining to the first modification of the fundamental embodiment illustrated in FIG. 7, the switching of the paths of the virus introducing-tube-A, the moist-air introducing-tube- B and the dry-air bypass-tube-C are possible effectively without using the on-off valve 32, the humidification-input valve 23a and the humidification-output valve 23b. In FIG. 3, the hermetic chamber embracing the encapsulating box 123 and the lid 121 was explained. In the virus test device pertaining to the first modification of the fundamental embodiment, the handle-attached susceptor 1001 is accommodated in the hermetic chamber illustrated in FIG. 3. For simplification, in FIG. 7, entire structure such that the handle-attached susceptor 1001 is accommodated in the hermetic chamber is represented as "a cell-enclosure (chassis) 1a". By the way, in FIG. 7, the labeling of the reference numeral 1003 to the ball SAW sensor as illustrated in FIG. 2 is omitted for convenience. In addition, the labeling of the reference numeral 1000 to the detection cell illustrated in FIG. 3 is also omitted. Associated with the omissions of the labeling of the reference numerals 1003 and 1000 in the corresponding diagrams, the representation method as "the ball SAW sensor (11a, 12, 13)" and "the detection cell (11a, 12, 13)" is adopted in the description of FIG. 7. The explanation of FIG. 3 was addressed to the structure that the common concentrating-mechanism 34 at the first-path end-plaque 124a and the inlet-canal 35a of the encapsulating box 123 are jointed hermetically with each other using mechanism of an O-ring 131b. However, the description of FIG. 7 should be read as a structure in which a position of a first concentrating-mechanism (a virus-concentrating-mechanism) 34a at a first-path end-plaque 124a and a position of an inlet-canal 35a cut in the wall of the cell-enclosure 1a will be mated hermetically by using mechanism of an O-ring 131b. The first concentrating-mechanism 34a is a dedicated concentrating-mechanism, which is purpose-built for generating a high-speed air-flow containing enriched target viruses with higher number density, and the high-speed air-flow containing the enriched target viruses is ejected to the pseudo-receptor film 13.

And, in the concrete configuration of the virus test device pertaining to the first modification of the fundamental embodiment illustrated in FIG. 7, a second-path end-plaque 124b and a third-path end-plaque 124c are provided. A central line of the second-path end-plaque 124b and a central line of the third-path end-plaque 124c have a common rotational axis with a central line of the first-path end-plaque 124a, implementing a radial relationship with regard to the common rotational axis. That is, ejecting directions of triple high-speed air-flows, which are defined respectively along the central lines penetrating respectively in the first-path end-plaque 124a, the second-path end-plaque 124b and the third-path end-plaque 124c, intersect each other at a center of a circle represented by a broken line surrounding the cell-enclosure 1a of FIG. 7. The first-path end-plaque 124a is prepared for ejecting the AUT 31a to the pseudo-receptor film 13 of the ball SAW sensor (11a, 12, 13). And therefore, a first concentrating-mechanism 34a is cut in the exit side of the first-path end-plaque 124a so that the AUT 31a can be ejected through the first concentrating-mechanism 34a. By the way, the first-path end-plaque 124a is independent and free from the potion of the cell-enclosure 1a. And therefore, an edge of the first-path end-plaque 124a can be attached to the cell-enclosure 1a, or alternatively can be separated from the cell-enclosure 1a, through the mechanism of O-ring 131b. That is, a mating relationship with the cell-enclosure 1a from the first-path end-plaque 124a can be exchanged to another jointing relationship from the second-path end-plaque 124b, or to still another jointing relationship from the third-path end-plaque 124c, through a relative-rotation process along the outer circumference of the circle represented by the broken line, which surrounds the cell-enclosure 1a of FIG. 7.

The term of the "relative-rotation" means that either schemes of rotating the cell-enclosure 1a located at an intermediate position in the circle, or rotating a turntable, on which the first-path end-plaque 124a, the second-path end-plaque 124b and the third-path end-plaque 124c are mounted, while the cell-enclosure 1a is fixed, can be elected. A purpose-built second concentrating-mechanism 34b or a purpose-built dry-air concentrating-mechanism 34b is cut in the second-path end-plaque 124b, implementing a shrinking tapered shape in the second-path end-plaque 124b, like the first-path end-plaque 124a. And, an entrance side of the second-path end-plaque 124b is connected hermetically to the connection tube 135b, providing a drying chamber 2b in an exist side of the connection tube 135b, so that a dryer can be installed in the drying chamber 2b. The second concentrating-mechanism 34b of the second-path end-plaque 124b and the inlet-canal 35a cut in the wall of the cell-enclosure 1a are configured to be mated hermetically, by using mechanism of an O-ring 131c, after the radial position of the inlet-canal 35a cut in the wall of the cell-enclosure 1a is rotationally moved relatively to an appropriate position of the second-path end-plaque 124b. The second-path end-plaque 124b is provided for operating the purging process, by suppling the cleaned dry-air on the surface of the pseudo-receptor film 13 of the ball SAW sensor (11a, 12, 13), and for removing the NSA material such as the impurities adsorbed on the inner wall of the cell-enclosure 1a and the surface of the detector-substrate 11a.

A purpose-built concentrating-mechanism 34c, or a purpose-built moist-air concentrating-mechanism 34c, which has a shrinking tapered shape, is cut in the third-path end-plaque 124c. And, the connection tube 135c is connected hermetically to the input of the third-path end-plaque 124c, thereby providing a humidification chamber 2c with the connection tube 135c so that a humidifier of a nebulizer, or liquid atomization apparatus, etc. can be installed in the humidification chamber 2c. A third filter 30c is connected hermetically to the entrance of the humidification chamber 2c. The third concentrating-mechanism 34c of the third-path end-plaque 124c and the inlet-canal 35a cut in the wall of the cell-enclosure 1a will be mated hermetically by using mechanism of an O-ring 131d, after the radial position of the inlet-canal 35a cut in the wall of the cell-enclosure 1a is rotationally moved relatively to an appropriate position of the third-path end-plaque 124c. The third filter 30c corresponds to, for example, the filter 21 in the configuration illustrated in FIG. 1, ingests outdoor air into the humidification chamber 2c from the outside, and makes cleaned dry-air by cleaning up the impurities from the outdoor air, the impurities having predetermined size or more, and by removing unnecessary moistures from the outdoor air. To the cleaned dry-air from the third filter 30c, the humidification chamber 2c provides moistures with a predetermined amount. Therefore, the cleaned moist-air can be ejected to the surface of the pseudo-receptor film 13 of the ball SAW sensor (11a, 12, 13), using the third concentrating-mechanism 34c, after mating hermetically the radial position of the third-path end-plaque 124c to the cell-enclosure 1a, which implements the detection cell (11a, 12, 13), so that the humidification process can be executed on the surface of the pseudo-receptor film 13.

The second filter 30b may be also connected hermetically to the entrance side of the drying chamber 2c of the second-path end-plaque 124b side. With aid of the second filter 30b, the purging of the inner wall of the cell-enclosure 1a and the surface of the detector-substrate 11a can be executed, by supplying the cleaned dry-air on the surface of the pseudo-receptor film 13 of the ball SAW sensor (11a, 12, 13), and removing the NSA material, which are adsorbed as the impurities on the inner wall of the cell-enclosure 1a and the surface of the detector-substrate 11a. The second filter 30b, for example, corresponds to the filter 21 in the configuration illustrated in FIG. 1. After ingesting the outdoor air from the outside, the second filter 30b generates cleaned dry-air from the outdoor air, by cleaning up the impurities having the predetermined size or more from the outdoor air, and by removing unnecessary moistures from the outdoor air. The drying chamber 2c has an objective to further dry the cleaned dry-air passed from the third filter 30c. However, the drying chamber 2c may be omitted, if the third filter 30c can generate enough cleaned dry-air. In FIG. 7, although the illustration is omitted, the filter corresponding to the filter 30a in the configuration illustrated in FIG. 1 may be provided at the entrance side of the first-path end-plaque 124a, of course.

Furthermore, the humidification chamber, configured to make a space for installing a humidifier of the nebulizer --the liquid atomization apparatus--, etc., may be connected hermetically between the first-path end-plaque 124a and the filter corresponding to the filter 30a, for ejecting the aerosols-under-test 33b. According to the virus test device pertaining to the first modification of the fundamental embodiment illustrated in FIG. 7, the triple dedicated nozzles, that is, the first concentrating-mechanism 34a, the second concentrating-mechanism 34b and the third concentrating-mechanism 34c are prepared depending on independent purposes, respectively. Therefore, since the virus test device encompasses the triple dedicated nozzles, and the dedicated gas piping systems independently connected to the triple dedicated nozzles respectively, the independent high-speed air-flows can be generated, and the triple dedicated nozzles are automatically replaced depending on independent purposes, respectively. As illustrated in FIG. 7, the piping system, the on-off valve 32, the humidification-input valve 23a and the humidification-output valve 23b, etc., which were recited in FIG. 1, are not used in the virus test device pertaining to the first modification of the fundamental embodiment. Therefore, the aerosols-under-test 33b, etc., which are sucked from the outside, can be supplied with the shortest traveling distances to the ball SAW sensor (11a, 12, 13) and to the inner wall of the cell-enclosure 1a, etc.

In addition, according to the virus test device pertaining to the first modification of the fundamental embodiment illustrated FIG. 7, the characteristics of the jets of the aerosols-under-test 33b and the flow velocities of the airflows, etc., can be adjusted by changing the shapes or structures of the first concentrating-mechanism 34a, the second concentrating-mechanism 34b and the third concentrating-mechanism 34c, depending on independent purposes, respectively. Furthermore, the humidification process, the sucking and examination processes and the purging process can be executed continuously, by successive procedures such that executing the humidification process by the third-path end-plaque 124c, next, executing the sucking and examination processes by the first-path end-plaque 124a, after that, executing the purging process by the second-path end-plaque 124b. Furthermore, although the illustration is omitted, for example, the cleaning and disinfecting of the ball SAW sensor (11a, 12, 13), which encompasses the detection cell (11a, 12, 13), and the inner wall of the cell-enclosure 1a can be executed, by adding a fourth constituent element for generating the alcohol vapor, and by using the gas ejected from the nozzle --the fourth concentrating-mechanism-- of the fourth constituent element, etc. According to the virus test device pertaining to the first modification of the fundamental embodiment, the cleaning and disinfection can be conducted, without preparing a new piping system and new valves.

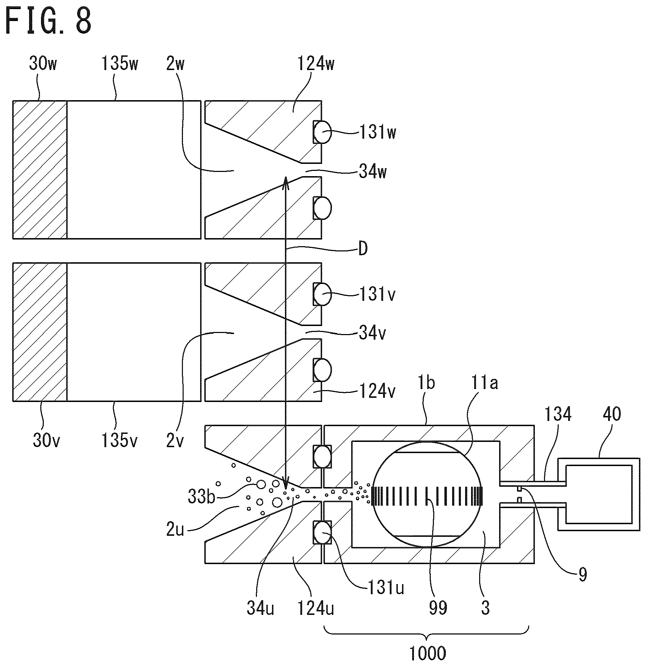

In FIG. 7, the architecture of the rotational-motion has been exemplified according to the virus test device pertaining to the first modification of the fundamental embodiment. However, by an architecture of a translational-motion, or a parallel translation, as illustrated in FIG. 8, the simple and compact virus test device can be realized, as with the effectiveness of the FIG. 7, according to the virus test device pertaining to the second modification of the fundamental embodiment. That is, in FIG. 8, all constituent elements of the structure such that the handle-attached susceptor 1001 is accommodated in the hermetic chamber encompassing the encapsulating box 123 and the lid 121 illustrated in FIG. 3, is simply indicated as "a cell-enclosure (chassis) 1b" as with the representation of FIG. 7. In the simplified representation of FIG. 8, a first concentrating-mechanism 34u provided in the first-path end-plaque 124u and an inlet-canal cut in a wall of the cell-enclosure 1b corresponds to the structure of FIG. 7, in which the first concentrating-mechanism 34a and the inlet-canal 35a mates hermetically by using the mechanism of the O-ring 131b. The surface of the first concentrating-mechanism 34a facing to the cell-enclosure 1a of FIG. 7 is a curved surface. However, the surface of a virus-concentrating-mechanism --the first concentrating-mechanism-- 34u facing to the cell-enclosure 1b of FIG. 8 is a flat surface. As illustrated in FIG. 8, in the virus test device pertaining to the second modification of the fundamental embodiment, a connection relationship of the first-path end-plaque 124u to the cell-enclosure 1b can be exchanged to another connection relationship of the second-path end-plaque 124v to the cell-enclosure 1b, or alternatively to still another connection relationship of the third-path end-plaque 124w to the cell-enclosure 1b, by relative translational-motions to the cell-enclosure 1b along the direction perpendicular to the gas ejecting direction. The term of the "relative translational-motion" means that the cell-enclosure 1b at the right side may be translationally moved, or a translational-motion table on which the first-path end-plaque 124u at the left side, the second-path end-plaque 124v and the third-path end-plaque 124w are mounted, may be translationally moved on the contrary, while the position of the cell-enclosure 1b is fixed.

That is, in a concrete configuration of the virus test device pertaining to the second modification of the fundamental embodiment illustrated in FIG. 8, the second-path end-plaque 124v and the third-path end-plaque 124w having respectively central axes which are parallel relationship to a central axis along the ejecting direction of the first-path end-plaque 124u, are arranged. That is, the ejecting directions of the triple high-speed air-flows from the first-path end-plaque 124u, the second-path end-plaque 124v and the third-path end-plaque 124w are parallel to each other. The second-path end-plaque 124v has the dry-air concentrating-mechanism --a second concentrating-mechanism-- 34v having a shrinking tapered shape as with the virus-concentrating-mechanism --a first concentrating-mechanism-- 34u provided in the first-path end-plaque 124u and is further connected to a connection tube 135v hermetically to the entrance of the second-path end-plaque 124v, thereby providing the drying chamber 2v, which can make a space for installing a dryer. The dry-air concentrating-mechanism 34v of the second-path end-plaque 124v and the inlet-canal cut in the wall of the cell-enclosure 1b will be mated hermetically by using mechanism of an O-ring 131v, after the translational-motion to an appropriate position. Through the second-path end-plaque 124v, the purging process for supplying the cleaned dray-air as the purge gas on the surface of the pseudo-receptor film 13 of the ball SAW sensor (11a, 12, 13) is executed, and the NSA material as the impurities adsorbed on the inner wall of the cell-enclosure 1b and the surface of the detector-substrate 11b is removed by the cleaned dry-air.

A dedicated moist-air concentrating-mechanism --a third concentrating-mechanism-- 34w, having a shrinking tapered shape, is provided in the third-path end-plaque 124w. And, a connection tube 135w is connected hermetically to the third-path end-plaque 124w, thereby providing a humidification chamber 2w, configured to make a space for installing a humidifier such as a nebulizer --a liquid atomization apparatus, etc.-- at an entrance side of the third-path end-plaque 124w. The a dedicated moist-air concentrating-mechanism 34w of the third-path end-plaque 124w and the inlet-canal cut in the wall of the cell-enclosure 1b will be mated hermetically by using mechanism of an O-ring 131w, after the translational-motion to an appropriate position. A third filter 30w is connected hermetically to the entrance of the humidification chamber 2w. The third filter 30w corresponds to, for example, the filter of FIG. 1. The third filter 30w ingests outdoor air into the humidification chamber 2w from the outside, and makes cleaned dry-air, by cleaning up the impurities having the predetermined size or more, and by removing unnecessary moistures. The humidification chamber 2w adds moistures of a predetermined amount to the cleaned dry-air, which is passed from the third filter 30w, to make cleaned moist-air. Therefore, after mating hermetically the third-path end-plaque 124w to the cell-enclosure 1b, the humidification process of the pseudo-receptor film 13 can be executed. In the humidification process, the cleaned moist-air is ejected to the surface of the pseudo-receptor film 13 of the ball SAW sensor (11a, 12, 13), by using the dedicated moist-air concentrating-mechanism --the third concentrating-mechanism-34w.

A second filter 30v may be also connected hermetically to the entrance of the drying chamber 2v of the second-path end-plaque 124v. With aid of the second filter 30v, the purging of the inner wall of the cell-enclosure 1b and the surface of the detector-substrate 11b can be executed, by supplying the cleaned dry-air to the surface of the pseudo-receptor film 13 of the ball SAW sensor (11a, 12, 13). By the purging process, the NSA material adsorbed as the impurities on the inner wall of the cell-enclosure 1b and the surface of the detector-substrate 11b is removed. The second filter 30v, which corresponds to the filter 21 in the configuration illustrated in FIG. 1, for example, ingests outdoor air into the drying chamber 2v from the outside, and makes cleaned dry-air, by cleaning up the impurities having the predetermined size or more, and furthermore, by removing unnecessary moistures. The drying chamber 2v is provided for the purpose of further drying the cleaned dry-air, passed from the second filter 30v. However, the drying chamber 2v may be omitted, if enough cleaned dry-air is generated by the second filter 30v.

In the virus test device pertaining to the second modification of the fundamental embodiment illustrated in FIG. 8, the illustration of a filter corresponding to the filter 30a in the configuration illustrated in FIG. 1 is omitted. However, the filter corresponding to the filter 30a may be provided at the entrance side of the first-path end-plaque 124u, of course. And, when the filter corresponding to the filter 30a is provided, a humidification chamber, configured to make a space for installing a humidifier of the nebulizer --a liquid atomization apparatus), etc., may be connected hermetically between the first-path end-plaque 124u and the filter corresponding to the filter 30a. for ejecting the aerosols-under-test 33b. The humidification chamber will be provided for the purpose of ejecting the aerosols-under-test 33b. According to the virus test device pertaining to the second modification of the fundamental embodiment illustrated in FIG. 8, the triple dedicated nozzles including the virus-concentrating-mechanism --a first concentrating-mechanism-- 34u, the dry-air concentrating-mechanism --a second concentrating-mechanism-- 34v and the a dedicated moist-air concentrating-mechanism --a third concentrating-mechanism-- 34w are prepared, depending on independent purposes, respectively. Therefore, according to the virus test device pertaining to the second modification illustrated in FIG. 8, triple purpose-built high-speed air-flows can be prepared, and the triple purpose-built high-speed air-flows are automatically exchanged sequentially. For preparing the triple purpose-built high-speed air-flows, the piping system, the on-off valve 32, the humidification-input valve 23a and the humidification-output valve 23b, etc., as illustrated in FIG. 1, are not required. Therefore, the aerosols-under-test 33b, etc., which are ingested or sampled, can be supplied to the ball SAW sensor (11a, 12, 13) and the inner wall of the cell-enclosure 1b, etc., by the shortest traveling distance.

According to the virus test device pertaining to the second modification of the fundamental embodiment illustrated FIG. 8, the jets of the aerosols-under-test 33b and the flow velocities of the airflows, etc., can be adjusted, by changing the shapes or structures of the first concentrating-mechanism 34u, the second concentrating-mechanism 34v and the third concentrating-mechanism 34w, depending on independent purposes, respectively. In addition, sequential exchanges of the humidification process, the sucking and examination processes, and the purging process can be executed continuously. The sequential exchanges of the process may include executing the humidification process at the third-path end-plaque 124w, next, executing the sucking and examination processes at the first-path end-plaque 124u, after that, executing the purging process by the second-path end-plaque 124v. Furthermore, although the illustration is omitted, for example, the cleaning and disinfecting of the ball SAW sensor (11a, 12, 13) and the inner wall of the cell-enclosure 1a can be executed, by adding a fourth constituent element for generating the alcohol vapor, and by using the gas ejected from the nozzle of the fourth constituent element, etc. Although the virus test device pertaining to the second modification of the fundamental embodiment illustrated in FIG. 8 is based upon a translational-motion-scheme, the cleaning and disinfection can be executed without preparing a new piping system and new valves because the virus test device encompasses triple dedicated nozzles and the independent gas piping systems, achieving the similar effectiveness explained by the rotational-motion-scheme of the virus test device illustrated in FIG. 7.

The signal processor 50 of the virus test system pertaining to the fundamental embodiment encompasses, as illustrated in FIG. 9, a data processor 500, a driver (a logical circuit) 510 for signal-generator and receiver, a data memory 511, a program memory 512, an output unit 513, and a bus 514 connected the above elements each other. The driver 510 for signal-generator and receiver is a hardware resource implemented by an electronic circuit, etc., which controls and drives an operation of the signal conditioner 12 on the detector-substrate 11a. The driver 510 sends the excitation burst-signals to the signal conditioner 12 on the detector-substrate 11a, and the signal conditioner 12 generates a collimated SAW beam. That is, the signal conditioner 12 receives the electric signals from the driver 510, generates the collimated SAW beam as the acoustic signals by an electro-acoustic conversion, so that the collimated SAW beam propagates around the detector-substrate 11a. Furthermore, the driver 510 sends the drive/control signal to the signal conditioner 12, and the signal conditioner 12 receives the acoustic signals of the collimated SAW beam.

That is, the acoustic signals of the collimated SAW beam, which roundtrips the predetermined number of times around the detector-substrate 11a, are converted to the electric signals by the electro-acoustic conversion using the signal conditioner 12. In addition, the driver 510 receives the electric signals transmitted from the signal conditioner 12, as the feedback burst-signals of the collimated beam, and stores the electric signals in the data memory 511. After that, the driver 510 reads out the waveform data of the feedback burst-signals from the data memory 511, and the waveform data is sent to the data processor 500. Here, a mode in which the feedback burst-signals is sent to the data processor 500 directly without storing in the data memory 511 can be adopted, when the driver 510 receives the feedback burst-signals. The propagation characteristic of the SAW varies owing to the changes of the weights of the pseudo-receptor film 13 in the physical state, when the pseudo-receptors 14 provided on the pseudo-receptor film 13 are bound specifically to the target viruses. In the architecture of the ball SAW sensor 1003 exemplified in FIG. 2, the sensor electrode implementing the signal conditioner 12 converts the acoustic signals representing the variations of the physical states of the pseudo-receptor film 13 to the electric signals by the electro-acoustic conversion. By the way, the signal processor 50 executes the arithmetic and logical operations utilizing the electric signals which are converted by the electro-acoustic conversion and measures the attenuation coefficients of the SAW and the delay times of the SAW.

The data processor 500 implementing the signal processor 50 of the virus test system pertaining to the fundamental embodiment encompasses logical hardware-resources, which include a humidification module (logical circuit) 501, an ejecting module (logical circuit) 502, an inspection module (logical circuit) 503, a valve controller (logical circuit) 504, a disinfection-gas controller (logical circuit) 505, a purge-gas controller (logical circuit) 506, a gas-line checking-module (logical circuit) 507, a sensor-exchange module (logical circuit) 508, and an arithmetic-sequence controller (logical circuit) 520. The humidification module 501 in the data processor 500 selects the moist-air introducing-tube- B by selecting flow paths in the humidification-input valve 23a and the humidification-output valve 23b, after closing the on-off valve 32, so that the cleaned moist-air is ejected to the pseudo-receptor film 13, which covers at least a partial area of the detector-substrate 11a, by starting the operations of the humidifier 20, MFC 22 and the suction pump 40. By supplying moistures of liquid, and by bedewing liquid droplets on the surface of the pseudo-receptor film 13, a liquid film scheduled to be employed in the specific-binding reaction on the pseudo-receptor film 13 is coated on the surface of the pseudo-receptor film 13.

The ejecting module 502 in the data processor 500 is the logical hardware-resource which sends a necessary instruction to the valve controller 504 for controlling operations of the on-off valve 32, the humidification-input valve 23a and the humidification-output valve 23b, in the configuration exemplified in FIG. 1. Under control of the valve controller 504 such that the on-off valve 32 is open state, and the moist-air introducing-tube- B is selected by the flow-paths in humidification-input valve 23a and the humidification-output valve 23b, the ejecting module 502 further sends instructions for starting the operations of the humidifier 20, the MFC 22 and the suction pump 40. The ejecting module 502 generates the high-speed air-flows of the desired aerosols-under-test 33b, and ejects a fluid flow of the aerosols-under-test 33b to the pseudo-receptor film 13, which is disposed on at least partial area in the detector-substrate 11a, through the first concentrating-mechanism 34a, by sending the required instructions to the valve controller 504, the humidifier 20, the MFC 22 and the suction pump 40, respectively. In the first modification of the fundamental embodiment exemplified in FIG. 7, the instructions for controlling the relative rotational-motion in the cell-enclosure 1a are sent to the sensor-exchange module 508. Similarly, in the second modification of the fundamental embodiment exemplified in FIG. 8, the instructions for controlling the relative translational-motion are sent to the sensor-exchange module 508. That is, the relative rotational-motions with the cell-enclosure 1a or the relative translational-motion with the cell-enclosure 1b are controlled through the sensor-exchange module 508. Then, the high-speed air-flows of the aerosols-under-test 33b are ejected to the pseudo-receptor film 13. As the result, the specific-binding reactions for implementing the spike-protein receptor-bindings 19 are accelerated, by the binding reactions of the RBD of the spike-glycoprotein 17 in the target viruses 60 to the pseudo-receptors 14 as illustrated in FIGs. 10A and 10B, on the surface of the pseudo-receptor film 13.

The inspection module 503 in the data processor 500 is the hardware resource, which executes the arithmetic and logical operations for the difference-integral detection, by using the waveform data of the feedback burst-signals as the electric signals which the driver 510 receives from the signal conditioner 12. That is, the inspection module 503 executes the difference-integral detection using the feedback burst-signals, integrating the signals representing the differences of the areal densities by weight due to the bindings of the target viruses 60 to the pseudo-receptors 14 via the spike-protein receptor-bindings 19, as the delay-time responses of the SAW. The inspection module 503 can inspect whether the target viruses 60 exist or not in the AUT 31a, by executing the difference-integral detection for integrating the differences of the areal densities by weight as the delay-time responses of the SAW.

For example, the inspection module 503 sends instructions to the driver 510 (logical circuit) 510, configured to execute the receiving process of the electric signals representing the information of the delay-time responses of the SAW from the signal conditioner 12. After receiving the instructions, the driver 510 receives the electric signals representing the information of the delay-time responses of the SAW from the signal conditioner 12, and the driver 510 stores the electric signals in the data memory 511. After that, the inspection module 503 reads out the electric signals representing the information of the delay-time responses of the SAW from the data memory 511, or directly receives the electric signals from the driver 510, and executes the arithmetic and logical operations necessary for executing the difference-integral detection about the information of the obtained delay-time response. According to the virus test system pertaining to the fundamental embodiment, an in-situ inspection whether the target viruses exists or not in the AUT 31a can be achieved easily, quickly, and with high sensitivity, by executing the arithmetic and logical operations via the difference-integral detection, using the inspection module 503.

By the way, the inspection module 503 in the data processor 500 installed in the virus test system pertaining to the fundamental embodiment sends instructions to the valve controller 504 to select the dry-air bypass-tube-C, by closing the on-off valve 32, and further, by selecting the flow paths in the humidification-input valve 23a and the humidification-output valve 23b. Under the state that the dry-air bypass-tube-C is selected by the control of the valve controller 504, the inspection module 503 sends the instructions to the MFC 22 and the suction pump 40 so that the MFC 22 and the suction pump 40 start operations respectively, thereby ejecting the cleaned dry- air to the pseudo-receptor film 13. That is, the inspection module 503 sends instructions, configured to eject the cleaned dry-air toward the pseudo-receptor film 13, at the timing after the fluid flow of the aerosols-under-test 33b are ejected to the pseudo-receptor film 13, and before the timing that whether the target viruses exist or not is examined. It is possible to blow away and remove impurities --viruses or particles--other than the target viruses, which are bound to the pseudo-receptors 14, from the upper portion side of the pseudo-receptor film 13, by sending instructions, configured to dry the surface of the detector-substrate 11a. Therefore, an in-situ measurement with high sensitivity is achieved.

As mentioned previously, the valve controller 504 in the data processor 500 controls circumstantial operations that include the open and close actions of the on-off valve 32, and the selections of the flow paths in the humidification-input valve 23 and the humidification-output valve 23b. The disinfection-gas controller 505 sends instructions, which are necessary to control the operations of the gas-supply unit 70, so that the gas-supply unit 70 can supply the disinfecting gas to the virus introducing-tube-A. The purge-gas controller 506 sends instructions to the gas-supply unit 70, the instructions are necessary to control the operation of the gas-supply unit 70, and the gas-supply unit 70 supplies the purge gas to the virus introducing-tube-A. The gas-line checking-module 507 executes checking operations of the virus introducing-tube-A, the moist-air introducing-tube- B, the dry-air bypass-tube-C and the purge-gas introducing-tube-D periodically. The sensor-exchange module 508 controls the rotational-motion operations pertaining to the first modification of the fundamental embodiment described in FIG. 7 or the translational-motion operations pertaining to the second modification described in FIG. 8. And the arithmetic-sequence controller 520 controls the sequences of the operations of the humidification module 501, the ejecting module 502, the inspection module 503, the valve controller 504, the disinfection-gas controller 505, the purge-gas controller 506, the gas-line checking-module 507, and the sensor-exchange module 508, respectively.

The above-described data processor 500 may be, for example, an element which is monolithically integrated into one chip to implement the central processing unit (CPU), configured to be incorporated in the virus test device pertaining to the fundamental embodiment. Or, the data processor 500 may be a part of the CPU employed in a general computer system such as a personal computer (PC), etc. The data processor 500 can implement a computer system using a microprocessor (MPU), etc., which is embedded in a microchip. A digital signal processor (DSP), in which the arithmetic function is enhanced, being specialized to a signal processor may be used as the data processor 500 of the virus test device pertaining to the fundamental embodiment. Or alternatively, a microcontroller (microcomputer) merging memories and peripheral circuitry, which has an objective of performing as an embedded controller, etc., may be used as the data processor 500.

The logical circuitry implementing at least one part of the humidification module 501, the ejecting module 502, the inspection module 503, the valve controller 504, the disinfection-gas controller 505, the purge-gas controller 506, the gas-line checking-module 507, the sensor-exchange module 508, and the arithmetic-sequence controller 520 can be provided by a programmable logic device (PLD), such as a field programmable gate array (FPGA), for the data processor 500 installed in the virus test system pertaining to the fundamental embodiment. When PLD constitutes a partial or entire area of the data processor 500, the data memory 511 may be a storage element such as a memory block, which is embedded in one partial area of the logical blocks in the PLD. Furthermore, the data processor 500 may have a configuration such that a CPU-core-like array and PLD-like programmable cores are merged in a same single chip. The CPU-core-like array may include a macro-CPU merged in the PLD previously as a hardware, and a software-macro-CPU implemented by the logical blocks in the PLD. That is, an architecture in which a software scheme and a hardware scheme are mixed in the PLD is available.

By the way, the data processor 500 may include an arithmetic and logic unit (ALU) executing arithmetic and logical operations, a plurality of registers for supplying the operand to the ALU and for storing the results of arithmetic operation by the ALU, and a control unit configured to orchestrate the fetches (from the memory) and executions. The fetches are ascribable to the instructions for instructing adjusted arithmetic in ALU. Furthermore, the data processor 500 installed in the virus test system pertaining to the fundamental embodiment may be an individual hardware resource, such as an electronic circuit merged in the logical circuit block or the monolithic integrated circuit (IC) chip. Or alternatively, the data processor 500 may be implemented by virtual equivalent logical functions, provided by software using the CPU in a general computer system.

The data memory 511 of the virus test system pertaining to the fundamental embodiment stores data used or will be used in the calculations for examining whether the spike-protein receptor-bindings 19 is existing or not. The data memory 511 may be any arbitrary and appropriate combination which is suitably selected from the group including a plurality of registers, a plurality of cache-memories, a main memory, and an auxiliary memory. The cache-memories may be a combination of first-level and second-level cache-memories, and further may have a hierarchy with a third-level cache-memory. In the case that the data processor 500 is implemented by a part or entire of the PLD, the data memory 511 may be made from the memory elements such as memory blocks, etc., which is included in a part of the logical blocks organizing the PLD.

The program memory 512 stores control programs for examining whether the spike-protein receptor-bindings 19 is existing or not. The control programs may be, as described below, stored in a computer-readable storage-medium, etc., and then, the control programs will be transferred to be stored in the program memory 512. Or alternatively, the control programs may be downloaded from the server storing the control programs through internet connections, and then, are transferred to be stored in the program memory 512. The output unit 513 may have a function for delivering the inspection results about whether the spike-protein receptor-bindings 19 is present or not present, as necessary. For example, the output unit 513 can include an alarm apparatus such as an alerting light, etc. As the virus test systems pertaining to the fundamental embodiment have very compactable features, the test devices can incorporate the alerting lights, which are integrable or connectable to the test devices.

If the presence of the intended target virus 60 in the AUT 31a is confirmed after the inspection process, the immediate escape from the inspected space will be alarmed to the people staying around the subject inspected space, by blinking the alerting light on and off, etc., and by reproducing voice sounds, conforming with the blinking of the alerting light, etc. Alternatively, it is possible to execute a screening process for virus-infection preventions, such as selectively allowing the movement of the specific people who has been tested negative as the result of the expiration measurement. For executing the screening process, a card writing apparatus shall be connected to the output unit 513, so that the result of expiration measurement can be write in the wearable individual card as the personal information. An information writing instrument for writing the personal information in the individual portable terminals, such as the smart phones, can be connected to the output unit 513. Then, if the histories of the personal information stored in the wearable cards or the portable terminals are employed as input information for the automatic ticket checkers of the mass transportation systems or the automatic open and close apparatuses at the gates of the event sites, it is possible to execute the screening of the people who enters the mass transportation systems and the event sites. The compactable feature of the virus test system pertaining to the fundamental embodiment facilitates an achievement of ubiquitous virus test system. Especially, if the technology standard for broadband cellular networks of fifth-generation(5G), sixth-generation(6G), seventh-generation (7G), etc. is used, the feature of the signal processor 50 becomes more compact and lighter, by transferring a part of the functions of the data processor 500 and the data memory 511, etc. illustrated in FIG. 9 to the cloud server. For executing the cloud computing by the 5G wireless technology, etc., the signal processor 50 is necessary to incorporate the communication capability of transmission and receiving. However, entire portions of the virus test system pertaining to the fundamental embodiment can be incorporated in a smart phone or a tablet terminal, as a part of the smart phone or the tablet terminal, by utilizing the compactable feature. In the case of incorporating the virus test system into the smart phone or the tablet terminal, it is unnecessary to add new communication capability for transmission and receiving to the configuration illustrated in FIG. 9.

As described above, according to the virus test system pertaining to the fundamental embodiment, in the first step, the liquid droplets are bedewed on the pseudo-receptor film 13 by supplying the cleaned moist-air. Furthermore, in the second step, the aerosols-under-test 33b are generated as the hydrous aerosols, by supplying liquid via cleaned moist-air to coarse aerosols 33a included in the AUT 31a, after that, the aerosols-under-test 33b are ejected to the pseudo-receptor film 13. That is, according to the virus test system pertaining to the fundamental embodiment, it is possible to coat the liquid film on the pseudo-receptor film 13 easily and quickly. Furthermore, the aerosols-under-test 33b made of the hydrous aerosols can be elaborated, by incorporating the liquid into the coarse aerosols 33a. In addition, since the liquid with minimum required quantity is supplied to the pseudo-receptor film 13 and the coarse aerosols 33a, the diffusion process of the viruses in the liquid will not play dominant role. Therefore, in-situ measurement of the viruses in a high speed of the order of seconds can be achieved.

In addition, as a simple in-situ measurement of the viruses with quick and high sensitivity can be provided, actions including an immediate escape from the dangerous air environment, and air cleaning by a strong air ventilation, etc., can be performed, immediately after the environment measurement. Therefore, the effectiveness that the infection risk can be decreased is achieved. Furthermore, since the difficulty in reducing size and weight of the liquid-based biosensor is eliminated, applications to transportable equipment, which can be used in the on-site and in-situ measurement in the transportation facilities, theater, etc. can be achieved, according to the virus test system pertaining to the fundamental embodiment. Therefore, a simpler expiration analyzer adapted for the screening processes at practical sites can be provided.

A receiving mechanism for liquid droplets or a drying mechanism for moistures, etc., may be provided in a part of the piping system, if there is a risk of bedewing with the liquid, which is scheduled to be used in the specific-binding reactions, in a path from the humidifier 20 to the detection vessel 10 and in a space of the detection vessel 10, when the ejection processes of the aerosols are continued.

### --TEST METHOD BY FUNDAMENTAL EMBODIMENT--

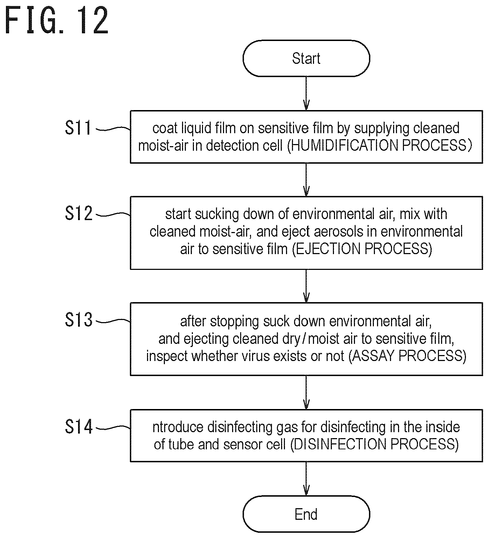

Hereinafter, an example of a virus test method pertaining to the fundamental embodiment will be described with reference to a flow chart illustrated in FIG. 12. In the following description, for simplicity of the description, a case of examining whether the viruses exists or not in the AUT 31a, by using the virus test system pertaining to the fundamental embodiment conceptionally illustrated in FIG. 1, will be explained.

### (1) (HUMIDIFICATION PROCESS)

At first, in a step S11 represented in FIG. 12, the liquid film 61 is coated on the pseudo-receptor film (the sensitive film of SAW) 13 merging the pseudo-receptors 14 as illustrated in FIG. 10B. The liquid film 61 is coated by the amount enough to maintain the activated state of the pseudo-receptors 14. The liquid film 61 is, for example, a water, or a buffer solution mimicking the biological environment. Concretely, the on-off valve 32 is set to the closed state, and the moist-air introducing-tube- B is selected by the flow-paths in humidification-input valve 23a and the humidification-output valve 23b, according to a specific flow-path in the three-way valve. The impurities and the moisture are removed, and the cleaned dry-air is produced by the filter 21 in which the activated charcoal, et al., is filled, after the environmental air has been taken in, by starting the operation of the suction pump 40. The cleaned dry-air becomes to the cleaned moist-air by the humidification, using the humidifier 20, after adjusting the flow rate to about 1L/min by the MFC 22. The liquid film 61 is adsorbed on the inner wall of the upper-stream tube toward the detector-substrate 11a and on the pseudo-receptor film 13 including the pseudo-receptors 14, when the cleaned moist-air is supplied to the pseudo-receptor film 13, which is disposed on at least partial area in the detector-substrate 11a, through the humidification-output valve 23b and the first concentrating-mechanism 34a.

### (2) (EJECTION PROCESS)

Next, in a step S12 represented in FIG. 12, the AUT 31a is sucked down. And, a fluid flow of the aerosols-under-test 33b implemented by hydrous aerosols, which are produced by the process of incorporating liquid into coarse aerosols 33a included in the sucked AUT 31a, is constructed in the step S12. And thereafter, the fluid flow of the aerosols-under-test 33b is ejected to the pseudo-receptor film (the sensitive film of SAW) 13 in the step S12. Concretely, the on-off valve 32 is controlled to an open state, and the moist-air introducing-tube- B is selected, by adjusting the humidification-input valve 23a and the humidification-output valve 23b, which are the three-way valves, respectively, and define a specific flow-path. The AUT 31a is sucked at the same time of sucking down the environmental air, by starting the operation of the suction pump 40. The fluid flow of the AUT 31a becomes the fluid flow of the aerosols-under-test 33b made of the hydrous aerosols, by mixing the cleaned moist-air produced by the humidifier 20. And, as illustrated in FIG. 11A or FIG. 11B, the fluid flows of the aerosols-under-test 33b become the high-speed air-flows, which are concentrated by the common concentrating-mechanism 34, and are supplied to the pseudo-receptor film 13, which is disposed on at least partial area in the detector-substrate 11a. As the result, if the target viruses 60 are contained in the fluid flow of the aerosols-under-test 33b, the specific-binding reactions between the REDs of the spike-glycoproteins 17 in the target viruses 60 to the pseudo-receptors 14 will occur on the pseudo-receptor film 13.

Here, there is some possibility that particles having larger size and the coarse aerosols 33a, which serve as the obstructions for the inspection process, are included in the AUT 31a ingested from the air-intake port. Then, the coarse aerosols 33a and the particles having sizes larger than a predetermined size, for example, four micrometers, are removed by the filter 30a to produce aerosols-under-test 33b, for improving the inspection precision.

When the fluid flow of the aerosols-under-test 33b is supplied to the pseudo-receptor film 13, for example, as illustrated in FIG. 10B, under the condition that the liquid film 61 is existing on the surface of the pseudo-receptors, the particles of the aerosols-under-test 33b containing the target viruses 60 are merged with the liquid film 61 on the surface of the pseudo-receptor film 13. On the other hand, as illustrated in FIG. 10A, even if the liquid film 61 is not existing on the surface of the pseudo-receptors, the particles of the aerosols-under-test 33b encapsulating the target viruses 60 will arrive at the surface of the pseudo-receptor film 13. And the target viruses 60 will be captured by the surface of the pseudo-receptor film 13 via the specific-binding reactions, which are implemented by the spike-protein receptor-bindings 19 of the RBD of the spike-glycoprotein 17 and the pseudo-receptors 14, in the liquid particle held by the aerosols-under-test as illustrated in FIG. 10A, or alternatively, in the liquid film 61 coated on the surface of the pseudo-receptors as illustrated in FIG. 10B. For example, in the case that the target viruses 60 is SARS-CoV-2 viruses, it is known that the RBD of the spike-glycoprotein 17 is bound to human receptor angiotensin-converting enzyme 2 (ACE2) by high affinity. Therefore, in the objective of examining whether the SARS-CoV-2 virus exists or not, the pseudo-receptors 14 shall mimic the configuration of the ACE2 receptor. For examine the presence of the SARS-CoV-2 virus, it is preferable that the flow rate of the cleaned moist-air is controlled to an optimum value, depending on the humidity of the AUT 31a, thereby maximizing the capturing efficiency. For example, the flow rate of the cleaned moist-air is limited to about 0.1 L/min by the MFC 22.

### (3) (ASSAY PROCESS)

Next, in a step S13 represented in FIG. 12, the on-off valve 32 is set to the closed state, after the operations of the humidifier 20, the MFC 22 and the suction pump 40 are stopped temporarily. After that, the operations of the MFC 22 and the suction pump 40 are started again, and further, the cleaned-dry air is ejected to the pseudo-receptor film (the sensitive film of SAW) 13 disposed on at least partial area in the detector-substrate 11a by selecting the dry-air bypass-tube-C, using the humidification-input valve 23a and the humidification-output valve 23b. Alternatively, the cleaned moist-air may be ejected to the pseudo-receptor film 13 disposed on at least partial area in the detector-substrate 11a by operating the humidifier 20 and selecting the moist-air introducing-tube- B, instead of the dry-air bypass-tube-C.

As described above, with the steps S12 and S13, a double-phase scheme is executed before the process of the difference-integral detection for inspecting whether the target viruses 60 exist or not is executed in the step S13, such that the cleaned-dry air or the cleaned moist air is ejected to the pseudo-receptor film 13 in the step S13, after the fluid flow of the aerosols-under-test 33b are ejected to the pseudo-receptor film 13 in the step S12. That is, with the double-phase scheme, the impurities such as dust particles, etc., or the viruses, which are nonspecifically adsorbed to the pseudo-receptor film 13 in the step S12 can be removed, before the process of the difference-integral detection in the step S13. By executing the double-phase scheme, the difference of areal densities by weight by the spike-protein receptor-bindings 19, via the specific-binding reaction of the target viruses 60 with the pseudo-receptor film 13 disposed on at least partial area in the detector-substrate 11a, can be detected by the difference-integral detection with a higher precision, the difference-integral detection may include a calculation of the delay-time response of the SAW, for example. Here, the double-phase scheme includes the process of ejecting the cleaned-dry air or the cleaned moist air toward the pseudo-receptor film 13 in the step S13 and the process executed by the step S12.

### (4) (DISINFECTION PROCESS)

Next, in a step S14 represented in FIG. 12, a disinfection process in the insides of the tubes and the equipment, which are contaminated by the assay process in the step S13, is executed. Concretely, in the step S14, the disinfection in the insides of the tubes of the virus test device and in the inner space of the detection vessel 10 according to the fundamental embodiment, etc., is executed by introducing the disinfecting gas from the gas-supply unit 70. If the assay processes using the virus test system pertaining to the fundamental embodiment are conducted continuously at other inspection sites in the step S14, the inspections with the higher precision can also be executed at other inspection sites, because the virus test system can be used safely with security, by cleaning the inside of the tubes, etc. Although the disinfection process of supplying the disinfecting gas is described as an example, the disinfection process is not limited to the above description. For example, techniques of nonactivations of the target viruses 60 using ultraviolet (UV)-based photocatalysis, etc. can be used. As to the following description of several disinfection processes, any other disinfection processes can be adapted similarly.

### --ESTIMATION OF ASSAY SENSITIBITY BY FUNDAMENTAL EMBODIMENT--

The assay sensitivity by using the virus test device, the virus test system and the virus test method of the fundamental embodiment will be described below, as the example of the case of using the ball SAW sensor 1003 illustrated in FIG. 1 and FIG. 2, etc. At first, a sensitivity of the ball SAW sensor 1003 is converted to a sensitivity of a SAW resonator using a ST-cut crystal-substrate. That is, by substituting physical constants of the crystal substrate to the theoretical formula, an estimate equation for the mass-loading sensitivity S is obtained as follows: $S = Δf / {Δm}_{s} = 1.32 {f}^{2}$ (1). Here, Δf (Hz) is a change of the resonance frequency, Δmₛ (µg/cm²) is a difference of areal densities by weight by the mass-loading, and f (MHz) is a frequency. In the case of f = 100 MHz, the mass-loading sensitivity S becomes as follows: $S = Δf / {Δm}_{s} = 1.32 \times {10}^{4} \left(Hz/\left(μg/{cm}^{2}\right)\right)$ (2).

In the ball SAW sensor 103, as the difference-integral detection of the delay times is executed, the relative sensitivity is 0.01 ppm. Converting the value of the relative sensitivity to the change of the resonance frequency, Δf=1 Hz is obtained. Then, the detection limit of the difference Δmₛ of the areal density by weight becomes as follows: ${Δm}_{s} = 75.8 pg / {cm}^{2} = 758 fg / {mm}^{2}$ (3).

Here, if we assume that the target viruses are influenza viruses, the mass M per one virus becomes as follows: $M = 0.8 fg$ (4). Then, the detection limit S becomes 758 fg / 0.8 fg/mm²=948 pieces/mm², using Eq. (2).

The virus concentration in an expiration of the influenza infected person is known to be 67 to 8500 pieces/L (see NPTL 2). Then, a possibility of detecting the viruses existing with such lower concentration, using the ball SAW sensor 1003 having the above detection limit, will be considered below.

In the detection of the viruses in the air by using the virus test system pertaining to the fundamental embodiment, for example, the AUT 31a is ejected to the pseudo-receptor film 13 of the ball SAW sensor 1003, after the AUT 31a is transported by high-speed air-flow, through the first concentrating-mechanism 34a having a nozzle-shape illustrated in FIG. 11. Assuming that a flow rate at a gas-entrance side of the ejection nozzle is 1 (L/min), and that an opening cross-sectional area at the inlet-canal of the first concentrating-mechanism 34a is 1 mm², the flow velocity v at the inlet-canal of the first concentrating-mechanism 34a becomes as follows: $v = 16.7 m / s$ (5). Here, the compression of the air is not considered.

In addition, if an average concentration nₚ of the viruses in the AUT 31a inside the first concentrating-mechanism 34a is assumed as: ${n}_{p} = {10}^{4} pieces / L = 10 pieces / mL = 10 \times {10}^{- 3} pieces / μL$ (6). The inflow amount V_{D} per second of the viruses, which is injected to the pseudo-receptor film 13 of the ball SAW sensor 1003, through the inlet-canal with the opening cross-sectional area 1 mm² in the first concentrating-mechanism 34a becomes: ${V}_{D} = 167 pieces / s$ (7).

Therefore, for example, the target viruses of 16700 pieces will be introduced on the pseudo-receptor film 13 of the ball SAW sensor 1003, if the AUT 31a is ejected from the first concentrating-mechanism 34a with a period of 100 seconds. In addition, assuming the capturing efficiency of the viruses on the pseudo-receptor film 13 is 50%, the viruses captured number in the period of 100 seconds after an instant when the gas is introduced, is 8350 pieces. The captured number is larger than the above estimated number of 948 pieces as the detection limit. And further, the signal-to-noise ratio is calculated as S/N = 8350/948 = 8.8. Therefore, we understand that the assay sensitivity of the virus test device pertaining to the fundamental embodiment is enough to inspect whether the viruses exists or not.

If the ejecting period is referred as "the concentration process, or the concentration period", the in-situ measurement of the target viruses 60 can be executed easily with a higher sensitivity, through a shorter concentration process of one minute to two minutes, according to the virus test system pertaining to the fundamental embodiment. As the result, since the in-situ measurement of the target viruses can be executed easily, quickly with a higher sensitivity, an in-situ measurement of the viruses in the exhaled breath becomes possible, and therefore, the risk degrees of aerial infections, etc. can be anticipated.

### --VIRUS TEST PROGRAM FOR FUNDAMENTAL EMBODIMENT--

The virus test system pertaining to the fundamental embodiment of the present invention is applied to a virus test program configured to execute the in-situ measurement of the target viruses 60, by using the above-mentioned architecture. The above-mentioned architecture may encompasses the detector-substrate 11a, the pseudo-receptor film 13 mounted on at least a partial area of the detector-substrate 11a, the virus introducing-tube-A configured to eject the aerosols-under-test 33b on the pseudo-receptor film 13 by sucking the aerosols-under-test33b, and the moist-air introducing-tube- B configured to supply the cleaned moist-air to the pseudo-receptor film 13 through the virus introducing-tube-A, by connecting to the middle bifurcation node of the virus introducing-tube-A. That is, the virus test program drives and controls the operations of a computer, by transmitting a sequence of instructions embracing humidification instructions configured to eject the cleaned moist-air passed from the moist-air introducing-tube- B to the pseudo-receptor film 13, ejecting instructions configured to eject the cleaned moist-air with the aerosols-under-test 33b on the pseudo-receptor film 13 by mixing the aerosols-under-test 33b with the cleaned moist-air, and inspection instructions configured to execute the difference-integral detection for inspecting whether the target viruses exists or not in the aerosols-under-test 33b, ascribable to the target viruses bound to the pseudo-receptors 14 on the pseudo-receptor film 13.

The virus test program pertaining to the fundamental embodiment can be executed, after reading out the subject virus test program from the program memory 512 in the signal processor 50 illustrated in FIG. 9. Or, alternatively, the virus test program may be stored normally in the storage portion of the main memory, etc., other than the program memory 512 in the signal processor 50 of the virus test system pertaining to the fundamental embodiment. Furthermore, the virus test program may be downloaded from an external network or through a computer-readable storage-medium, and then, the virus test program is transferred to the storage portion of the main memory, etc., in the signal processor 50 or to the program memory 512, and thereafter, the virus test program may be read out for executing the sequence of the processes. In addition, the virus test program pertaining to the fundamental embodiment can be achieved by a circuit, for example, an application specific integrated circuit (ASIC), which can provide one or more functional capabilities.

The virus test program pertaining to the fundamental embodiment may be stored, for example, in an external memory or a computer-readable storage-medium, etc. Here, the data processor 500 can execute the assay processes such that whether the spike-protein receptor-bindings 19 is present or not present, depending on the sequence of the instructions described in the virus test program, by reading out the virus test program stored in the external memory, the computer-readable storage-medium, etc., to the program memory 512. Here, "the computer-readable storage-medium" means storage-mediums which can store any virus test program, such as external memory units of the computer, a semiconductor memory, a magnetic disc, an optical disc, a magnetooptical disc, or a magnetic tape, etc.

### (FIRST DERIVED-EMBODIMENT)

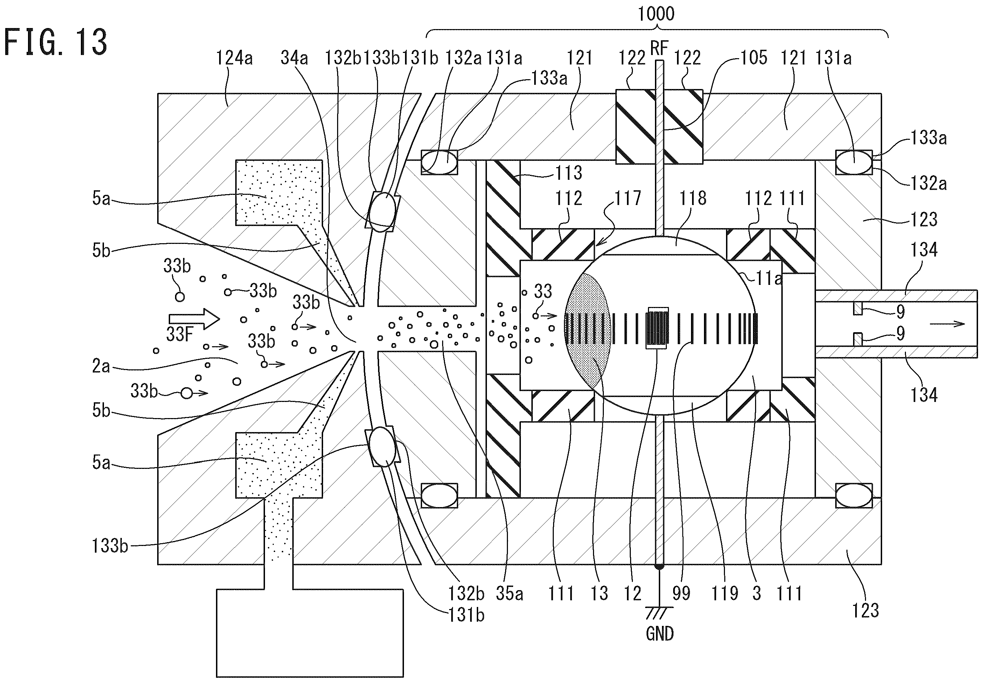

Next, a first derived-embodiment of the present invention which extends the technical concept of the above fundamental embodiment will be described below. The virus test device pertaining to the first derived-embodiment of the present invention has the similar structure as the structure illustrated in FIG. 3, as illustrated by FIG. 13, as to the structural features which embraces an inlet-canal 35a at one side (the left side in FIG. 13) of the detection vessel 10 accommodating the ball SAW sensor 1003, and an exhaust tube 134 at the other side (the right side in FIG. 13) being opposite to the inlet-canal 35a. A detection cell 1000 of the virus test device pertaining to the first derived-embodiment encompasses, as represented by FIG. 13, the ball SAW sensor 1003 which has been illustrated in FIG. 2, and a detection vessel 10 housing the ball SAW sensor 1003. Here, in the following description, the ball SAW sensor 1003 which has been illustrated in FIG. 2 is represented as "the ball SAW sensor (11a, 12, 13, 14)", for convenience, and the symbol 1003 is omitted in the corresponding drawings. An eject-side edge of the virus introducing-tube-A is connected to the inlet-canal 35a of the detection vessel 10 from the left side of FIG. 13. A suction pump 40 recited in FIG. 1 is connected to an exhaust tube 134 of the detection vessel 10 at the right side in FIG. 13. The detection vessel 10 has a hollow structure, and the ball SAW sensor (11a, 12, 13, 14) is provided in the detection vessel 10. A detector-substrate 11a is spherical, and as recited in FIG. 2, a partial area or entire portion of the surface of the detector-substrate 11a is covered by a pseudo-receptor film 13.

As illustrated in FIG. 13, the detection vessel 10 encompasses a rectangular hermetic chamber embracing a metallic encapsulating box 123 which is opened at the ceiling portion, and a metallic lid 121 which closes the upper portion (the ceiling portion) of the encapsulating box 123. An O -ring groove 132a is engraved as the U-groove at the uppermost edge of the encapsulating box 123 for accomplishing the hermetic chamber, so as to surround the upper portion of the encapsulating box 123, and an O-ring 131a is fitted in the O-ring groove 132a. An O-ring groove 133a is engraved as the U-groove at the bottom plane of the lid 121 by a topology corresponding to the O-ring groove 132a. An orifice 9 is disposed inside an exhaust tube 134 which is provided at the other side being opposite to the inlet-canal 35a of the encapsulating box 123. One portion of the virus introducing-tube-A schematically (conceptionally) recited in FIG. 1 is implemented by a first concentrating-mechanism 34a and the inlet-canal 35a is merged with the first concentrating-mechanism 34a, and a clump of aerosols-under-test 33b passed through a filter 30a in the configuration recited in FIG. 1 are ejected as a high-speed air-flow toward the pseudo-receptor film 13 of the ball SAW sensor (11a, 12, 13, 14) housed in the detection vessel 10.

However, the detection vessel 10 of the virus test device pertaining to the first derived-embodiment can be made of a metallic block. A feature of quadruple rooms, which embraces an inlet-canal 2a of the aerosols-under-test 33b, a hollow space 3 for cell-enclosure the ball SAW sensor (111a, 12, 13, 14), an exhaust tube 134 implementing an exhaust-canal of the aerosols-under-test 33b, and a set of cavities 5a, 5b for generating the sheath flow-F with the collimation-focusing capability, is different from the triple-room structure illustrated in FIG. 3. In the detector-substrate 11a, a north-pole electrode of the detector-substrate 11a is connected to an external-electrode 105 as a RF electrode in the hollow space 3, and a south-pole electrode of the detector-substrate 11a is connected to a ground (GND) electrode. A belt of the equational zone is fixed, being parallel to a straight line connecting the inlet-canal 2a and the exhaust tube 134 as the exhaust-canal. A signal conditioner 12 encompassing an interdigital electrode is provided on the equational zone surrounding the detector-substrate 11a, and a pseudo-receptor film 13 for detecting the target viruses is also coated on a specific location, which includes at least a partial area of the equational zone. The signal conditioner 12 converts physical signals representing variations of the physical states of the pseudo-receptor film 13, ascribable to the specific bindings of the pseudo-receptors 14 to the target viruses, to the electric signals. In addition, the arithmetic and logical operations, configured to measure the attenuation coefficients of the SAW and the changes of the delay times of the SAW, are executed utilizing the electric signals transmitted from the signal conditioner 12, by using the signal processor 50.

The cavity 5b is a thin space as a flow channel, which is surrounded by outer and inner conic surfaces, the outer and the inner conic surfaces having different gradients, respectively. And the cavity 5b surrounds a tip of the first concentrating-mechanism - -a virus-concentrating-mechanism-- 34a. The first concentrating-mechanism 34a and the inlet-canal 35a cut in the wall of the cell-enclosure (chassis) can implement a hermetic structure, with a mechanism of an O-ring 131b, such that the first concentrating-mechanism 34a facilitates a rotational-motion relatively to the position of the inlet-canal 35a, like the architecture of the virus test device pertaining to the first modification of the fundamental embodiment, which has been illustrated in FIG. 7. Although the illustrations are omitted, nozzles of a second concentrating-mechanism and a third concentrating-mechanism could be also prepared in addition to the first concentrating-mechanism 34a, and then, a rotational-motion of the inlet-canal 35a, cut in the wall of the cell-enclosure, to the positions of the nozzles of the second concentrating-mechanism and the third concentrating-mechanism, relatively is possible in the configuration illustrated in FIG. 13. A scheme of triple dedicated nozzles can be prepared also, as with the structure illustrated in FIG. 7, in the virus test device pertaining to the first derived-embodiment. Then, a sequential exchange of triple high-speed air-flows is possible, automatically addressing to independent purposes, respectively, by elaborating the high-speed fluid-flows using the triple dedicated nozzles respectively.

However, the structure illustrated in FIG. 13 can be replaced by the configuration of the virus test device pertaining to the fundamental embodiment illustrated in FIG. 3, in which the multipurpose-common concentrating-mechanism 34 and the inlet-canal 35a of the encapsulating box 123 are mated hermetically, by using the mechanism of the O-ring 131b. Focusing to the structure of the virus introducing-tube, the aerosols-under-test 33b are introduced from the inlet-canal 2a through the first concentrating-mechanism 34a, surrounded by the sheath of the air --the sheath flow-which is spurted from the cavity 5b. Therefore, a beam of the aerosols-under-test 33b is converged to a narrower stream of the aerosols-under-test 33b, and the converged beam of the aerosols-under-test 33b are ejected to the belt of the equational zone on the ball SAW sensor (11a, 12, 13, 14). The structure of the virus test device pertaining to the first derived-embodiment represents a concreate-operative example of the nozzle structure, which has the dual-wall structure having the main flow and the sheath flow surrounding the main flow, already recited in FIG. 11B. The aerosols-under-test 33b can be analyzed further efficiently, by the structure of the virus test device pertaining to the first derived-embodiment illustrated in FIG. 13. Furthermore, the sheath flow ejected from the cavity 5b can be accelerated, by using the cavity 5a and a pressure build-up pump connected to the cavity 5a by the virus test device pertaining to the first derived -embodiment. Therefore, the jet of the aerosols-under-test 33b adjacent to the sheath flow can be accelerated.

Although the illustration is omitted, a suction pump such as vacuum pump, etc., is connected to an exhaust tube 134 implementing the virus test device pertaining to the first derived-embodiment, like the configuration already exemplified in FIG. 1. Therefore, the AUT 31a is evacuated from the inlet-canal 2a, after starting the operation of the suction pump. A small orifice 9 is provided at an exhaust-canal. The orifice 9 has a capability for controlling the amount of the AUT 31a in the process of sucking. And, detoxifications of the target viruses 60 can be executed in a path between the exhaust-canal and the suction pump, through UV-light irradiations with UV-light LEDs, or UV semiconductor lasers, etc. Furthermore, the aerosols-under-test 33b can be retrieved, by providing a bubbler in the path between the exhaust-canal and the suction pump. A narrow jet of the aerosols-under-test 33b are ejected toward the equator of the detector-substrate 111a from a tip of the first concentrating-mechanism 34a, since the inlet-canal 2a has a topology of tapered nozzle, in which the diameter becomes smaller toward the termination gradually. Regarding the configurations of the moist-air introducing-tube, the dry-air bypass-tube, and the purge-gas introducing-tube, etc., the technical concept is the same as the virus test device pertaining to the first modification of the fundamental embodiment, which has already been illustrated in FIG. 7.

### (SECOND DERIVED-EMBODIMENT)

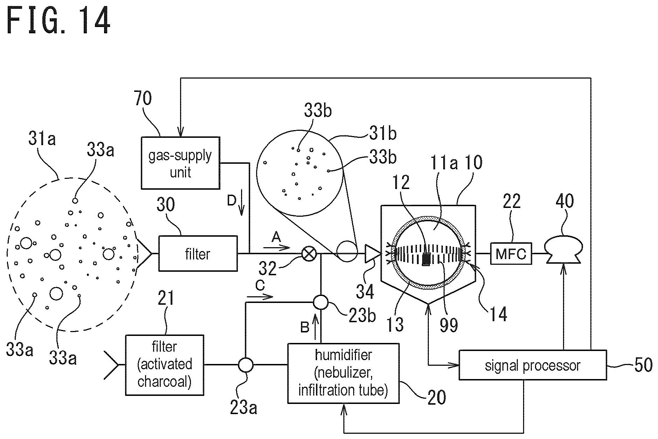

Furthermore, a second derived-embodiment of the present invention which extends the technical concept of the above fundamental embodiment will be described below. In a virus test device pertaining to the second derived-embodiment of the present invention, a MFC 22 is connected between a detection vessel 10 and a suction pump 40, as a conceptual diagram is illustrated schematically in FIG. 14. The flow rates of the air 31b under test, which is introduced via the virus introducing-tube-A, the cleaned moist-air, which is introduced from the moist-air introducing-tube-B , the cleaned dry-air, which is introduced from the dry-air bypass-tube-C, and the purge gas or the disinfecting gas, which is introduced from the purge-gas introducing-tube-D can be controlled by single MFC 22. Therefore, it is possible to realize the virus test device with the miniaturized size, having the high-performance, according to the virus test system pertaining to the second derived-embodiment.

In the virus test system pertaining to the second derived-embodiment, a signal processor 50 executes the difference-integral detection, as with the virus test system pertaining to the fundamental embodiment. If performance-decline of the activation of antigen-antibody reaction in the pseudo-receptors 14 occurs, that is, the ability of the specific-binding reactions between the viruses and the pseudo-receptors 14, etc. has been impaired, it is necessary to replace the detection cells 1000 (see FIG. 3 and FIG. 13). For example, a scheme of preparing a plurality of backup detection cells in other room isolated from the inspection room, and the user can replace the performance-declined detection cell 1000 to anew backup detection cell, with a noncontact handling, can be adopted. For example, an architecture facilitating the automatic replacement of the performance-declined detection cell with the rotational movement, etc., may be acceptable. In addition, a scheme of wrapping a subject portion of the used detection cell with a noncontact handling method, the subject portion has a possibility of contamination, when exhausting the used detection cell to the outside of the virus test system, can be employed. The subject portion having the possibility of contamination may be the pseudo-receptor film 13, for example.

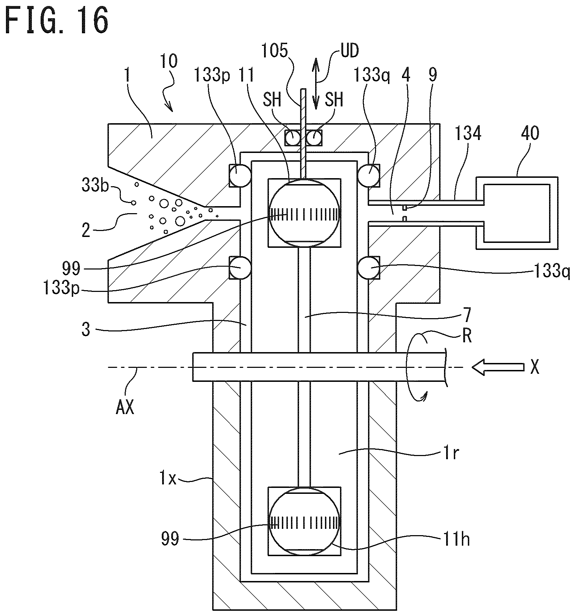

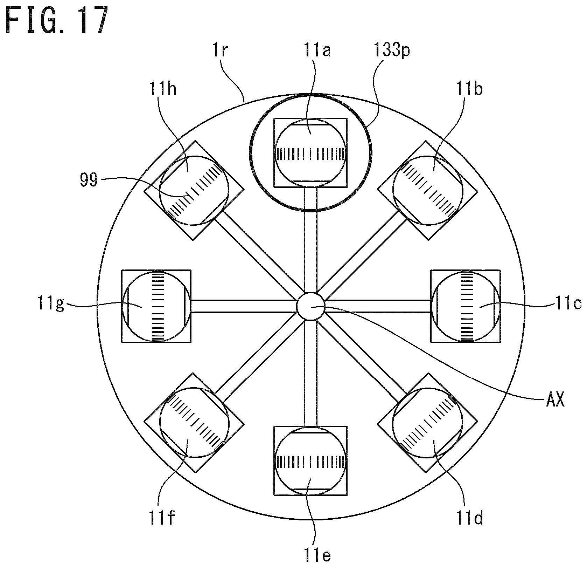

As illustrated in FIG. 17, the virus test device pertaining to the second derived-embodiment has a disk-based rotary cell-enclosure 1r in which octuple spherical detector-substrates are supposed to be housed. The octuple spherical detector-substrates include a first detector-substrate 11a, a second detector-substrate 11b, a third detector-substrate 11c, ......, and an eighth detector-substrate 11h. Each of the first detector-substrate 11a, the second detector-substrate 11b, the third detector-substrate 11c, ......, and the eighth detector-substrate 11h is implemented by a ball SAW sensor, which has the similar structure of the configuration illustrated in FIG. 2 respectively. Therefore, the detailed illustrations of the configurations of the first detector-substrate 11a, the second detector-substrate 11b, the third detector-substrate 11c, ......, and the eighth detector-substrate 11h are omitted. In the descriptions of FIG. 16 and FIG. 17, the first detector-substrate 11a, the second detector-substrate 11b, the third detector-substrate 11c, ......, and the eighth detector-substrate 11h represent the first ball SAW sensor a, the second ball SAW sensor b, the third ball SAW sensor c, ......, and the eighth ball SAW sensor h, respectively. Furthermore, the interdigitated electrodes are provided as the signal conditioners on the belt of the equational zones of the first detector-substrate 11a to the eighth detector-substrate 11h, respectively. And the pseudo-receptor films 13, each of which detects the target viruses 60, are coated respectively on subject locations, each of which includes at least a partial area of the equational zone. However, the illustrations of the signal conditioner 12 and the pseudo-receptor films 13, etc., are omitted in FIGs. 16 and 17. In addition, although the virus test device pertaining to the second derived-embodiment is supposed to use the handle-attached susceptors 1001 illustrated in FIG. 4 to FIG. 6, which are explained in the descriptions of the virus test device pertaining to the fundamental embodiment, the illustrations of the susceptors with the handle are omitted in FIGs. 16 and 17.

A view taken along the X-direction in FIG. 16 corresponds to the structure represented by FIG. 17, while FIG. 16 is a cross-sectional view of the virus test device pertaining to the second derived-embodiment. As illustrated in FIG. 17, octuple spherical detector-substrates, including the first detector-substrate 11a, the second detector-substrate 11b, the third detector-substrate 11c, ... ..., and the eighth detector-substrate 11h, are arranged respectively on a circumference of a circle defined in the rotary cell-enclosure 1r, being separated by an equal interval on the circumference. The octuple spherical detector-substrates are located on top of each of octuple radial lines, the radial lines are shifted by 45 degrees each other. As illustrated in FIG. 16, the detection vessel 10 has a hollow space 3 defined in the detection vessel 10. And, the rotary cell-enclosure 1r is accommodated hermetically in the hollow space 3, so that the detection vessel 10 can rotates along circular-direction R, with respect to a center at a rotation axis AX. At the left upper portion of FIG. 16, an empty space for an inlet-canal 2, which has a shrinking tapered topology, is provided to implement the common concentrating-mechanism at an exit edge of a path by the virus introducing-tube-A. Although the illustration is omitted in FIG. 16, the filter 30 shall be provided at an entrance edge of the path by the virus introducing-tube-A, as represented by FIG. 14. In addition, a piping system, including an on-off valve 32, a humidification-input valve 23a and a humidification-output valve 23b, etc., is provided between the output edge and the filter 30, so that the aerosols-under-test 33b made by hydrous aerosols can be ejected.

The suction pump 40 such as a vacuum pump, etc., is connected to an exhaust-canal 4 illustrated at the right upper portion of FIG. 16, and the aerosols-under-test 33b are introduced from the inlet-canal 2, by starting the operation of the suction pump 40. A small orifice 9 is provided at the exhaust-canal 4. The orifice 9 has a capability for controlling the amount of the aerosols-under-test 33b in the process of sucking. And, detoxifications of the target viruses 60 can be executed in a path between the exhaust-canal 4 and the suction pump 40, through UV-light irradiations with UV-light LEDs, or UV semiconductor lasers, etc. Furthermore, the aerosols-under-test 33b can be retrieved, by providing a bubbler in the path between the exhaust-canal 4 and the suction pump 40. As illustrated in FIG. 16, the left upper surface of the rotary cell-enclosure 1r and the right-edge plane at the eject-side edge of the virus introducing-tube-A, the right-edge plane being opposite to the left upper surface of the rotary cell-enclosure 1r, implements a hermetic structure by an O-ring 133p.

Furthermore, the right upper surface of the rotary cell-enclosure 1r and the inner vertical-wall of the hollow space 3, which is opposite to the right upper surface of the rotary cell-enclosure 1r, and through which the exhaust-canal 4 is cut for the suction pump 40, also implements a hermetic structure by an O-ring 133q. That is, even if the rotations of the first detector-substrate 11a to the eighth detector-substrate 11h are generated, the hermetic structure can keep a gas-leak-free performance. When the first detector-substrate 11a to the eighth detector-substrate 11h are rotated along the circular-direction R, the rod-like external-electrode 105 contacted to each of the north-pole electrodes of the first detector-substrate 11a to the eighth detector-substrate 11h executes slide-movements along an up-down direction UD illustrated in FIG. 16 so that the rod-like external-electrode 105 can be separated from the first detector-substrate 11a to the eighth detector-substrate 11h, respectively, facilitating the rotation of the rotary cell-enclosure 1r. Therefore, an O-ring SH is provided at a sliding portion of the external-electrode 105, facilitating the execution of the slide movement of the external-electrode 105, while keeping the hermetic structure. In addition, it is necessary to implement a split structure, configured to break the detection vessel 10 in two, for inserting the rotary cell-enclosure 1r in the hollow space 3 of the detection vessel 10. Therefore, a hermetic seal member such as an O-ring, etc., shall be incorporated in the split structure, of course.

Any of the first detector-substrate 11a to the eighth detector-substrate 11h is set to be a fixed state, when one of the positions of the first detector-substrate 11a to the eighth detector-substrate 11h which is allocated at a mating position, or is allocated at an exit side of the path of the virus introducing-tube-A, being just opposite to the inlet-canal. In any of the fixed state, a north-pole electrode of one of the first detector-substrate 11a to the eighth detector-substrate 11h is contacted to the external-electrode 105 in the hollow space 3, and a south-pole electrode, which is the counterpart of the north-pole electrode contacted to the external-electrode 105, is contacted to ground potential in the one of the first detector-substrate 11a to the eighth detector-substrate 11h. Thereby, in the fixed state, a center line penetrating the belt of the equational zone of a sphere, the sphere having the north-pole electrode contacted to the external-electrode 105 and the south-pole electrode contacted to ground potential, becomes parallel to a straight line connecting the inlet-canal 2 and the exhaust-canal 4, when any one of the first detector-substrate 11a to the eighth detector-substrate 11h is rotationally moved to be located at the opposite location to the inlet-canal, which is provided at the eject-side edge of the virus introducing-tube-A. Furthermore, the detector-substrate approaching to end of detector's usefulness is replaced by other one of the first detector-substrate 11a to the eighth detector-substrate 11h, by rotating the rotary cell-enclosure 1r as to the center of the rotation axis AX, if anyone of the first detector-substrate 11a to the eighth detector-substrate 11h prepared for virus test is measured to have little time left to be used.

The disk-based rotary cell-enclosure 1r is a block made of resin or metal. The detection vessel 10 has triple rooms, which embraces the inlet-canal 2 of the aerosols-under-test 33b, the hollow space 3 for arranging the rotary cell-enclosure 1r, and the exhaust-canal 4 for the aerosols-under-test 33b. The inlet-canal 2 implements a common concentrating-mechanism having a nozzle structure, in which the diameter of the nozzle becomes smaller toward the termination gradually. And, a narrow jet of the aerosols-under-test 33b is ejected from the tip of the common concentrating-mechanism toward the equator of any sphere of the first detector-substrate 11a to the eighth detector-substrate 11h, by rotating the rotary cell-enclosure 1r. It is possible to analysis the aerosols-under-test 33b efficiently, by ejecting the aerosols-under-test 33b. Regarding the other features, since the constitution or ordonnance is the same as the virus test device pertaining to the fundamental embodiment, duplicate explanations are omitted.

In the virus test device pertaining to the second derived-embodiment, the user-side processes of the accurate alignment adjustments of the north-pole electrode, the south-pole electrode, and the belt of the equational zone of the first detector-substrate 11a to the eighth detector-substrate 11h becomes unnecessary, by using handle-attached susceptors, as with the virus test device pertaining to the fundamental embodiment. Therefore, the objective of the virus test device pertaining to the second derived-embodiment is addressing to more and more easy handlings of the sensor unit, which includes the first detector-substrate 11a to the eighth detector-substrate 11h. Then, a rotary cell-enclosure 1r accommodating octuple ball SAW sensors, in which the alignment adjustments to the first detector-substrate 11a to the eighth detector-substrate 11h are already completed respectively, can be assembled as "a sensor unit", so that the sensor unit can be sold as a commercial product.

### --TEST METHOD BY SECOND DERIVED-EMBODIMENT--

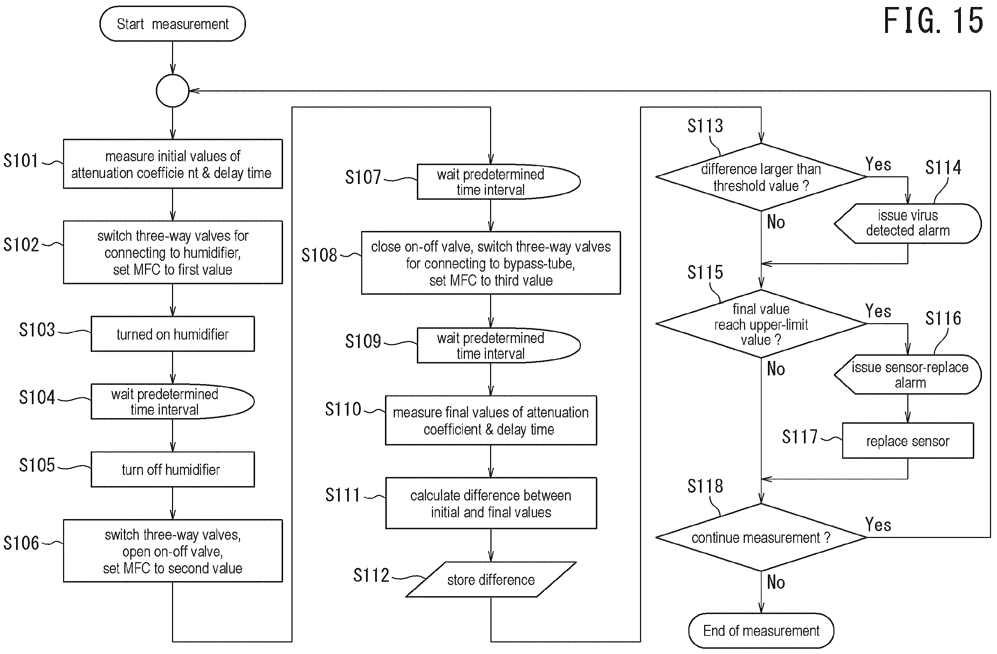

Next, a virus test method pertaining to the second derived-embodiment will be described with reference to the flow chart illustrated in FIG. 15. At first, after the virus test system of the second derived-embodiment has started, in a step S101 illustrated in FIG. 15, when a starting button of the difference-integral detection is pushed, initial values of attenuation coefficient and delay time of the ball SAW sensor (11a, 12, 13, 14), at a starting time of the difference-integral detection are measured, by the inspection module (logical circuit) 503 in the signal processor 50. The inspection module 503 stores the initial value acquired by the step S101 in the data memory 511 (see FIG. 9). Next, in a step S102 represented in FIG. 15, the moist-air introducing-tube- B is selected by controlling the humidification-input valve 23a and the humidification-output valve 23b, which are three-way valves, respectively, defining flow-paths in the three-way valves, and further a flowrate through the MFC 22 is set to a predetermined first value. After that, in a step S103 illustrated in FIG. 15, after the humidifier 20 is turned on, a cleaned dry-air passed through the filter 21 changes to a cleaned moist-air by the aid of the humidifier 20, and further, the cleaned moist-air is supplied to the surface of the detector-substrate 11a, through the virus introducing-tube-A.

And, in a step S104 illustrated in FIG. 15, for example, a liquid film 61 such as an ultrathin film of water, etc., is coated on the surface of the pseudo-receptor film 13 by the cleaned moist-air (the vapor of water) from the humidifier 20, after waiting a predetermined time interval. A volume and a thickness of the liquid film 61, which is scheduled to be provided on the pseudo-receptor film 13, can be easily controlled by adjusting the humidity in the cleaned moist-air, by using an independently-prepared ball SAW sensor as a humidity-detection apparatus. Further, the volume and the thickness of the liquid film 61 can be easily controlled by setting a time-span for the step S104 with the controlled humidity in advance. In a step S105 illustrated in FIG. 15, the humidifier 20 is turned off, after elapsing a predetermined time interval from the turn-on instant of the humidifier 20.

Next, in a step S106 illustrated in FIG. 15, the on-off valve 32 is opened, after setting the humidification-input valve 23a and the humidification-output valve 23b, which are three-way valves, respectively, so that the flow-paths in the three-way valves will not select any of the moist-air introducing-tube- B and the dry-air bypass-tube-C. Then, in the step S106, the virus introducing-tube-A is selected, and further, in the step S106, the flowrate through the MFC 22 is set to a predetermined second value. As the result, the AUT 31a sucked from a suction opening is introduced into the virus introducing-tube-A via the filter 30a. Here, the filter 30a removes larger particles and coarse aerosols 33a, etc., which have sizes larger than the predetermined size, for example, four micrometers. Then, the air including the aerosols-under-test 33b having the predetermined size or the sizes smaller than the predetermined size, for example, about 0.1 micrometer to about three micrometers, is introduced only into the virus introducing-tube-A, so that only the filtered air can be led to the surface of the pseudo-receptor film 13.

And, after waiting a predetermined time interval, in a step S107 illustrated in FIG. 15, the aerosols are ejected to the surface of the pseudo-receptor film 13 as the high-speed air-flow, which is generated by the common concentrating-mechanism 34. If the target viruses 60 are contained in the aerosols, which are ejected on the surface, the target viruses 60 shall be caught by the pseudo-receptors disposed on the surface of the pseudo-receptor film 13. However, the humidifier 20 may be turned off at a timing after the step S107, on the contrary to the above process order by the description of the virus test method pertaining to the second derived-embodiment, in which the case that the humidifier 20 was turned off at the timing before the step S106 and the step S107. If the humidifier 20 is turned off at the timing after the step S107, the coarse aerosols 33a ejected to the surface of the pseudo-receptor film 13 shall be the aerosols-under-test 33b, which are made of the hydrous aerosols including the moisture, the specific-binding reactions are expected to be promoted more and more on the surface of the pseudo-receptor film 13 in the steps S106 and S107.

Next, in a step S108 illustrated in FIG. 15, the dry-air bypass-tube-C is selected by controlling the humidification-input valve 23a and the humidification-output valve 23b, after closing the on-off valve 32, and further the flowrate through the MFC 22 is set to a predetermined third value. As the result, the cleaned dry-air introduced through the filter 21 is ejected to the surface of the pseudo-receptor film 13 as the high-speed air-flow, which is produced by the common concentrating-mechanism 34. And, by continuing the process of ejecting the cleaned dry-air for a predetermined time interval, the liquid film 61 coated on the surface of the pseudo-receptor film 13 is removed, in a step S109 illustrated in FIG. 15. Furthermore, by continuing the ejection of the cleaned dry-air for the predetermined time, NSA material-viruses and materials other than the target viruses 60--, existing on the surface of the pseudo-receptor film 13, is also removed in the step S109. The time interval in the step S109 shall be defined as a length of time required for removing the NSA material to reach a level of state such that the sensor response is stabilized to keep feeding a specific constant value.

Next, in a step S110 illustrated in FIG. 15, the inspection module (logical circuit) 503 in the signal processor 50 measures final values of the attenuation coefficient and the delay time of the SAW, which has been propagated on the ball SAW sensor (11a, 12, 13, 14). Continuously, in a step S111 to a step S112 illustrated in FIG. 15, the inspection module 503 reads out the initial value acquired by the step S101 from the data memory 511. The inspection module 503 calculates the difference between the initial value acquired by the step S101 and the final value acquired by the step S110. The inspection module 503 stores the calculated difference in the data memory 511 (see FIG. 9) in the virus test system pertaining to the fundamental embodiment. And, in a step S113 to a step S114 illustrated in FIG. 15, if a difference between the initial value and the final value is judged to be larger than a predetermined threshold value by the inspection module 503, the inspection module 503 determines that the intended target virus 60 are existing in the AUT 31a. That is, if a response amount is judged to be larger than the predetermined threshold value due to the specific adsorptions of the target viruses 60, by the inspection module 503, the inspection module 503 send instructions to the output unit 513 so that the output unit 513 can issue "target-virus detected-alarm".

On the other hand, in the step 113 to the step 115 illustrated in FIG. 15, in the case of being judged that the difference between the initial value and the final value is equal to the threshold value or less, by the inspection module 503, the inspection module 503 judges that the intended target virus 60 does not exist in the AUT 31a, and the inspection module 503 continues the in-situ measurement of the intended target virus 60 in the AUT 31a until an assay-end button is pushed.

In the virus test method pertaining to the second derived-embodiment, since the specific-binding reactions are irreversible integral-reactions basically, the number of target viruses 60 which can specifically adsorb is limited. Therefore, in the step S115 to a step S116 illustrated in FIG. 15, if the final value in the step S110 is reached to an upper-limit value, the sensor-replace alarm is issued. And, in a step S117 illustrated in FIG. 15, the replace of the sensor is executed by rotating the rotary cell-enclosure 1r illustrated in FIGs. 16 and 17 automatically, or manually. In the process of the step S115 to a step S118 illustrated in FIG. 15, the sensor-replace alarm is stopped, when the replace of sensor is completed, or when the alarm-stop button is pushed. Then from the step S118, the in-situ measurement of the intended target virus 60 in the AUT 31a continues. In the step S118, when the assay-end button is pushed, the in-situ measurement of the intended target virus 60 is finished.

### (THIRD DERIVED-EMBODIMENT)

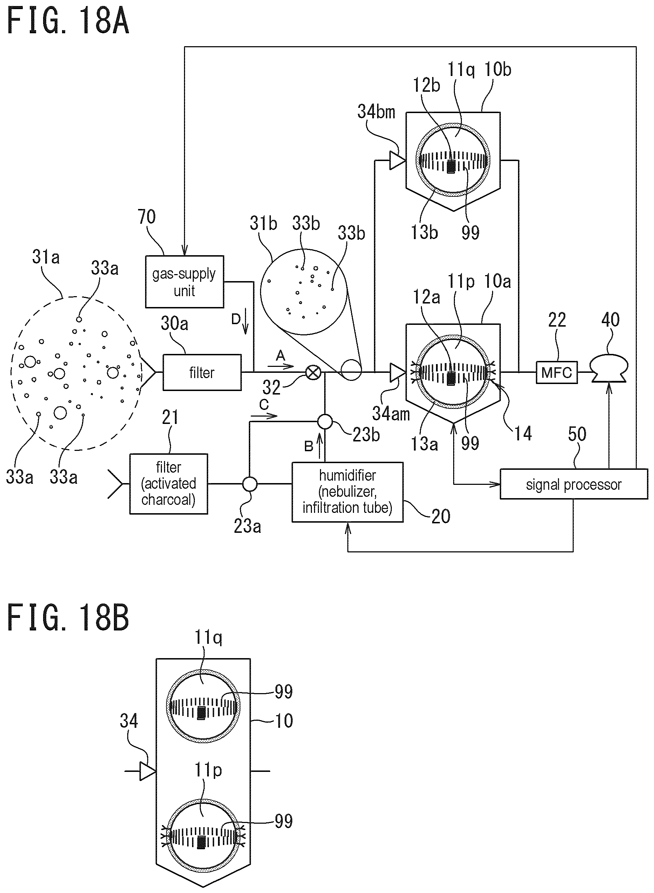

Furthermore, a third derived-embodiment of the present invention, which is derived from the above fundamental embodiment, extending the technical concept of the above fundamental embodiment, will be described below. In the virus test device pertaining to the third derived-embodiment of the present invention, a MFC 22 is connected between exhaust sides of a first detection vessel 10a and a second detection vessel 10b and a suction pump 40, as represented by FIG. 18. The virus test device pertaining to the third derived-embodiment encompasses a first ball SAW sensor (11p, 12a, 13a, 14) and a second ball SAW sensors (11q, 12b, 13b), which are accommodated in a first detection vessel 10a and a second detection vessel 10b respectively. The electric signals delivered from a first signal conditioner 12a of the first ball SAW sensors (11p, 12a, 13a, 14) and a second signal conditioner 12b of the second ball SAW sensors (11q, 12b, 13b) respectively, are transmitted to the signal processor 50. The virus test system pertaining to the third derived-embodiment has a feature of facilitating high-speed responses, by entering a couple of different electric signals obtained from the first signal conditioner 12a and the second signal conditioner 12b to a single signal processor 50, and by executing differential measurements with the couple of different electric signals, by using the signal processor 50.

The first detection vessel 10a houses the first ball SAW sensor (11p, 12a, 13a, 14) having a first signal conditioner 12a and a first pseudo-receptor film 13a. In the architecture of the first ball SAW sensor (11p, 12a, 13a, 14) exemplified in FIG. 18, the first signal conditioner 12a with an interdigital electrode converts the physical signals representing variations of the physical states of a pseudo-receptor film 13 due to the specific binding of the pseudo-receptors 14 to the target viruses, to the corresponding electric signals. The pseudo-receptors 14 being bound to the target viruses 60 are provided on the pseudo-receptor film 13a of a first detector-substrate 11p. On the other hand, a second detection vessel 10b houses a second ball SAW sensor (11q, 12b, 13b) having a second signal conditioner 12b and a second pseudo-receptor film 13b. As illustrated in FIG. 18, although a second signal conditioner 12b with the interdigital electrode is provided on the second ball SAW sensor (11q, 12b, 13b), the pseudo-receptors does not exist. The second pseudo-receptor film 13b of the second detector-substrate 11q is almost same as the first pseudo-receptor film 13a of the first detector-substrate 11p, except for a feature that the pseudo-receptors 14, being scheduled to be bound to the target viruses 60, are not provided on the second pseudo-receptor film 13b of the second detector-substrate 11q. That is, the second detector-substrate 11q is a sphere which implements a body of the reference sensor, having only a nonspecific-adsorption suppression-surface for suppressing the nonspecific-adsorption.

Air 31b under test, which is introduced via a virus introducing-tube-A is ejected to the first detector-substrate 11p in the first detection vessel 10a through the first concentrating-mechanism --a first common concentrating-mechanism-- 34am. And simultaneously, the air 31b under test, which is introduced via the virus introducing-tube-A is ejected to the second detector-substrate 11q in the second detection vessel 10b through the second concentrating-mechanism --a second common concentrating-mechanism-- 34bm. Or alternatively, cleaned moist-air, which is introduced via a moist-air introducing-tube- B or the cleaned dry-air, which is introduced via the dry-air bypass-tube-C is ejected to the first detector-substrate 11p in the first detection vessel 10a through the first concentrating-mechanism 34am, and simultaneously, the cleaned moist-air, which is introduced via the moist-air introducing-tube- B or the cleaned dry-air, which is introduced via the dry-air bypass-tube-C is ejected to the second detector-substrate 11q in the second detection vessel 10b through the second concentrating-mechanism 34bm. In the virus test device pertaining to the third derived-embodiment, the first ball SAW sensor (11p, 12a, 13a, 14) and the second ball SAW sensor (11q, 12b, 13b) may be, as represented by FIG. 18B, accommodated in the single detection vessel 10, when executing the differential measurement. As illustrated in FIG. 18B, in the case that the double sensors are accommodated in the single detection vessel 10, the first signal conditioner 12a of the first ball SAW sensor (11p, 12a, 13a, 14) has the interdigital electrode, and the second signal conditioner 12b of the second ball SAW sensor (11q, 12b, 13b) has also the interdigital electrode, similarly. In the architecture illustrated in FIG. 18B, the air 31b under test, the cleaned moist-air, or the cleaned dry-air may be ejected to the pseudo-receptor film 13a of the first ball SAW sensor (11p, 12a, 13a, 14) and to the pseudo-receptor film 13b of the second ball SAW sensor (11q, 12b, 13b) respectively, by the single common concentrating-mechanism 34. Regarding the other features, since the same is true for the virus test device pertaining to the fundamental embodiment, therefore, duplicated description is omitted.

### --TEST METHOD BY THIRD DERIVED-EMBODIMENT--

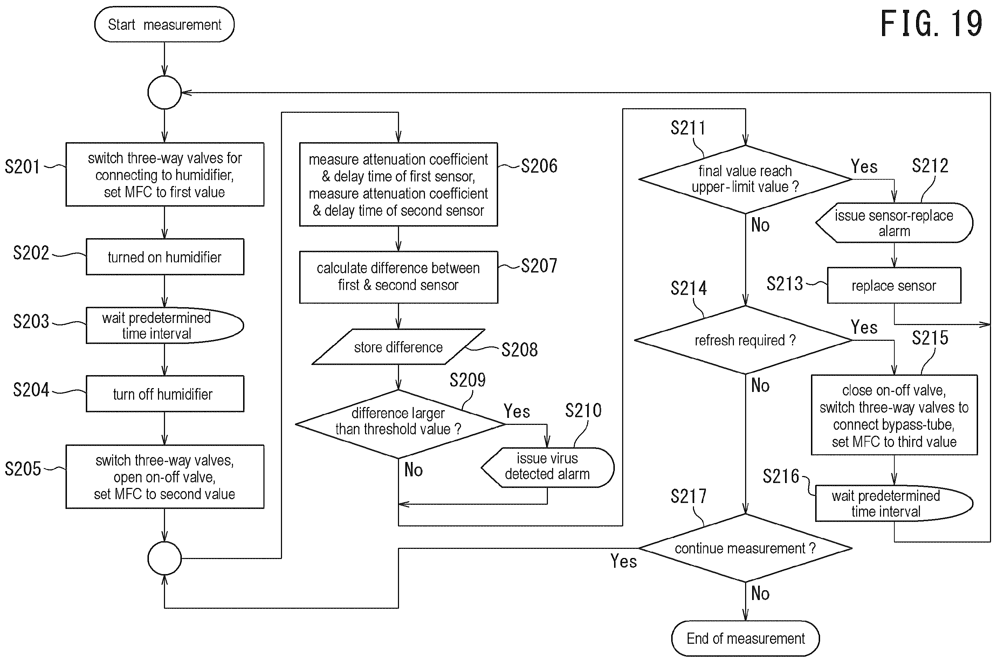

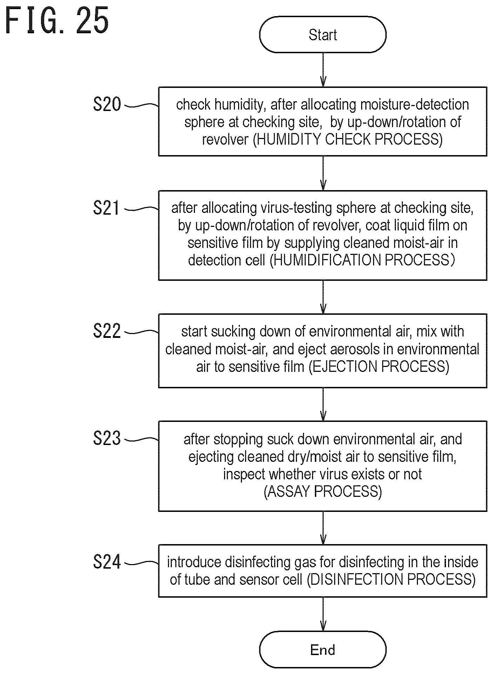

Next, a virus test method pertaining to the third derived-embodiment will be described with reference to the flow chart illustrated in FIG. 19. In the following, in the case of examining whether the intended target virus 60 exist or not in the AUT 31a, will be described, referring to the virus test system illustrated in FIG. 18A. Here, the procedures of virus test method of the third derived-embodiment, may be executed by the processes according to the flowchart of the virus test method pertaining to the fundamental embodiment, which has already been described using FIG. 12. Or alternatively, the virus test method pertaining to the third derived-embodiment will be executed by procedures according to a flowchart of a virus test method pertaining to a sixth derived-embodiment which will be illustrated in FIG. 25, scheduled to be described below.

At first, after starting the virus test device pertaining to the third derived-embodiment, when a measurement-start button is pushed in a step S201 illustrated in FIG. 19, the humidification-input valve 23a and the humidification-output valve 23b, which are three-way valves, are controlled respectively, so that flow-paths in the three-way valves illustrated in FIG. 18A can select the moist-air introducing-tube- B, and further, the flowrate through the MFC 22 is set to a predetermined first value. After that, in a step S202 illustrated in FIG. 19, after the humidifier 20 turns on, a cleaned dry-air passed through the filter 21 changes to a cleaned moist-air by the aid of the humidifier 20, and further, the cleaned moist-air is supplied to the surface of the pseudo-receptor film 13 through the virus introducing-tube-A.

And, after waiting a predetermined time interval, in a step S203 illustrated in FIG. 19, for example, a liquid film 61 such as an ultrathin film of water, etc., is coated on a surface of the pseudo-receptor film 13 by the cleaned moist-air -- vapor of water or aerosols-- from the humidifier 20. Regarding the estimation of volume or thickness of the liquid film 61, which is scheduled to be coated on the surface of the pseudo-receptor film 13, the humidity in the cleaned moist-air can be controlled, using another ball SAW sensor prepared independently as the humidity-detection apparatus. Then, the volume or the thickness of the liquid film 61 can easily adjusted, by setting a time span for the step S203. In a step S204 illustrated in FIG. 19, the humidifier 20 is turned off, after elapsing a predetermined time interval from the turn-on instant of the humidifier 20.

Next, in a step S205 illustrated in FIG. 19, the virus introducing-tube-A is selected, when the on-off valve 32 is opened, after setting the humidification-input valve 23a and the humidification-output valve 23b, which are three-way valves, respectively, so that the flow-paths in the three-way valves can selecting no route for the moist-air introducing-tube- B and the dry-air bypass-tube-C. Furthermore, the flowrate through the MFC 22 is set to a predetermined second value at the same time. As the result, the AUT 31a sucked from a suction opening is introduced into the virus introducing-tube-A via the filter 30a. Here, the filter 30a removes larger particles and coarse aerosols 33a, etc., which have sizes larger than the predetermined size, for example, four micrometers. Then, the air including the aerosols-under-test 33b having the predetermined size or the sizes smaller than the predetermined size, for example, about 0.1 micrometer to about three micrometers, is introduced only into the virus introducing-tube-A, so that only the filtered air can be led to the first ball SAW sensor (11p, 12a, 13a, 14) and the second ball SAW sensor (11q, 12b, 13b).

And, after waiting a predetermined time interval, if the target viruses 60 exists in the aerosols-under-test 33b, by ejecting the aerosols-under-test 33b to the surface of the first ball SAW sensor (11p, 12a, 13a, 14) as the high-speed air-flow from the first concentrating-mechanism 34am, the target viruses 60 shall be captured by the pseudo-receptors on the first pseudo-receptor film 13 of the first ball SAW sensor (11p, 12a, 13a, 14). Simultaneously, the NSA material --viruses and materials other than the target viruses-- as the impurities, which are included in the aerosols-under-test 33b, is also adsorbed on the first pseudo-receptor film 13a of the first ball SAW sensor (11p, 12a, 13a, 14). Simultaneously with the ejection to the surface of the first pseudo-receptor film 13a, in the step S205, the aerosols-under-test 33b are ejected to the surface of the second ball SAW sensor (11q, 12b, 13b) as the high-speed air-flow from the second concentrating-mechanism 34bm. As the result, in the step S205, NSA material as the impurities included in the aerosols-under-test 33b is also adsorbed on the second pseudo-receptor film 13b of the second ball SAW sensor (11q, 12b, 13b).

Furthermore, the impurities in the AUT 31a are equally adsorbed on the both of double pseudo-receptor films of the first pseudo-receptor film 13a of the first ball SAW sensor (11p, 12a, 13a, 14) and the second pseudo-receptor film 13b of the second ball SAW sensor (11q, 12b, 13b). And therefore, the changes of the acoustic signals caused by the adsorptions are converted to the corresponding electric signals respectively, by using the first signal conditioner 12a and the second signal conditioner 12b. The signal processor 50 calculates the initial values of the attenuation coefficient of the SAW and the delay time of the SAW respectively, by utilizing the electric signals which are transmitted by the first signal conditioner 12a and the second signal conditioner 12b respectively. The above scheme with the first signal conditioner 12a and the second signal conditioner 12b can eliminate the steps S101 and S108 -S109 illustrated in FIG. 15, which are required in the virus test method pertaining to the second derived -embodiment described with reference to FIG. 15. As stated above, in the virus test method pertaining to the second derived-embodiment, the initial values of the attenuation coefficient and the delay time of the SAW must be measured by the ball SAW sensor (11a, 12, 13, 14) in advance, via the signal processor 50 --the step S101 --, and furthermore, the removal step of the impurities --the steps S108 to S109-- is required. Therefore, the virus test method pertaining to the third derived-embodiment has an effectiveness that the higher speed virus test can be achieved, compared with the virus test method pertaining to the second derived-embodiment.

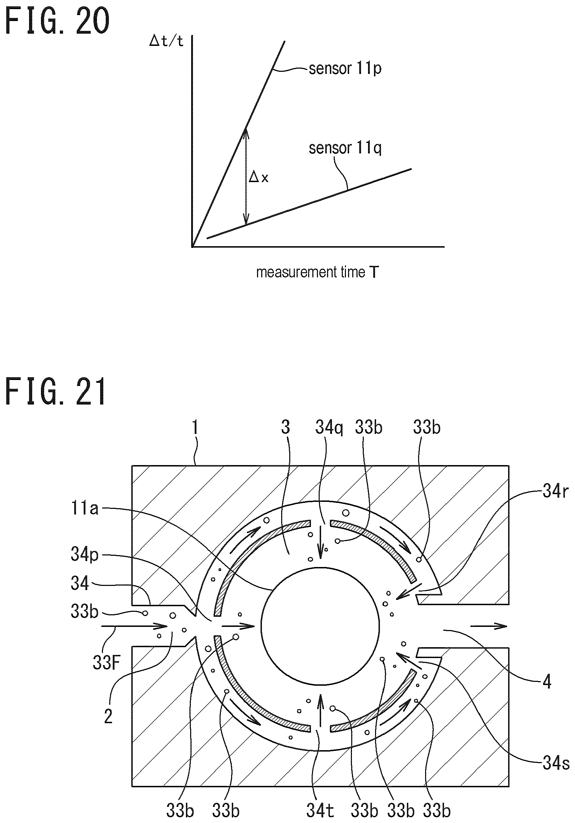

Concretely, the attenuation coefficient and the delay time of the SAW, which are transmitted by the first ball SAW sensor (11p, 12a, 13a, 14), are measured in a step S206 illustrated in FIG. 19. And, at the same time of the measurement of the first ball SAW sensor (11p, 12a, 13a, 14), the attenuation coefficient and the delay time of the SAW, which are transmitted by the second ball SAW sensor (11q, 12b, 13b), are measured. Continuously, in a step S207 to a step S208 illustrated in FIG. 19, the difference between the values measured by the first ball SAW sensor (11p, 12a, 13a, 14) and the second ball SAW sensor (11q, 12b, 13b) is calculated. And then, the differences are stored in the data memory 511 --see FIG. 9-- in the virus test system pertaining to the fundamental embodiment. As illustrated in FIG. 20, the difference Δx is an accurate response representing a correct amount of the target viruses 60 which does not depend on the impurities in the aerosols-under-test 33b.

Therefore, in accordance with a process-flow of a step S209 to a step S210 illustrated in FIG. 19, if a difference between the values measured by the first ball SAW sensor (11p, 12a, 13a, 14) and the second ball SAW sensor (11q, 12b, 13b) exceeds a threshold value, it is judged that the intended target virus 60 exists in the AUT 31a. That is, regarding the responses due to the specific adsorptions of the target viruses 60, if a response amount is larger than the predetermined threshold value, after the judgement that the intended target virus 60 exists in the AUT 31a, a target-virus detected-alarm is issued. On the other hand, in accordance with the process-flow of the step S209 to the step S210 illustrated in FIG. 19, if the differences between the values measured by the first ball SAW sensor (11p, 12a, 13a, 14) and the second ball SAW sensor (11q, 12b, 13b) are equal to or less than the threshold value, it is judged that the intended target virus 60 does not exist in the AUT 31a. And, the in-situ measurement of the target viruses 60 in the AUT 31a is continued, until an assay-end button is pushed.

In the virus test method pertaining to the second derived-embodiment, the case that the humidifier 20 was turned off before the process of step S205 has been explained. In contrast, in the virus test method pertaining to the third derived-embodiment, the humidifier 20 may be turned off after the process of the step S205, instead of the turning off before the process of the step S205. When he humidifier 20 is turned off after the process of step S205, the aerosols-under-test 33b ejected to the surfaces of the first ball SAW sensor (11p, 12a, 13a, 14) and the second ball SAW sensor (11q, 12b, 13b) become the aerosols-under-test 33b including the moisture.

In the specific-binding reactions in the virus test method pertaining to the third derived-embodiment, the number of the pseudo-receptors, or the number of the target viruses which are specifically adsorbed, has a limit value, because the specific-binding reaction is the irreversible integral-reaction basically, as with the second derived-embodiment. Therefore, if the values of the attenuation coefficient and the delay time which are measured by the first ball SAW sensor (11p, 12a, 13a, 14) in a step S206 are approaching to upper-limit values respectively, in processes of a step S211 to a step S212 illustrated in FIG. 19, a sensor-replace alarm is issued. And in a step S213 illustrated in FIG. 19, the replacement of the ball SAW sensor is executed automatically, or manually, by driving a rotary mechanism having the same architecture as the rotary cell-enclosure 1r illustrated in FIGs. 16 and 17, which have been described already as the virus test device of the second derived-embodiment. After the replacement of the ball SAW sensor is completed, in accordance with the flow of the step S213 to a step S201 illustrated in FIG. 19, the sensor alarm is stopped, and the in-situ measurement of the target viruses 60 in the AUT 31a continues.

Furthermore, in a step S214 illustrated in FIG. 19, if the amount of the NSA material of the first ball SAW sensor (11p, 12a, 13a, 14) and the second ball SAW sensor (11q, 12b, 13b) becomes larger, refreshing of the first ball SAW sensor (11p, 12a, 13a, 14) and the second ball SAW sensor (11q, 12b, 13b) can be executed. Here, the "refreshing" means a process for removing the NSA materials, which are adsorbed on the surfaces of the first ball SAW sensor (11p, 12a, 13a, 14) and the second ball SAW sensor (11q, 12b, 13b), so that the virus test device can restore to the initial clean state, in which the NSA materials do not exist. So, the effectiveness that the usefulness-spans, or the estimated usefulness periods of the first ball SAW sensor (11p, 12a, 13a, 14) and the second ball SAW sensor (11q, 12b, 13b) can be extended can achieved, by executing the above refreshing process. And specifically, as to the second ball SAW sensor (11q, 12b, 13b) serving as the reference sensor, labor or effort and time required for replacing the second ball SAW sensor (11q, 12b, 13b) are eliminated, because the replacement of the second ball SAW sensor (11q, 12b, 13b) is unnecessary.

Namely, in accordance with the flow of the step S214 to a step S215 illustrated in FIG. 19, if the refresh requirement is judged, after closing the on-off valve 32, controlling the humidification-input valve 23a and the humidification-output valve 23b to select the dry-air bypass-tube-C, and further, the flowrate through the MFC 22 is set to a predetermined third value. As the result, the cleaned dry-air introduced to the dry-air bypass-tube-C through the filter 21 is ejected on the surfaces of the first ball SAW sensor (11p, 12a, 13a, 14) and the second ball SAW sensor (11q, 12b, 13b) as high-speed air-flows from the first concentrating-mechanism 34am and the second concentrating-mechanism 34bm.

In a step S216 illustrated in FIG. 19, the above step of ejecting the cleaned dry-air on the surfaces of the first ball SAW sensor (11p, 12a, 13a, 14) and the second ball SAW sensor (11q, 12b, 13b) is continued for a predetermined time interval. Then, the liquid films on the surfaces of the first detector-substrate 11p and the second detector-substrate 11q are removed, and then, the NSA materials which exist on the surfaces of the first detector-substrate 11p and the second detector-substrate 11q are also removed. The time interval in the step S216 is defined as a time period required for removing the NSA material to reach a level of state that the sensor response keeps feeding a specific constant value. After that, obeying the flow of the step S216 to the step S201 illustrated in FIG. 19, the in-situ measurement of the target viruses 60 in the AUT 31a is continued.

By the way, if the refreshing process is unnecessary, the process flow advances to a step S217, and whether the difference-integral detection is continued or not is confirmed in the step S217. And, for example, the in-situ measurement of the intended target virus 60 is finished, when an assay-end button is pushed. On the contrary, the in-situ measurement of the target viruses 60 in the AUT 31a continues, when an assay-continue button is pushed (the step S217 to the step S206 illustrated in FIG. 19).

### (FOURTH DERIVED-EMBODIMENT)

A virus test device pertaining to a fourth derived-embodiment based on the fundamental embodiment relates to a configuration of a cell-enclosure 1 which can eject the aerosols-under-test 33b along with the almost entire of the belt of the equational zone, surrounding on the detector-substrate 11a, as recited in FIG. 21. The cell-enclosure 1 is a block made of resin or metal. The cell-enclosure 1 has triple rooms, which embraces an inlet-canal 2 for aerosols-under-test 33b, a hollow space 3 for accommodating the detector-substrate 11a implementing the ball SAW sensor, and an exhaust-canal 4 for the aerosols-under-test 33b. The detector-substrate 11a is provided in the hollow space 3, and the detector-substrate 11a is fixed in an orientation such that a centerline of the equational zone is parallel to a line connecting the inlet-canal 2 and the exhaust-canal 4.

Although the illustration is omitted, a suction pump such as vacuum pump, etc., is connected to the exhaust-canal 4, and the aerosols-under-test 33b are introduced via the inlet-canal 2 by starting the operation of the suction pump. Aerosols-under-test 33b are ejected from quintuple ejecting-canals including a first ejecting-canals 34p, a second ejecting-canals 34q, a third ejecting-canals 34r, a fourth ejecting-canals 34s, and a fifth ejecting-canals 34t, which are provided along the belt of the equational zone surrounding the detector-substrate 11a. However, a topology such that the aerosols-under-test 33b are ejected through the common concentrating-mechanism 34, toward the belt of the equational zone surrounding the detector-substrate 11a, is exemplified in FIG. 21. According to the virus test system pertaining to the fourth derived-embodiment, a capacity for the ingredient amount of the target viruses 60, which are bound to the surface of the pseudo-receptor film, can be increased relatively, because the aerosols-under-test 33b are ejected uniformly toward the pseudo-receptor film arranged along the equational zone. Therefore, the usefulness-span of the ball SAW sensor can be extended. By the way, the usefulness-span of the ball SAW sensor can be extended relatively, by providing gate structures to each of the quintuple ejecting-canals of the first ejecting-canals 34p to the fifth ejecting-canals 34t, and, on a time-series basis, by changing the ejecting locations to each of the pseudo-receptor films, which are provided along the equational zone.

### (FIFTH DERIVED-EMBODIMENT)

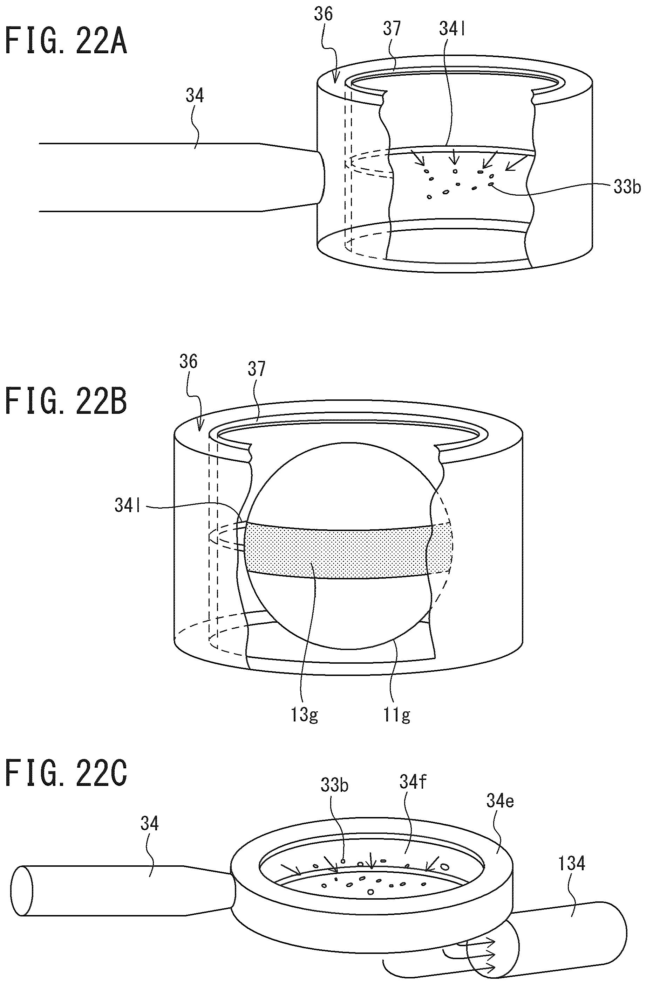

Each of virus test devices pertaining to a fifth derived-embodiment illustrated in FIGs. 22A, 22B and 22C encompasses a band-like slit for ejecting aerosols-under-test 33b to the pseudo-receptor film 13g, linearly along entire of a belt of equational zone surrounding a detector-substrate 11g, like the configuration illustrated in FIG. 21. As illustrated in FIG. 22A and FIG. 22B, each of the virus test devices further encompasses a plate-like cylindrical inner wall 37 surrounding the detector-substrate 11g, and an ejecting-slit 341 is provided at a middle level portion in a height of the inner wall 37 along the equator of the detector-substrate 11g. A nozzle edge of a common concentrating-mechanism is merged with an inlet-canal side in a cell-enclosure 1, and a gas-circumventing chamber 36 is provided between the nozzle edge and the inner wall 37.

Since the structure illustrated in FIG. 22A and FIG. 22B has the gas-circumventing chamber 36 with a large height, the gas-circumventing chamber 36 is susceptible to be contaminated easily. Therefore, as illustrated in FIG. 22C, a gas-circumventing chamber 34e isolated by an inner wall 34f with lower height shall be provided, so that a disinfection process and a cleaning process of the inner wall 34f of the gas-circumventing chamber 34e becomes easy, by reducing the volume of the gas-circumventing chamber 34e. An exhaust tube 134 is provided as illustrated at a lower-right side of FIG. 22C. Although the illustration is omitted, a suction pump such as vacuum pump, etc., may be connected to the right-side edge of the exhaust tube 134, so that the aerosols-under-test 33b can be ejected through the thin ejecting-slit, by starting the operation of the suction pump. According to the virus test system pertaining to the fifth derived-embodiment, since the aerosols-under-test 33b are ejected uniformly to the pseudo-receptor film 13g which is provided along the equational zone, a capacity for the ingredient amount of the target viruses 60, being bound to the surface of the pseudo-receptor film 13g, can be increased relatively. Therefore, the usefulness-span of the ball SAW sensor can be extended relatively by the virus test system pertaining to the fifth derived-embodiment.

### (SIXTH DERIVED-EMBODIMENT)

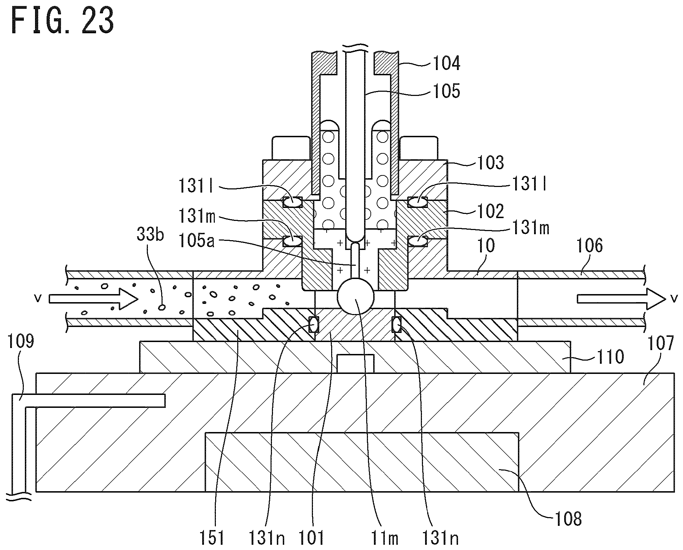

As illustrated in FIG. 23, a virus test device pertaining to a sixth derived-embodiment of the present invention derived from the fundamental embodiment, which has been mentioned in the opening section, encompasses a detection vessel 10 which accommodates a spherical detector-substrate 11m implementing a ball SAW sensor at a flow channel v of aerosols-under-test 33b. A susceptor 101 is provided at a bottom of the detector-substrate 11m, mounting the detector-substrate 11m. Since the detector-substrate 11m is spherical, the susceptor 101 has a concave portion for mounting the detector-substrate 11m, prohibiting the rolling and the movement out of the detector-substrate 11m. Into a through-hole cut at a central position of a bottom of the detection vessel 10, a bottom portion of the projecting cylinder is inserted downward. The projecting cylinder is provided at a lower portion of the electrode-holder base 102 to protrude downward. That is, the electrode-holder base 102 is provided above the detector-substrate 11m, and a bottom portion of the electrode-holder base 102 is inserted into an inner wall of the through-hole, which cuts vertically the bottom of the detection vessel 10. Here, the bottom of the detection vessel 10 reside at a location which serves as a top-sheathing wall for the detector-substrate 11m. And the electrode-holder base 102 is fixed at the upper portion of the detection vessel 10. The through-hole is provided at a top of a flow channel v. Although the through-hole cuts the bottom portion of the electrode-holder base 102 along the vertical direction, the location of the through-hole covers partially the above portion of the detector-substrate 11m. Furthermore, an upper portion of the electrode-holder base 102 is closed by a sensor-cell cap 103, stacked on the electrode-holder base 102.

A cylindrical electrode-feedthrough 104 is provided at the upper side of the detector-substrate 11m along the vertical direction to a main surface of the susceptor 101, or the vertical direction to the direction of the flow channel v. A rod-like external-electrode 105 is inserted in a space of the hollow structure of the electrode-feedthrough 104, so that a bottom of the external-electrode 105 can penetrates the sensor-cell cap 103. A north-pole electrode of the detector-substrate 11m is contacted to a lower end of the external-electrode 105 through a contact pin 105a, which stands along a vertical direction to the direction of the flow channel v, in the bottom portion of the electrode-holder base 102. The aerosols-under-test 33b are introduced with the gas flow velocity v through a tube 106 arranged horizontally, so as to eject the aerosols-under-test 33b to a pseudo-receptor film provided on a surface of the detector-substrate 11m. Although the tube 106 is illustrated as a pipe having a uniform thickness in FIG. 23, the tube 106 at the left side of the detector-substrate 11m has a topology of tapered nozzle, implementing the common concentrating-mechanism which concentrates the concentration, or the number density per unit volume of the target viruses 60.

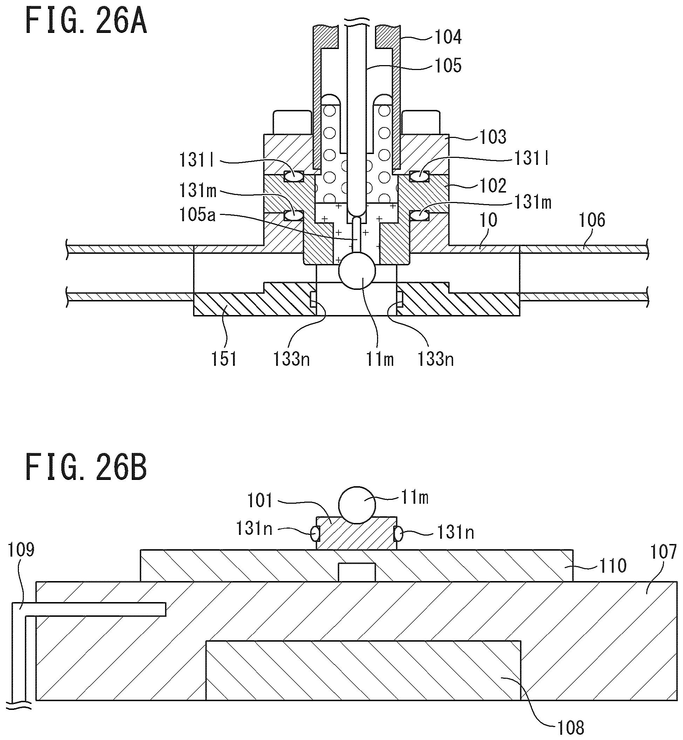

As illustrated in FIGs. 26A and 26B, at a lower side at a center of the detection vessel 10 and at a location being opposite to the susceptor 101 represented in FIG. 23, a piping member 151 for inserting in the susceptor 101 from the downward, and for detaching out the susceptor 101 toward the downward, is provided as a plate-like structure, having a thickness larger than a thickness of the tube 106. As illustrated in FIG. 26A, an O-ring groove 133n for inserting an O-ring 131n as a seal member is engraved at an inner wall of the vertical through-hole cut in the center at a bottom of the piping member 151. Corresponding to the above O-ring groove 133n, as illustrated in FIG. 26B, the O-ring 131n, which is fitted to an O-ring groove, is attached at a vertical side-wall of the cylindrical susceptor 101. By inserting the cylindrical susceptor 101 illustrated in FIG. 26B into the through-hole cut in the center at the bottom of the piping member 151 illustrated in FIG. 26A, a hermetical performance can be achieved, such that the O-ring 131n between the side-wall of the cylindrical susceptor 101 and the inner wall of the through-hole of the piping member 151 can prevent the leakage of gas.

Since the virus test device pertaining to the sixth derived-embodiment has a decomposable framing of the piping member 151 and the susceptor 101, as illustrated in FIGs. 26A and 26B, the hermetic structure by the O-ring 131n, such as the susceptor is removable through the piping member 151, whereby the gas does not leak from the space between the susceptor 101 and the piping member 151, when the detector-substrate 11m is replaced, is achieved. As understanding from FIG. 23 and FIG. 26A, the O-ring 131m is inserted between the upper surface of the detection vessel 10 and the electrode-holder base 102, and furthermore, the O-ring 131l is inserted between the electrode-holder base 102 and the sensor-cell cap 103, so that a decomposable feature such that the structure can be breakdown into the detection vessel 10, the electrode-holder base 102 and the sensor-cell cap 103, etc., can be achieved, while preventing the leakage of gas. A Peltier element 108 is accommodated in the lower portion of the holder 107 which is located at the direct under of the detector-substrate 11m. A thermistor 109 is inserted at a sideward location of the holder 107. The Peltier element 108 is used for heating and cooling the detector-substrate 11a through an adaptor 110. The thermistor 109 can be exchanged to other temperature sensor, such as thermocouple.

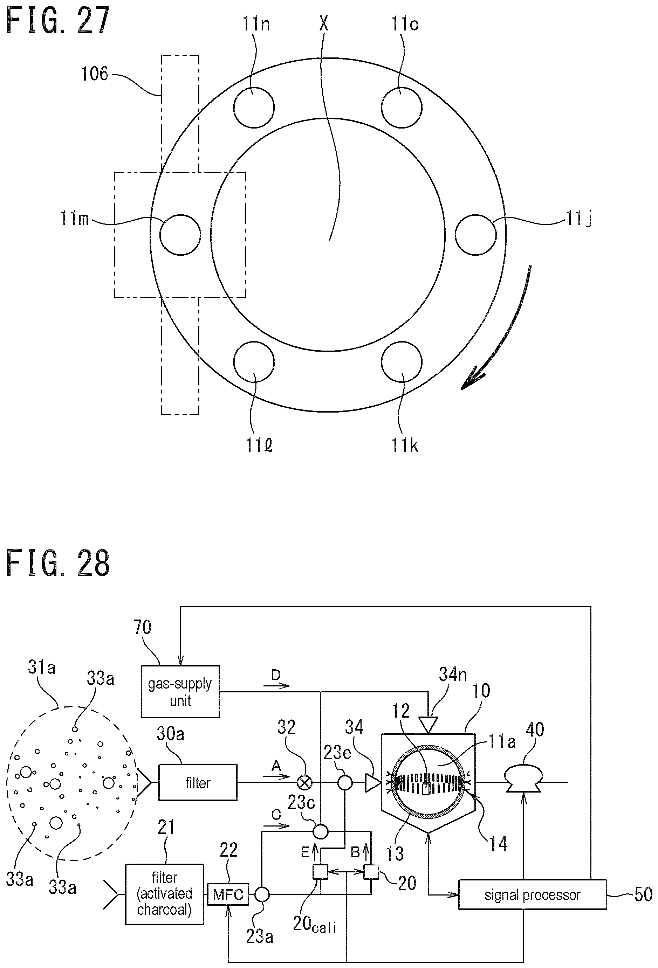

The susceptor 101 illustrated in FIG. 26B can be modified to a mechanism using a sextuple-spheres rotational-motion scheme, on which sextuple detector-substrates including a first detector-substrate 11j, a second detector-substrate 11k, a third detector-substrate 111, a fourth detector-substrate 11m, a fifth detector-substrate 11n, and a sixth detector-substrate 11o are mounted respectively, as illustrated in FIG. 27. If the first detector-substrate 11j illustrated in FIG. 27 is defined as a "subject ball SAW sensor", the remaining quintuple detector-substrates including the second detector-substrate 11k, the third detector-substrate 111, the fourth detector-substrate 11m, the fifth detector-substrate 11n, and the sixth detector-substrate 11o are "replacing ball SAW sensors". Since the virus test device pertaining to the sixth derived-embodiment illustrated in FIG. 23 has the structure which can be disassembled as illustrated in FIGs. 26A and 26B, the subject ball SAW sensor can be replaced to one of the replacing ball SAW sensors sequentially, by rotating the sextuple detector-substrates 11j, ..., 11o as to the center of the rotation axis X. The used detector-substrates 11j, ..., 11o are, for example, rotated, and moved to a sphere-exchange location, and replaced to a new sphere by exhausting one of the used detector-substrate, respectively, after disinfecting the one of the used detector-substrate by the disinfecting gas. The sextuple-spheres rotational-motion scheme may have an up-down mechanism in addition to the mechanism of the rotational-motion scheme. When adding the up-down mechanism to the rotational-motion scheme, the positions of the tube 106 and the detection vessel 10 disposed in the flow channel v shall be fixed, while the sextuple-spheres rotational-motion scheme illustrated in FIG. 27 shall execute the up-down operation and the rotary operation. As the result, a selective replacement of the spheres become more easily. In addition, although a case that the disk of the rotational-motion scheme can mount sextuple spheres has been explained in FIG. 27, the number of mountable spheres is not limited to six, and the number may be lower than six, or higher than six. Furthermore, as described below, if mounting a detector-substrate addressing to a virus test and another detector-substrate addressing to a moisture detection, it is preferable that the number of detector-substrates being mountable on the disk of the rotational-motion scheme shall be even numbers.

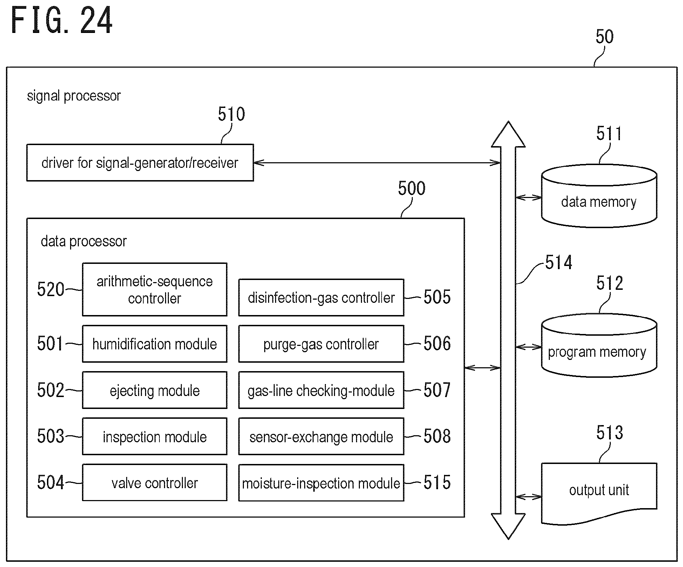

The signal processor 50 in the virus test system pertaining to the sixth derived-embodiment illustrated in FIG. 24 encompasses almost same logical circuits as the signal processor 50 in the virus test system pertaining to the fundamental embodiment recited in FIG. 1. However, a feature that the data processor 500 has a moisture-inspection module 515 is distinguishable from the signal processor 50 recited in FIG. 1. The moisture-inspection module 515 is scheduled to detect the moisture amount in the air, and is not scheduled to detect the target viruses 60 in the air. Furthermore, the moisture amount in the air can be estimated by preparing a specific material for adsorbing water molecules in the air on the moisture-detection sphere, and by difference-integral detection of the differences of areal densities by weight due to the adsorption of the water molecules as the delay times of the SAW, using the waveform data of the feedback burst-signals from the ball SAW sensor. Regarding the other technical features, since the same is true for the virus test system pertaining to the fundamental embodiment, duplicated description is omitted.

### --TEST METHOD BY SIXTH DERIVED-EMBODIMENT--

Next, a virus test method pertaining to a sixth derived-embodiment will be described with reference to FIG. 25 and FIG. 27. In the virus test method pertaining to the sixth derived-embodiment, triple detector-substrates including the first detector-substrate 11j, the third detector-substrate 111, and the fifth detector-substrate 11n, serving as moisture-detection spheres, and other triple detector-substrates including the second detector-substrate 11k, the fourth detector-substrate 11m, and the sixth detector-substrate 11o, serving as the virus-testing sphere, are prepared. And, each of the triple moisture-detection spheres and each of the triple virus-testing sphere are arranged to be inserted alternately. Then, after starting the humidifier 20, and before starting the virus test, humidity, or moisture amount inside the pipe is controlled to be kept at the optimum humidity, thereby executing the virus test.

### (0) (HUMIDITY CHECK PROCESS)

At first, in a step S20 illustrated in FIG. 25, the disk-based upon the rotational-motion scheme (hereafter, referred to as "the revolver") illustrated in FIG. 27 is driven by up-down and rotation motions, and then, values of humidity in the piping system and the detection vessel 10 are checked, after allocating the first detector-substrate 11j serving as the moisture-detection sphere at a predetermined checking site. Concretely, at first, the revolver is driven in up-down directions, and a limited part of the susceptor implementing the revolver illustrated in FIG. 26B is detached from the body of the detection vessel illustrated in FIG. 26A. And the triple detector-substrates including the first detector-substrate 11j, the third detector-substrate 111, and the fifth detector-substrate 11n, as the moisture-detection spheres, and the triple detector-substrates including the second detector-substrate 11k, the fourth detector-substrate 11m, and the sixth detector-substrate 11o, as the virus-testing spheres, are mounted on the revolver alternately, while the revolver is rotationally driven. After that, the revolver is driven by up-down and rotation motions, and the first detector-substrate 11j as the moisture-detection sphere is allocated at a predetermined checking site. Continuously, the on-off valve 32 is set to an open state, and the humidification-input valve 23a and the humidification-output valve 23b, which are three-way valves, are set to closed states respectively. The purge gas is introduced via the gas-supply unit 70, and a purging of undesired gas which remains inside the piping system and the detection vessel 10, etc., of the virus test device, is executed by the suction pump.

Next, the on-off valve 32 is set to the closed state, and the moist-air introducing-tube- B is selected by the flow-paths in humidification-input valve 23a and the humidification-output valve 23b. The cleaned dry-air is produced by cleaning up the impurities and the moisture using the filter 21 filled with the activated charcoal, etc., after sucking down the environmental air, by starting the operation of the suction pump. The cleaned dry-air becomes the cleaned moist-air by the humidification using the humidifier 20 after the flow rate is set to about 1 L/min by the MFC 22. The cleaned moist-air is supplied to the pseudo-receptor film which covers at least a partial area of the first detector-substrate 11j through the humidification-output valve 23b and the first concentrating-mechanism 34a, and then, the detection of the humidity inside the piping system and the detection vessel 10 is executed by the moisture-inspection module 515 in the data processor 500 installed in the virus test system illustrated in FIG. 24. And, if the humidity inside the piping system and the detection vessel 10 of the virus test device is judged to be the predetermined humidity, the sequence of process-flow is advanced to the following step.

### (1) (HUMIDIFICATION PROCESS)

At first, in a step S21 illustrated in FIG. 25, the second detector-substrate 11k as the virus-testing sphere is allocated at a predetermined checking site, instead of the moisture-detection sphere, by the up-down and rotation motions being driven with the revolver illustrated in FIG. 27. Then, in the step S21, a liquid film 61 is coated on the pseudo-receptor film which covers at least a partial area of the second detector-substrate 11k. The liquid film 61 is coated with an amount which is enough to maintain the activated state of the pseudo-receptors 14 on the pseudo-receptor film. The liquid film 61 is, for example, a buffer solution which mimics a water or a biological environment. Regarding the detailed concrete operations, since the same is true for the virus test method pertaining to the fundamental embodiment described in the flow chart illustrated in FIG. 12, duplicated description is omitted.

### (2) (EJECTION PROCESS)

Next, in a step S22 illustrated in FIG. 25, the aerosols-under-test 33b are ejected to the pseudo-receptor film, after sucking down the coarse aerosols 33a, including the coarse aerosols 33a the liquid, and manufacturing the fluid flow of the aerosols-under-test 33b made of the hydrous aerosols. Regarding the detailed concrete operations, since they are already described in the section of the virus test method pertaining to the fundamental embodiment with reference to the flow chart illustrated in FIG. 12, duplicated description is omitted.

### (3) (ASSAY PROCESS)

Next, in a step S23 illustrated in FIG. 25, difference-integral detections for inspecting whether the target viruses 60 exist or not in the aerosols-under-test 33b, are executed, which are ascribable to the amount of the target viruses 60 bound to the pseudo-receptors 14 on the pseudo-receptor film. Regarding the detailed concrete operations, since they are already described in the virus test method pertaining to the fundamental embodiment described with reference to the flow chart illustrated in FIG. 12, duplicated description is omitted.

### (4) (DISINFECTION PROCESS)

Next, in a step S24 illustrated in FIG. 25, a disinfection in the insides of the tubes and the equipment which are contaminated by the assay process in the step S23 is executed. Regarding the detailed concrete operations, since they are already described in the virus test method pertaining to the fundamental embodiment described with reference to the flow chart illustrated in FIG. 12, duplicated description is omitted.

As described above, according to the sixth derived-embodiment, since the optimum moisture amount is checked in advance, the specific-binding reactions, which will be executed successively, can be accelerated more and more. Moreover, a moisture-detection sphere for inspecting the moisture amount in the air, and a virus-testing sphere for inspecting the target viruses 60 in the air can be exchanged automatically, by using the sextuple-spheres rotational-motion scheme. Therefore, the in-situ measurement of the target viruses 60 can be executed more simply and more quickly.

### (SEVENTH DERIVED-EMBODIMENT)

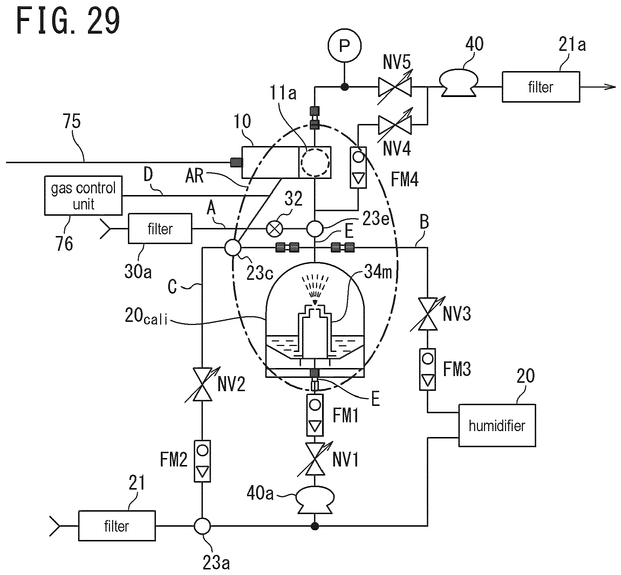

As illustrated in FIG. 28, a detection vessel 10 of a virus test device in a virus test system pertaining to a seventh derived-embodiment of the present invention encompasses an inlet-canal (first inlet-canal) at an outer wall of the left side of the detection vessel 10. And, the detection vessel 10 further encompasses a second inlet-canal at a ceiling side of the detection vessel 10, as with the structure of the virus test device pertaining to the fundamental embodiment recited in FIG. 1. From the left side in FIG. 28, a virus concentrating-mechanism 34, which is located at the eject-side edge of a virus introducing-tube-A, is connected to the inlet-canal of the detection vessel 10 through a testing-input valve 23e, which is a three-way valve (first three-way valve). The virus introducing-tube-A is a gas piping system which ejects the AUT 31a to the surface of the pseudo-receptor film 13. The virus test device pertaining to the seventh derived-embodiment further encompasses a calibration tube- E for ejecting calibration-purpose aerosols, which includes viruses with a prescribed concentration, to a pseudo-receptor film 13 laminated on a detector-substrate 11a. As illustrated in FG. 28 and FIG. 29, in piping-path extending to the rightward to connect the humidifier 20, from the MFC 22 via a humidification-input valve 23a, which is a third three-way valve toward the humidifier 20, a bifurcation node is allocated at a location between the humidification-input valve 23a and the humidifier 20. And, a T-branched tube is provided at the bifurcation node so that a calibration-purpose aerosol-generator 20_{cali} is connected to one of the tubes extending upward in the T-branched tube.

In the description of the virus test system pertaining to the fundamental embodiment, which is already described by using FIG. 1, it is described that the humidifier 20 encompasses the moisture-generating nebulizer --a liquid atomization apparatus-- and the infiltration tube. However, the nebulizer --liquid atomization apparatus-- is generally used also for an intended use which generates the aerosols including the water, the air, and the fine particles, by atomizing a suspension of fine particles including water and viruses, etc., other than the use application for generating the aerosols of the water. As illustrated in FIG. 28 and FIG. 29, the virus test system and the virus test device pertaining to the seventh derived-embodiment of the present invention, which is derived from the fundamental embodiment is, encompasses the humidifier 20 connected the bifurcation node defined in the piping system. Here, the piping system include a tube reaching the humidification-input valve 23a, as the third three-way valve, through the MFC 22 from the filter 21, and further, the piping system extends to the rightward through the humidification-input valve 23a to a humidification-output valve 23c. The bifurcation node is provided in the path between the humidification-input valve 23a and the humidification-output valve 23c.

As illustrated in FIG. 28 and FIG. 29, the testing-input valve 23e of the virus test device pertaining to the seventh derived-embodiment is implemented by a first three-way valve, and the humidification-output valve 23c is implemented by a second three-way valve. A set of triple connection ports of the first to third valve-ports corresponds to a topology of triple tubes, which are connected to the first and second three-way valves respectively. Here, "the first to third valve-ports" are mere terms, which are used to specify locations in a circuit implemented by the piping system, for convenience. Therefore, the terms of "the first to third valve-ports" do not always presuppose a situation that individual physical members are independently existing.

As illustrated in FIG. 28, the detection vessel 10 of the virus test device according to the virus test system pertaining to the seventh derived-embodiment encompasses, an inlet-canal --a first inlet-canal-- at an outer wall of the left side of the detection vessel 10, like the structure of the virus test device pertaining to the fundamental embodiment recited in FIG. 1. However, the detection vessel 10 encompasses further a second inlet-canal at a ceiling side of the detection vessel 10. A virus concentrating-mechanism 34 which is located at the eject-side edge of the virus introducing-tube-A is connected to the inlet-canal of the detection vessel 10 through the third valve-port of the testing-input valve 23e, as the first three-way valve, from the left side in FIG. 28. The virus introducing-tube-A is a gas piping system which ejects the AUT 31a to the surface of the pseudo-receptor film 13. The virus test device pertaining to the seventh derived-embodiment further encompasses the calibration tube- E for ejecting the calibration-purpose aerosols containing the viruses of the prescribed concentration on the pseudo-receptor film 13 laminated on the detector-substrate 11a. As illustrated in FIG. 28 and FIG. 29, a piping system extends from the MFC 22 to the humidification-input valve 23a, as the third three-way valve, and the piping system extends further to the rightward, so that the connection with the humidifier 20 can be facilitated. The T-branched tube is provided at a intermediate position of the piping system extending to the rightward to connect the humidifier 20, and the calibration-purpose aerosol-generator 20_{cali} is connected to one of the ports of the T-branched tube.

As illustrated at the center in FIG. 29, the aerosol generator 20_{cali} has, a structure of a liquid tank which can accumulate a virus suspension defined by a prescribed concentration, and a compressor-based nebulizer --liquid atomization apparatus-- having a splay chamber covering an upper portion of the liquid tank. The aerosol generator 20_{cali} further encompasses a suspension nozzle 34m which ejects the aerosols containing the viruses with prescribed concentration, by sucking up the virus suspension from the liquid tank to the inside of the splay chamber. The suspension nozzle 34m can have a concentric dual pipe nozzle, for example, as illustrated in FIG. 29. The virus test device pertaining to the seventh derived-embodiment differs from the virus test device pertaining to the fundamental embodiment in a technical feature such that the virus test device encompasses the calibration-purpose aerosol-generator 20_{cali}, which can generate the calibration-purpose aerosols in addition to the humidifier 20, as illustrated in FIG. 28 and FIG. 29. The aerosol generator 20_{cali} may include further a function of an ultrasonic capability based on the structure of the compressor-based nebulizer --liquid atomization apparatus--. According to the virus test device pertaining to the seventh derived-embodiment, as the virus test device encompasses the calibration-purpose aerosol-generator 20_{cali}, the calibration of the detector-substrate 11a can be executed, by ejecting the calibration-purpose aerosols containing viruses with the prescribed concentration to the detector-substrate 11a accommodated in the detection vessel 10, and by measuring responses at multiple times after the instant of ejection. As described in the virus test device pertaining to the fundamental embodiment, a geometry of the detector-substrate 11a is a spherical, and the pseudo-receptor film 13 is arranged at least a partial area of the detector-substrate 11a.

If the testing-input valve 23e allows the flow of the calibration-purpose aerosols from the calibration tube- E, by controlling the testing-input valve 23e implemented by the first three-way valve, the calibration-purpose aerosols are ejected to the detector-substrate 11a accommodated in the detection vessel 10, as a result of concentration by the common concentrating-mechanism--a first common concentrating-mechanism-- 34, and the calibration-purpose aerosols are used for the calibration of the detector-substrate 11a. If the testing-input valve 23e allows the flow of the AUT from the virus introducing-tube-A, by controlling the testing-input valve 23e, the concentration of the target viruses is measured by using the calibrated detector-substrate 11a. The virus test system pertaining to the seventh derived-embodiment further encompasses a gas-supply unit70, and a purge-gas introducing-tube-D which is connected to the second inlet-canal of the ceiling side of the detection vessel 10. As illustrated in FIG. 28, a humidification-output valve 23c made of the second three-way valve has a third valve-port connected to the purge-gas introducing-tube-D. In addition, a humidification-output valve 23c has a first valve-port connected to the moist-air introducing-tube- B and a second valve-port connected to the dry-air bypass-tube-C. The gas-supply unit 70 supplies the disinfecting gas or the purge gas into the detection vessel 10 alternatively, through the purge-gas introducing-tube-D. A second common concentrating-mechanism 34n is provided at the exit end of the purge-gas introducing-tube-D. The second common concentrating-mechanism 34n is connected to the second inlet-canal of the ceiling side of the detection vessel 10.

As illustrated in FIG. 28, the purge-gas introducing-tube-D is T-branched, and one of the branches is connected to the third valve-port of the humidification-output valve 23c, and at the edge of another branch of the purge-gas introducing-tube-D is provided with the second common concentrating-mechanism 34n, which is connected to the ceiling side of the detection vessel 10. Namely, the purge-gas introducing-tube-D is connected to the ceiling side of the detection vessel 10, and a purge-gas flow is introduced from the upper portion of the detection vessel 10 toward the lower portion of the detection vessel 10. Since the suction pump 40 is connected to the detection vessel 10, the disinfecting gas or the purge gas 10 from the purge-gas introducing-tube-D is fed to the suction pump 40 through the detection vessel 10, by sucking action ascribable to the operation of the suction pump 40. Via the humidification-output valve 23c, the moist-air introducing-tube- B and the dry-air bypass-tube-C are connected to one of the ports of the T-branched tube provided in the purge-gas introducing-tube-D, which is provided between the gas-supply unit 70 and the second common concentrating-mechanism 34n. Therefore, either of the moist-air introducing-tube- B or the dry-air bypass-tube-C can be elected by switching the humidification-output valve 23c. Then, it is possible to introduce one of the cleaned moist-air and the cleaned dry-air into the purge-gas introducing-tube-D alternatively, by valve operations of the humidification-output valve 23c. The cleaned moist-air, the cleaned dry-air and the purge gas are sequentially ejected from the second common concentrating-mechanism 34n to the surface of the detector-substrate 11a through the second inlet-canal of the detection vessel 10, by switching the flow paths.

In addition, since the calibration tube- E is connected at an intermediate position of the virus introducing-tube-A through the first valve-port of the testing-input valve 23e implemented by the first three-way valve, it is possible to introduce one of the AUT 31a or the calibration-purpose aerosols to the virus introducing-tube-A alternatively, by controlling the testing-input valve 23e. Therefore, the calibration of the detector-substrate 11a can be executed, by ejecting the calibration-purpose aerosols containing the calibration-purpose virus, in which the virus concentration is set to a plurality of values, through the first common concentrating-mechanism 34 to the detector-substrate 11a, by controlling the testing-input valve 23e implemented by the first three-way valve. According to the virus test system pertaining to the seventh derived-embodiment, the difference-integral detection, which has a higher reliability compared with the virus test device pertaining to the fundamental embodiment, can be executed by calibrating the sensitivity of the detector-substrate 11a, using the calibration-purpose aerosols.

As illustrated in FIG. 28, the virus test system pertaining to the seventh derived-embodiment encompasses a signal conditioner 12 which converts physical signals representing variations of the physical states of the pseudo-receptor film 13 due to the specific bindings of the target viruses to the pseudo-receptors 14 provided on the pseudo-receptor film 13, to corresponding electric signals, and a signal processor 50 which executes the difference-integral detection utilizing the electric signals transmitted from the signal conditioner 12. Here, the signal processor 50 further has a functional capability for driving and controlling the gas-supply unit 70. The existence of the viruses, which are bound to the surface of the pseudo-receptor film 13 by the spike-protein receptor-bindings, can be detected by executing the arithmetic process with the difference-integral detection, using the signal processor 50. Simultaneously, as the signal processor 50 executes the difference-integral detection utilizing the electric signals transmitted from the signal conditioner 12, the sensitivity in the virus test system can be calibrated, by detecting the amount of bindings of the calibration-purpose viruses, which are contained in the calibration-purpose aerosols, to the surface of the pseudo-receptor film 13.

As described above, the virus introducing-tube-A, the moist-air introducing-tube- B, the dry-air bypass-tube-C, the purge-gas introducing-tube-D, and the calibration tube- E can be selected as one of the gas introducing paths, by controlling the testing-input valve 23e implemented by the first three-way valve and the humidification-output valve 23c made of the second three-way valve. In addition, as illustrated in FIG. 29, the calibration tube-

E may be implemented by a series connection of a line pump - compressor-- 40a into an inlet side of the calibration-purpose aerosol-generator 20_{cali}. Furthermore, as to an example of concrete piping system for the virus test device pertaining to the seventh derived-embodiment illustrated in FIG. 29, a part of a piping-path for the calibration tube- E is implemented by a series connection of the line pump 40a, the first variable leakage valve NV1, the first flow-meter FM1, and the calibration-purpose aerosol-generator 20_{cali}. In the example illustrated in FIG. 29, the calibration tube- E can be considered as being divided into an upper side tube of the calibration-purpose aerosol-generator 20_{cali} and a lower side tube of the calibration-purpose aerosol-generator 20_{cali}. Namely, the upper side tube of the calibration tube- E and the lower side tube of the calibration tube- E are assigned to both sides of the calibration-purpose aerosol-generator 20_{cali}, so as to sandwich the calibration-purpose aerosol-generator 20_{cali}. And a tip of the splay chamber of the aerosol generator 20_{cali} is connected to the first valve-port of the testing-input valve 23e through the upper side tube of the calibration tube- E. The cleaned dry-air pressurized by the line pump 40a through the filter 21, is ejected from the tube located at the center of the suspension nozzle 34m having the dual tube structure.

And an opening provided at bottom of a sheath between an outer wall defined by an outside tube and an inner wall defined by a center tube of the dual tube structure sucks up the virus suspension having the prescribed concentration from a liquid tank of the aerosol generator 20_{cali}, when a cleaned dry-air is ejected upward from the center tube of the suspension nozzle 34m. In addition, the virus suspension having the prescribed concentration, which is introduced in a gap of the sheath from the bottom of the sheath, is evacuated up to the top of the sheath, and the virus suspension is splayed from the top of the sheath in the splay chamber to establish an atomized state. As illustrated in FIG. 29, after the virus suspension is splayed as the mist in the splay chamber, the splayed virus suspension is introduced in a tube extending to the first valve-port of the testing-input valve 23e implementing the calibration tube- E, by splaying the calibration-purpose aerosols containing viruses with the prescribed concentration from the tip of the splay chamber.

The calibration-purpose aerosol-generator 20_{cali} amasses the virus suspension --antigen solution-- in which the concentration is controlled, and the aerosols containing the viruses in which the concentration is controlled is generated as the calibration-purpose aerosols. Here, FIG. 29 is a schematic piping diagram for convenience. Namely, the illustrated piping diagram is different from the actual piping configuration. In FIG. 29, a portion of the piping system residing above the testing-input valve 23e is a virtual state, which is rotated counter-clockwise by 90 degrees from the orientation of real state. Furthermore, it is necessary to note that FIG. 29 is schematically representing a three-dimensional structure. Namely, in the near side of the paper face of FIG. 29, the moist-air introducing-tube- B is connected to the first valve-port of the humidification-output valve 23c, and the dry-air bypass-tube-C is connected to the second valve-port of the humidification-output valve 23c, thereby implementing the three-dimensional structure. In addition, the upper side tube of the calibration tube-E extending from the splay chamber of the calibration-purpose aerosol-generator 20_{cali} is connected to the first valve-port of the testing-input valve 23e, without meeting with the moist-air introducing-tube- B and the dry-air bypass-tube-C in the three-dimensional space.

In addition, the moist-air introducing-tube- B implements a piping-path embracing the third variable leakage valve NV3, which is connected between the humidifier 20 and the first valve-port of the humidification-output valve 23c in series, and the third flow-meter FM3. The dry-air bypass-tube-C implements a bypass piping-path embracing the second variable leakage valve NV2, which is connected between the humidification-input valve 23a made of the third three-way valve and the second valve-port of the humidification-output valve 23c made of the second three-way valve in series, and the second flow-meter FM2. Furthermore, as illustrated in FIG. 29, a bypass path in which the fourth variable leakage valve NV4 and the fourth flow-meter FM4 are connected in series is branched at a node between the third valve-port of the testing-input valve 23e and the detection vessel 10. As illustrated at the upward of the paper of FIG. 29, in a piping-path of the outlet side --exhaust side-- passing through the detector-substrate 11a, a pressure gauge P is attached at an L-shaped tube. The L-shaped tube extends toward the upward from the detection vessel 10 and further bends toward the right side. And a fifth variable leakage valve NV5 is connected to the L-shaped tube, to which the pressure gauge P is attached. As already explained, in the representation of FIG. 29, the portion of the piping system residing above the testing-input valve 23e is illustrated schematically as a virtual state, which is counter-clockwise rotated by 90 degrees from the real orientation. Therefore, the pressure gauge P shall be located at the rightward of the detection vessel 10, in the real orientation. In addition, since the L-shaped tube virtually illustrated in FIG. 29 which extends toward the upward from the detection vessel 10 and further bends in the right side shall be returned by 90-degrees clockwise rotation, in the real orientation. Therefore, the L-shaped tube is the pipe which extends toward the rightward from the detection vessel 10 and further curves in the downward in the real orientation. In addition, the L-shaped tube is further connected to the suction pump 40, after combining with the branching tube of the bypass path extending from the outlet side of the fifth variable leakage valve NV5.
A filter 21a, which is connected to the output side of the suction pump 40, is a piping element provided for preventing the leakage of the viruses to the external environment.

Furthermore, in the virus test device according to the virus test system pertaining to the seventh derived-embodiment, the moist-air introducing-tube- B, the dry-air bypass-tube-C, and the purge-gas introducing-tube-D are provided independently to the virus introducing-tube-A and the calibration tube- E respectively. In FIG. 29, although the virus introducing-tube-A and the calibration tube- E are expressed in the level of the paper face. However the moist-air introducing-tube- B, the dry-air bypass-tube-C, and the purge-gas introducing-tube-D shall have three-dimensional configurations, which are locating at the near side of the paper face respectively. As illustrated in FIG. 29, in the middle of a pipe between the third valve-port of the humidification-output valve 23c and the first inlet-canal of the detection vessel 10, the purge-gas introducing-tube-D extending from a gas control unit 76 is connected via a T-branched tube to the pipe between the humidification-output valve 23c and the detection vessel 10. The gas control unit 76 has a functional capability for supplying selectively the disinfecting gas or the purge gas to the piping system extending from the third valve-port of the humidification-output valve 23c, through the gas transfer pipe D, like the gas-supply unit 70 which has been illustrated in FIG. 28. Because FIG. 29 represents the three-dimensional piping diagram, a location, at which the purge-gas introducing-tube-D is connected to the middle of the tube between the third valve-port of the humidification-output valve 23c and the first inlet-canal of the detection vessel 10, shall be the near side of the paper face.

The contamination possibility with viruses in the cleaned moist-air or the cleaned dry-air can be eliminated, by providing the moist-air introducing-tube- B, the dry-air bypass-tube-C, and the purge-gas introducing-tube-D, which are independent from the piping system of the virus introducing-tube-A and the calibration tube- E. In addition, the above architecture can achieve an effectiveness that dispersion of the virus concentration in the calibration-purpose aerosols can be suppressed. Furthermore, the pressure of the purge gas is monitored by the pressure gauge P illustrated at the upward of FIG. 29, because the NSA material is not removed from the detector-substrate 11a by blowing out, if the pressure of the purge gas is lower. Furthermore, under a condition that the purge-gas introducing-tube-D is provided independently from the piping system of the virus introducing-tube-A and the calibration tube- E, the direction of the purge gas is flowed from an upper portion to a lower portion of the detection vessel 10. By setting the pressure of the purge gas to higher, and by flowing the purge gas from the upper to the lower portion of the detection vessel 10, the NSA material can be removed efficiently from roundtrip path of SAW, by the purge gas. As recited in FIG. 3 and FIG. 13, the external-electrode 105 is provided at the north-pole side of the ball SAW sensor (11a, 12, 13). The high-frequency transmission-line 75 which is connected to the external-electrode 105 of the ball SAW sensor (11a, 12, 13) is illustrated horizontally at the left-upper portion in FIG. 29. However, since FIG. 29 is the schematic diagram, in which the piping system residing above the testing-input valve 23e is rotated counter-clockwise by 90-degrees from the real orientation, the high-frequency transmission-line 75 shall be an electrical line led from the upward to the north-pole side of the ball SAW sensor (11a, 12, 13) along the vertical direction. In addition, the extending portion of the purge-gas introducing-tube-D, which extends from the third valve-port of the humidification-output valve 23c to the second inlet-canal of the detection vessel 10, is drawn obliquely. The reason why the extending portion of the purge-gas introducing-tube-D is illustrated obliquely is to represent a configuration for flowing the purge gas from the north-west direction toward the south-east direction in the real orientation, by connecting the purge-gas introducing-tube to the second inlet-canal provided at the north-west location of the detection vessel 10, while avoiding the high-frequency transmission-line 75 will meet the north-pole side electrode of the ball SAW sensor.

According to the virus test device pertaining to the seventh derived-embodiment, a more compact structure can be obtained by integrally molding piping elements in the three-dimensional structure, which are surrounded by a dot-dashed line as an area AR at the center in FIG. 29, using an injection molding apparatus such as a 3D printer, etc. Concretely, as the piping elements included in the three-dimensional area AR illustrated in FIG. 29, the detection vessel 10 for accommodating the detector-substrate 11a, the calibration-purpose aerosol-generator 20_{cali}, and the humidification-output valve 23c inserted between the detection vessel 11 and the calibration-purpose aerosol-generator 20_{cali}, etc., can be assigned. The humidification-output valve 23c illustrated in FUG. 29 is located at the near side of the paper face. The humidification-output valve 23c made of the second three-way valve has the third valve-port connected to the purge-gas introducing-tube-D at the near side of the paper face. In addition, the humidification-output valve 23c has the first valve-port connected to the moist-air introducing-tube- B at the near side of the paper face and the second valve-port connected to the dry-air bypass-tube-C at the near side of the paper face. Although the illustration is omitted in FIG. 28, the bypass path, which encompasses the series-connection of the fourth variable leakage valve NV4 and the fourth flow-meter FM4, is branched to a middle of the tube between the detection vessel 10 and the third valve-port of the testing-input valve 23e.

Partial members of tubes or piping joints for the quintuple piping routes such as the moist-air introducing-tube- B, the dry-air bypass-tube-C, and the bypass path, etc., which are respectively connected to the humidification-output valve 23c located at the near side of the paper face, may be also included in the three-dimensional area AR, which is surrounded by the dot-dashed line illustrated in FIG. 29, of course. In a one-piece structure molded by the 3D printer, etc., the first inlet-canal at the left side of the detection vessel 10 and the second inlet-canal at the ceiling side are not required to be always existing as separated openings, which are physically existing independently. Namely, for example, the first and second inlet-canals may serve as nomenclatures for defining the names of specific site, just only illustrating the specific locations in which the first common concentrating-mechanism 34 and the second common concentrating-mechanism 34n are connected. On the other hand, even if the one-piece molded structure by the 3D printer, etc. is employed, a separatable structure, which facilitates a contact-sliding movement with the first and second inlet-canals relatively to the first common concentrating-mechanism 34 and the second common concentrating-mechanism 34n, respectively, can be prepared. Therefore, even if the single piece molded structure by the 3D printer, etc. is considered, the first and second inlet-canals become the physically existing openings, which are cut in the wall of the detection vessel 10 as the substantial physical structure, if the operations of contact-sliding movement with the first and second inlet-canals relatively to the first common concentrating-mechanism 34 and the second common concentrating-mechanism 34n, respectively, are scheduled to be used. According to the virus test system pertaining to the seventh derived-embodiment, a more and more downsized structure can be achieved, by the method of integral mold through the 3D printer, etc.

### --TEST METHOD BY SEVENTH DERIVED-EMBODIMENT--

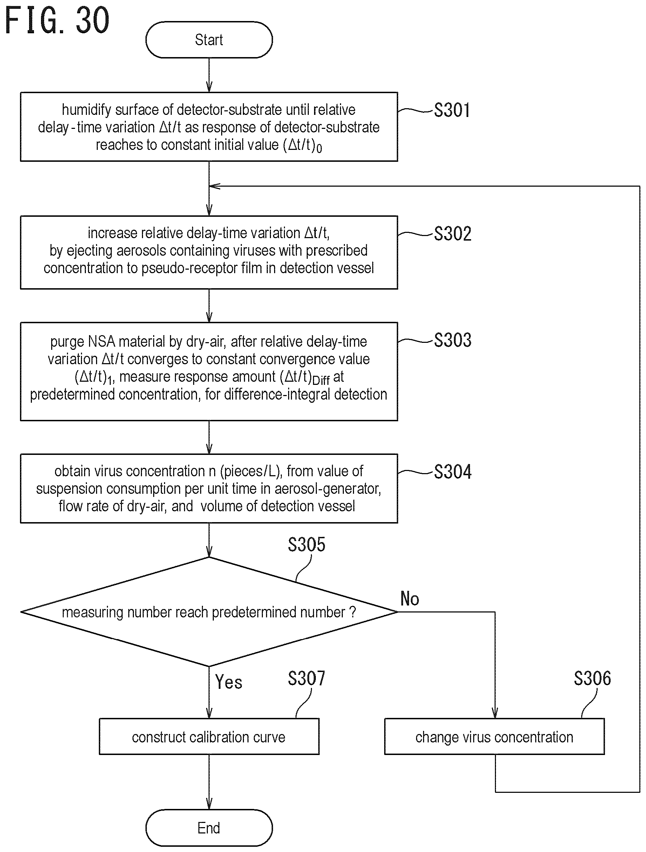

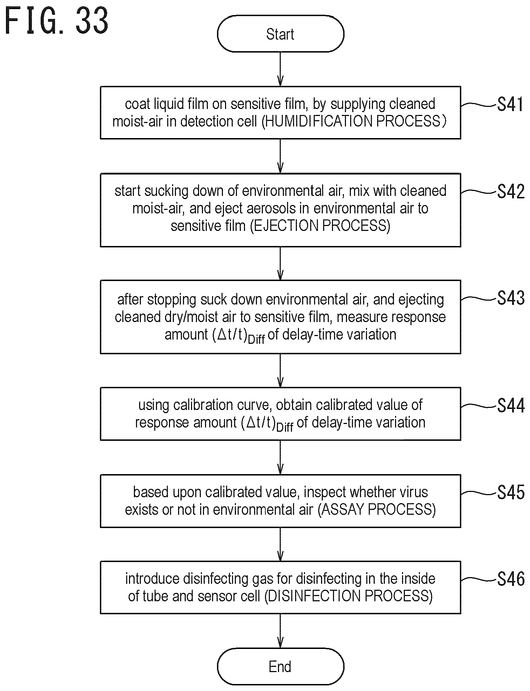

Next, a method of executing a sensitivity calibration of the detector-substrate 11a implementing the ball SAW sensor using the virus test system and the virus test device pertaining to the seventh derived-embodiment which has been illustrated in FIG. 28 and FIG. 29, will be described with references to the flow charts, etc., illustrated in FIG. 30. Furthermore, a method of executing a virus test with a higher sensitivity, by using the calibrated ball SAW sensor will be described with references to the flow charts, etc., illustrated in FIG. 33. As illustrated below, the virus test method pertaining to the seventh derived-embodiment is broadly separated into double-stages of a construction process of the calibration curve and a virus test process by the sensitivity calibration.

### (a) CONSTRUCTION OF CALIBRATION CURVE

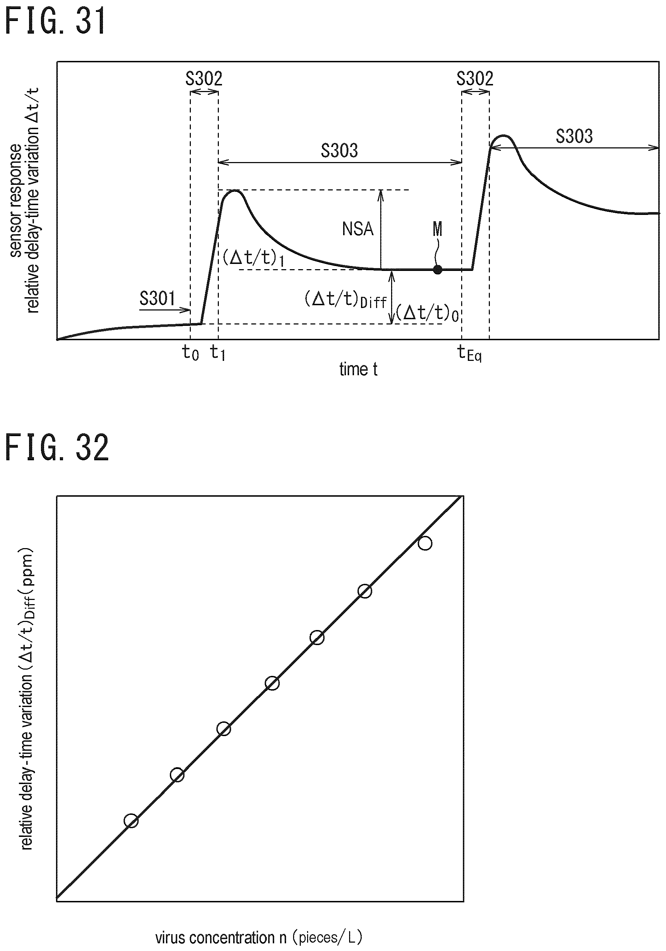

At first, as illustrated in a step S301 of the flow chart illustrated in FIG. 30, a humidification at a surface of the detector-substrate 11a by the moist air is executed, by supplying the moist air, the flow rate of which is controlled via the third variable leakage valve NV3 and the third flow-meter FM3 illustrated in FIG. 29, from the moist-air introducing-tube- B to the detection vessel 10. In the step S301 of FIG. 31, a relative delay-time variation Δt/t as the response of the detector-substrate 11a is set to a value which reaches to a constant initial value (Δt/t)₀. However, the step S301 may be omitted when the surface of the detector-substrate 11a has already been humidified. In FIG. 31, a timing that the relative delay-time variation Δt/t is reached to the constant initial value (Δt/t)₀ defines the starting point t=0 on the time axis.

Next, in a step S302, the cleaned air, the flow rate of which is controlled by the first variable leakage valve NV1 and the first flow-meter FM1 illustrated in FIG. 29, is supplied to the suspension nozzle 34m of the calibration-purpose aerosol-generator 20_{cali}, for a constant time interval t1. In the step S302, the suspension is evacuated up from the liquid tank which amasses the virus suspension having the prescribed concentration n₀, the value of which is set in advance, to the suspension nozzle 34m. After that, in the step S302, the aerosols containing viruses with the prescribed concentration n₀, the value of which is set in advance, are splayed from the suspension nozzle 34m of the calibration-purpose aerosol-generator 20_{cali}. The aerosols containing viruses with the prescribed concentration n₀ is introduced via the calibration-purpose aerosol-generator 20_{cali} to the tube which connects the detection vessel 10 to the third valve-port of the testing-input valve 23e through the first valve-port of the testing-input valve 23e. The calibration-purpose aerosols containing viruses with the prescribed concentration n₀ which are introduced to the tube connecting the detection vessel 10 and the third valve-port of the testing-input valve 23e, are ejected to the pseudo-receptor film 13 in the detection vessel 10 through the nozzle 34 provided at the detection vessel 10. After the calibration-purpose aerosols containing viruses with the prescribed concentration n₀ are ejected to the pseudo-receptor film 13 at the timing of the step S302, as represented by the response-curve illustrated in FIG. 31, the relative delay-time variation Δt/t increases with time.

Next, after elapsing a time interval t₁ illustrated in FIG. 31, in a step S303 of the flow chart illustrated in FIG. 30, the NSA material other than the viruses specifically bound to the pseudo-receptor film 13 in the detection vessel 10, is purged. The purge of the NSA material is executed by ejecting the cleaned dry-air, the flow rate of which is controlled by the second variable leakage valve NV2 and the second flow-meter FM2 illustrated in FIG. 29, from the dry-air bypass-tube-C to the pseudo-receptor film 13 in the detection vessel 10. In the process stage of purging of the NSA material in the step S303, illustrated in FIG. 31, after the increase of the relative delay-time variation Δt/t is reached to the saturation, the relative delay-time variation Δt/t changes to decreasing behavior, and then, the relative delay-time variation Δt/t converges to a constant convergence value (Δt/t)₁, before reaching the time t_{Eq}. After the decreasing of the relative delay-time variation Δt/t is converged to the convergence value (Δt/t)₁, for example, at the time of the point M illustrated in FIG. 31, a difference (Δt/t)_{Diff} between the initial value and the convergence value is measured. The difference (Δt/t)_{Diff} is the gap between the initial value (Δt/t)₀ and the convergence value (Δt/t)₁ illustrated in Eq. (8), and a response amount at the concentration n₀ of the difference-integral detection can be measured, as the response by the viruses on the detector-substrate 10a other than the nonspecific-adsorption. ${\left(Δt/t\right)}_{Diff} = {\left(Δt/t\right)}_{1} - {\left(Δt/t\right)}_{0}$ (8).

Next, in a step S304, the virus concentration n (pieces/L) in the detection vessel 10 is obtained from the values of the suspension consumption per unit time in the calibration-purpose aerosol-generator 20_{cali}, the flow rate of the cleaned dry-air, and the volume of the detection vessel 10. After that, in a step S305 and a step S306, the step 302 to a step S304 of the flow chart illustrated in FIG. 30 are repeated by changing the virus concentration in the suspension to the virus concentration n₁, n₂, n₃, n₄, ......... sequentially, while the measuring number is reached the predetermined number. In addition, in a step S305 and a step S307, the calibration curve as illustrated in FIG. 32 is constructed as the relationships between the virus concentration n = n, n₁, n₂, n₃, n₄, ......... and the response amount (Δt/t)_{Diff} of the difference-integral detection, and the process procedure illustrated by the flow chart illustrated in FIG. 30 is finished, when the measuring number is reached the predetermined number.

### (b) ASSAY WITH CALIBRATED SENSITIVITY

Next, a virus test procedure with calibrated sensitivity will be described with reference to the flow chart illustrated in FIG. 33. Since the procedure of the flow chart illustrated in FIG. 33 is depending on the virus test method pertaining to the fundamental embodiment recited in FIG. 12, the overlapped detailed description is omitted as to the description of the virus test method pertaining to the fundamental embodiment.

At first, in a step S41 illustrated in FIG. 33, a moist air, the flow rate of which is controlled via the third variable leakage valve NV3 and the third flow-meter FM3 illustrated in FIG. 29 is supplied from the moist-air introducing-tube- B to the detection vessel 10, and a humidification of the surface of the detector-substrate 11a by the moist air is executed. As the result, the pseudo-receptors 14 is covered by the liquid film 61, like the state recited in FIG. 10B. The liquid film 61 is coated with the amount which is enough to maintain the activation of the pseudo-receptors 14. The liquid film 61 is, for example, a water, or a buffer solution which mimics the biological environment.

Next, in a step S42, a fluid flow of the aerosols-under-test 33b is produced, after sucking AUT 31a, and by removing dust particles and aerosols, which have sizes larger than the predetermined size respectively, from the coarse aerosols 33a contained in the AUT 31a, using the filter 30. And then, in the step S42, the fluid flow of the aerosols-under-test 33b is ejected to the pseudo-receptor film 13. The aerosols-under-test 33b becomes high-speed air-flow which is concentrated by the first common concentrating-mechanism 34, which has a topology of tapered nozzle, like the embodiments recited in FIG. 11A and FIG. 11B, and the aerosols-under-test 33b are supplied to the pseudo-receptor film 13 disposed on at least partial area in the detector-substrate 11a. As the result, if the target viruses 60 are existing in the aerosols-under-test 33b, the specific-binding reactions between the RBD of the spike-glycoprotein 17 in the target viruses 60 to the pseudo-receptors 14 occur on the pseudo-receptor film 13.

Next, in a step S43, the cleaned dry-air are ejected from the dry-air bypass-tube-C to the pseudo-receptor film 13 disposed on at least partial area in the detector-substrate 11a. Simultaneously, a cleaned moist-air may be supplied to the pseudo-receptor film 13 disposed on at least partial area in the detector-substrate 11a, by selecting the moist-air introducing-tube- B instead of the dry-air bypass-tube-C. Then, the NSA material as the impurities of the viruses and the dust particles, etc., other than the target viruses which is nonspecific-adsorbed to the pseudo-receptor film 13, can be removed by the process of the step S43. After that, for example, the response amount (Δ t /t)_{Diff} of the delay-time variation of the ball SAW sensor (11a, 12, 13, 14) is measured.

Next, in a step S44, by using the calibration curve prepared by the construction process of the calibration curve, in the flow chart illustrated in FIG. 30, a calibrated value of the response amount (Δ t /t)_{Diff} of the delay-time variation, the delay-time is measured in the step S43 illustrated in FIG. 33, is obtained. Concretely, the calibrated value can be obtained by converting the ordinate values of the calibration curve illustrated in FIG. 32 to the abscissa values of the calibration curve. In addition, in a step S45, the inspection whether the viruses exists or not in the environmental air and the measurement of the virus concentration when the viruses exist in the environmental air, are executed, based upon the calibrated value acquired by a step S44.

Finally, in a step S46, disinfection processes in the insides of the tubes and the equipment which are contaminated by the processes of the step S43 to the step S45, is executed. If executing the virus tests at other inspection sites continuously, employing the procedure of the virus test method illustrated by the flow chart of FIG. 33, the executions of the virus tests with the higher precision at the next virus test site, can be achieved under the same condition of the virus test system and the virus test device, which can be used safety and reassuringly.

### (EIGHTH DERIVED-EMBODIMENT)

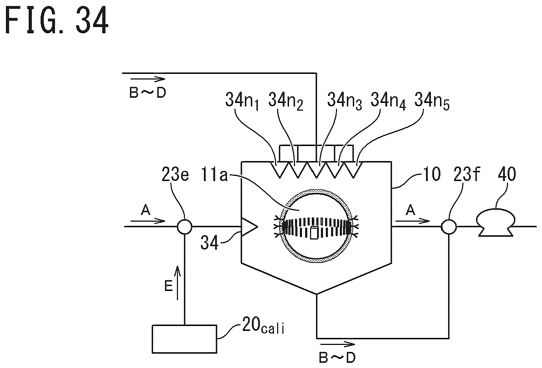

Like the virus test device pertaining to the seventh derived-embodiment, a detection vessel 10 of a virus test device pertaining to an eighth derived-embodiment encompasses an inlet-canal in an outer wall of a left side of a detection vessel 10, with the similar configuration illustrated in FIG. 28 basically, as illustrated in FIG. 34. However, the detection vessel 10 of the virus test device pertaining to the eighth derived-embodiment further encompasses a plurality of individual concentrating-mechanisms, or quintuple array of concentrating-mechanisms 34ₙ₁, 34ₙ₂, 34ₙ₃, 34ₙ₄, 34ₙ₅ as the purging nozzles, at the ceiling side of the detection vessel 10. The architecture of the detection vessel 10 according to the virus test system pertaining to the eighth derived-embodiment illustrated in FIG. 34 is addressing to improve the performance of the virus test method pertaining to the seventh derived-embodiment, by reducing the measurement errors due to the nonspecific-adsorptions. Namely, the divided concentrating-mechanisms 34ₙ₁, 34ₙ₂, 34ₙ₃, 34ₙ₄, 34ₙ₅ as the purging nozzles, are provided at a top of the detection vessel 10, and then, the virus test system pertaining to the eighth derived-embodiment has a feature that the flows of the purge gases into the detection vessel 10 have directional qualities.

A first common concentrating-mechanism 34 located at the eject-side edge of the virus introducing-tube-A is connected to the inlet-canal of the detection vessel 10 from the left side, like the structure illustrated in FIG. 28 basically. A group of the quintuple divided concentrating-mechanisms 34ₙ₁, 34ₙ₂, 34ₙ₃, 34ₙ₄, 34ₙ₅ illustrated in FIG. 34 corresponds to the second common concentrating-mechanism 34n according to the virus test system pertaining to the seventh derived-embodiment recited in FIG. 28. In the following description of the virus test device pertaining to the eighth derived-embodiment, the first common concentrating-mechanism 34 is referred to as "the common concentrating-mechanism 34" simply. In the virus test system pertaining to the seventh derived-embodiment recited in FIG. 28, the architecture encompassing the first inlet-canal cut in the outer wall of the left side of the detection vessel 10, and the second inlet-canal cut in the ceiling of the detection vessel 10, has been explained. On the other hand, in the structure pertaining to the eighth derived-embodiment, which has the quintuple divided concentrating-mechanisms 34ₙ₁, 34ₙ₂, 34ₙ₃, 34ₙ₄, 34ₙ₅ assigning quintuple nozzles, respectively, as illustrated in FIG. 34, it is preferable that the nozzle group implemented by quintuple nozzles shall be allocated in an inner space of the detection vessel 10, and therefore, the second inlet-canal in the ceiling of the detection vessel 10 can be omitted. As a result, the number of the openings which are cut in the outer wall of the left side of the detection vessel 10 is one, and the single opening will be referred as "the inlet-canal" simply, in the description of the virus test device pertaining to the eighth derived-embodiment illustrated in FIG. 34.

In the schematic diagram according to the virus test system pertaining to the seventh derived-embodiment recited in FIG. 28, one second common concentrating-mechanism --purging nozzle-- 34ₙ is schematic expressed by a single inverted-isosceles triangle comprehensively. As illustrated in FIG. 34 corresponding to the enlarged diagram illustrated in FIG. 28, the quintuple divided concentrating-mechanisms 34ₙ₁, 34ₙ₂, 34ₙ₃, 34ₙ₄, 34ₙ₅ are expressed as quintuple inverted-isosceles triangles which are provided at the upward of the inner wall of the detection vessel 10. Furthermore, as illustrated in FIG. 34, the moist-air introducing-tube- B, the dry-air bypass-tube-C, and the purge-gas introducing-tube-D are provided independently from the virus introducing-tube-A, like the piping system recited in FIG. 28 --entire of the moist-air introducing-tube- B, the dry-air bypass-tube-C, and the purge-gas introducing-tube-D are represented as a single line labeled by "B-D" in simplified and inclusive expression in FIG. 34--. That is, in FIG. 34, a symbol B-D is labeled to a single tube which is connected to a collecting tube in a quintuple-branch reducing-manifoldquintuple-barreled branched-coupling--, each of the branches is connected to the quintuple divided concentrating-mechanisms 34ₙ₁, 34ₙ₂, 34ₙ₃, 34ₙ₄, 34ₙ₅, respectively. The quintuple-branch reducing-manifold is provided at the top of the detection vessel 10. However, the single tube labeled with "B-D" shall be read as the tubes B to D, which are piping systems distinguishable from the virus introducing-tube-A. As understood from FIG. 28, the moist-air introducing-tube- B and the dry-air bypass-tube-C are connected to the middle of the purge-gas introducing-tube-D. Therefore, tubes B to D introduced to the upper portion of the detection vessel 10 are implemented by the moist-air introducing-tube- B, the dry-air bypass-tube-C and the purge-gas introducing-tube-D.

As illustrated in FIG. 34, the reducing-manifold having quintuple branches, each of which is connected to the quintuple divided concentrating-mechanisms 34ₙ₁, 34ₙ₂, 34ₙ₃, 34ₙ₄, 34ₙ₅, respectively, is provided at an outside of the detection vessel 10. However, the quintuple-branch reducing-manifold may be integrated into a single piece as a partial member of the cell-enclosure implementing the detection vessel 10. The portion to which the quintuple-branch reducing-manifold is merged may be the ceiling side, etc., of the detection vessel 10, for example. Furthermore, the whole of the quintuple-branch reducing-manifold may be provided in the inside of the detection vessel 10, for example, at the ceiling side in the cell-enclosure which implements the detection vessel 10. In the above description of the virus test device pertaining to the eighth derived-embodiment, a sentence that "the second inlet-canal in the ceiling of the detection vessel 10 can be omitted" has already stated. However, if an architecture that facilitates the replacement of the detection cells, by movingthrough contact-sliding movement-- the detection vessel relatively to the gas piping system as represented by FIGs. 7, 8, 13, etc., for example, is desired, a reducing-manifold having a multi-barreled branch such as quintuple-barreled branch, etc., may be provided in the inside of the detection vessel 10, and then, an edge of the collecting tube in the reducing-manifold having multi-barreled branch may be assigned as the second inlet-canal.

A scheme of relatively moving, by contact-sliding with the detection vessel, to the gas piping system for exchanging the detection cells, while maintaining a hermetically sealed state, is achieved by connecting an edge of the collecting tube in the multibranched reducing-manifold to the second inlet-canal. The scheme for exchanging the detection cells shall be provided with a structure which facilitates the alignment to the end port of the gas piping system at the outside. An additional scheme, in which each of the quintuple divided concentrating-mechanisms 34ₙ₁, 34ₙ₂, 34ₙ₃, 34ₙ₄, 34ₙ₅ has an inlet canal respectively, can be considered. However, the scheme having multiple inlet canals is not preferable, because the structure becomes complicated, and the problems of the leakage and the alignment, etc., will be generated. However, the additional scheme of providing a plurality of inlet-canals, which correspond to the quintuple divided concentrating-mechanisms 34ₙ₁, 34ₙ₂, 34ₙ₃, 34ₙ₄, 34ₙ₅ respectively, shall not be rejected absolutely in the virus test device pertaining to the eighth derived-embodiment.

As already described, "the first to third valve-ports" of the three-way valve are just addressing to the name only, for specifying the location in the piping circuit, and "the first to third valve-ports" do not always presuppose actual existences of the individual physical components or constituent elements independent from each other. In the same intent and meaning, if the modes of contact-sliding the detection vessel relatively to the gas piping system as represented by FIGs. 7, 8, 13, etc., are employed, the inlet-canal cut in the left side of the detection vessel 10 shall be the opening physically existing independently from the others. However, if the positional relationship between the gas piping system and the detection vessel 10 is fixed, the inlet-canal cut in the left side of the detection vessel 10 does not have the substantial signification. And therefore, the inlet-canal is just only the name illustrating the location at which the first common concentrating-mechanism 34 is connected to the detection vessel 10. For example, the first common concentrating-mechanism 34 and the detection vessel 10 may be merged into a single piece, and then, the structure that the AUT 31a is ejected as the high-speed air-flow through the first common concentrating-mechanism 34, may be constructed. If the first common concentrating-mechanism 34 and the detection vessel 10 are merged into the single piece, "the inlet-canal" which is cut in the detection vessel 10 is just only the name for convenience, for specifying the location in the piping circuit, and therefore, the actual existence of the individual member in the detection vessel 10, which is independent from the others physically, is not always presupposed.

In the virus test system and the virus test device pertaining to the eighth derived-embodiment illustrated in FIG. 34, the ejection nozzle having a shrinking tapered topology implementing the common concentrating-mechanism 34 for concentrating the target viruses contained in the AUT 31a from the virus introducing-tube-A and for ejecting the target viruses as the high-speed air-flows, is provided in the left side-wall of the detection vessel 10. In addition, the divided concentrating-mechanisms 34ₙ₁, 34ₙ₂, 34ₙ₃, 34ₙ₄, 34ₙ₅ for ejecting the purge gases as a shower-like beam are provided at the upper portion of the detection vessel 10, in addition to the common concentrating-mechanism 34. In the purging process, the purge gases, which are introduced via the purge-gas introducing-tube-D are introduced in the divided concentrating-mechanisms 34ₙ₁, 34ₙ₂, 34ₙ₃, 34ₙ₄, 34ₙ₅ through the introducing piping-path B-D. As illustrated in FIG. 34, for a structure in which the common concentrating-mechanism 34 is provided at the leftward of the detection vessel 10, it may be preferable that the divided concentrating-mechanisms 34ₙ₁, 34ₙ₂, 34ₙ₃, 34ₙ₄, 34ₙ₅ are allocated at the upward of the inner wall of the detection vessel 10. However, the topology illustrated in FIG. 34 is mare example, and therefore, the virus test system or the virus test device pertaining to the eighth derived-embodiment is not limited to the arrangement exemplified in FIG. 34. In addition, the number of the divided concentrating-mechanisms 34ₙ₁, 34ₙ₂, 34ₙ₃, 34ₙ₄, 34ₙ₅, ......... is not limited at five. Therefore, a plurality of purge-gas barrels of six or more may be provided concentrically on a top of the detection vessel 10, and the purge gases may be introduced via the top tips of the divided concentrating-mechanisms 34ₙ₁, 34ₙ₂, 34ₙ₃, 34ₙ₄, 34ₙ₅, ... ... ... , for pouring down the purge gases from the bottom tips of the divided concentrating-mechanisms 34ₙ₁, 34ₙ₂, 34ₙ₃, 34ₙ₄, 34ₙ₅, ... ... ... to the detector-substrate 11a like the shower.

In addition, a piping route passing through the testing-input valve 23e made of a three-way valve at an introducing side is connected to the input side of the detection vessel 10, and a piping route passing through a testing-output valve 23f made of a three-way valve at an exhausting side is connected to the output side of the detection vessel 10, basically in a similar fashion to the topology recited in FIG. 28. Although the detailed illustration of the piping system is omitted, the testing-input valve 23e is the same three-way valve as the testing-input valve 23e recited in the virus test system pertaining to the seventh derived-embodiment. Therefore, the calibration tube- E extends from the lower side in FIG. 34 to the first valve-port of the testing-input valve 23e implemented by the three-way valve at the introducing side, and the virus introducing-tube-A extends from the left side in FIG. 34 to the second valve-port of the testing-input valve 23e. Therefore, the AUT or the calibration-purpose aerosol is selected by controlling the testing-input valve 23e, and the selected AUT or the selected aerosol can be introduced to the detection vessel 10 through the third valve-port of the testing-input valve 23e.

As already mentioned, the NSA material can be removed efficiently from the roundtrip path of SAW, by flowing the purge-gas flow from the upper to the lower portion of the detection vessel 10, because the purge-gas introducing-tube-D is provided independently from the virus introducing-tube-A and the calibration tube- E in an analogous manner with the virus test device pertaining to the seventh derived-embodiment. By the way, if the purge-gas introducing-tube-D is the same as the virus introducing-tube-A, like the piping-path according to the virus test system pertaining to the fundamental embodiment recited in FIG. 1, it is not enough to remove the NSA material by blowing out from the detector-substrate 11a, because the pressure of the purge gas becomes lower. When the removal of the NSA material is not enough, the integrated difference response (Δt/t)_{Diff} ascribable to the sensor illustrated in FIG. 31, explained in the virus test system pertaining to the seventh derived-embodiment, becomes larger than the value of the case that only the target viruses are selectively adsorbed. The increase of the integrated difference response (Δt/t)_{Diff} becomes a nonnegligible measurement error in a domain that the concentration of the target viruses in AUT 31a is lower, and further, the absolute value of the integrated difference response (Δt/t)_{Diff} is smaller.

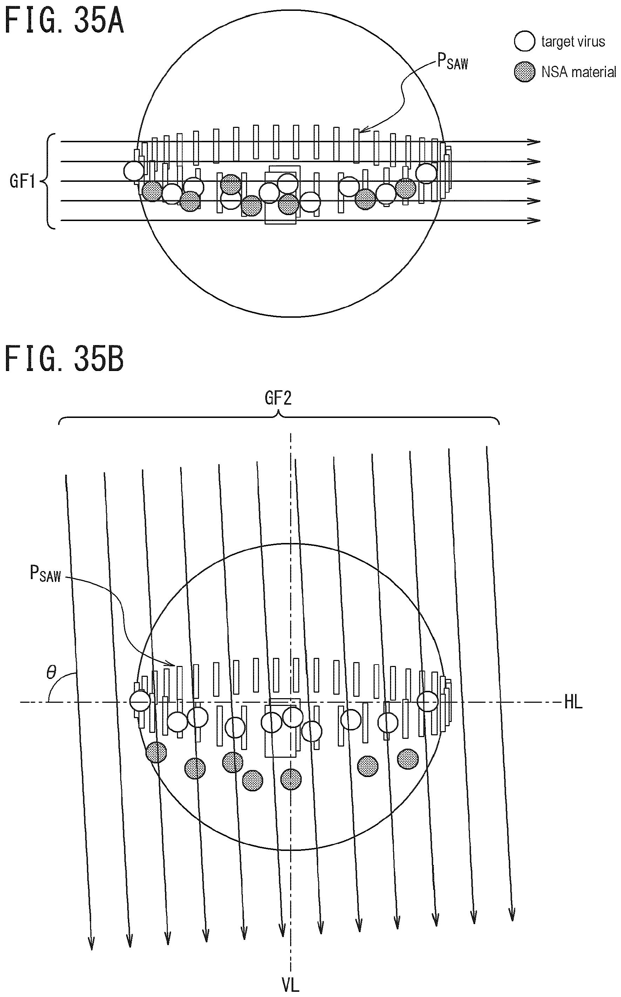

As illustrated in FIG. 35A, jet gas-streams DF₁ are flowed from the leftward to rightward of the detection vessel 10 through the common concentrating-mechanism 34 when the AUT 31a is ejected, by controlling the testing-input valve 23e, the testing-output valve 23f, and the suction pump 40, etc. In addition, as illustrated in FIG. 35B, the purging gas-streams GF₂ are flowed from the tips of the divided concentrating-mechanisms 34ₙ₁, 34ₙ₂, 34ₙ₃, 34ₙ₄, 34ₙ₅, ........., which are concentrically arranged at the top of the detection vessel 10, respectively, to the downward of the detection vessel 10, when the purging process is executed. For generating the shower-like beam directing uniformly toward the downward, the exhaust tube B-D is preferable to be connected to the exhaust-canal of the lower portion of the detection vessel 10 as illustrated in FIG. 34. As to the exhaust tube B-D, the detailed representations of the concrete exhaust tube B to the concrete exhaust tube D are omitted in FIG. 34. The cleaned dry-air and the cleaned moist-air are also exhausted through the exhaust tube B-D, in addition to the purge gas.

In the purging process, the purge gases which are ejected from the tips of the divided concentrating-mechanisms 34ₙ₁, 34ₙ₂, 34ₙ₃, 34ₙ₄, 34ₙ₅, ......... respectively become the flows of the shower-like beam toward the downward, by evacuating the inside of the detection vessel 10 through the exhaust tube B-D using the suction pump 40. At an instant of ejecting the purge gas from the tips of the divided concentrating-mechanisms 34ₙ₁, 34ₙ₂, 34ₙ₃, 34ₙ₄, 34ₙ₅, ... ... ... respectively, simultaneously the purge gas can be humidified by controlling the humidification-input valve 23a located at the downstream side of the MFC 22. And, the purging process may be implemented by a double-phase scheme including a first phase of the purge using the cleaned moist-air and a second phase of the purge using the cleaned dry-air.

As illustrated in FIG. 35A, when the jet gas-stream DF₁, which are ejected from the common concentrating-mechanism 34, flows from the left to the right along roundtrip path of SAW P_{SAW}, the target viruses and the NSA material are adsorbed to the roundtrip path of SAW P_{SAW}, and have the distribution along roundtrip path of SAW P_{SAW} in the virus test system pertaining to the eighth derived-embodiment. Due to the adsorption of the target viruses and the NSA material on the roundtrip path of SAW P_{SAW}, the sensor response Δt/t represents the maximum value, as understood from FIG. 31, which was explained in the virus test system pertaining to the seventh derived-embodiment. In addition, as illustrated in FIG. 35B, through the tips of the divided concentrating-mechanisms 34ₙ₁, 34ₙ₂, 34ₙ₃, 34ₙ₄, 34ₙ₅, ........., which are concentrically arranged at the top of the detection vessel 10, the purging gas-streams flow from the upward toward the downward of the detection vessel 10, in the direction which is orthogonal to or almost orthogonal to the roundtrip path of SAW P_{SAW}. As the result, although the target viruses caught by the antibodies on the surface of the detector-substrate 11a cannot move from the adsorbed state, the NSA material is removed from the roundtrip path of SAW P_{SAW}, and the removed NSA material flows toward the downward. Therefore, the NSA material can be eliminated from the roundtrip path of SAW P_{SAW}. Therefore, in the time interval of the step S303 located at a center of the response-curve in FIG. 31, the intensity of sensor response begins decreasing, and the intensity approaches gradually to a constant value, which corresponds to the virus concentration in the environment, as illustrated by the variation of the transient response-curve.

And, for an objective to complete the measurements of the virus concentrations in shorter time by removing the NSA material more quickly. it is preferable to control the direction of the purge-gas flow as illustrated in FIG. 35B. Furthermore, the directions of the purge-gas flows which are ejected from the tips of the divided concentrating-mechanisms 34ₙ₁, 34ₙ₂, 34ₙ₃, 34ₙ₄, 34ₙ₅, ........., which are provided at the upper portion of the detection vessel 10, are preferable to be controlled to a direction almost parallel to the vertical line VL illustrated in FIG. 35B. Concretely, the directions of the purge-gas flows are acceptable to be controlled within an angle θ larger than 20 degrees and equal to or lower than 90 degrees to roundtrip path of SAW P_{SAW}, or the horizontal line HL as illustrated in FIG. 35B. For controlling the directions of the purge-gas flows in the angle θ from the horizontal line HL, a tool for adjusting the ejecting angle may be provided to the divided concentrating-mechanisms 34ₙ₁, 34ₙ₂, 34ₙ₃, 34ₙ₄, 34ₙ₅, .........., respectively.

As described above, ejecting additionally the calibration-purpose aerosols containing the calibration-purpose virus is desirable, the concentration of the calibration-purpose aerosols is set to a plurality of values from the common concentrating-mechanism 34 to the detector-substrate 11a, by switching the testing-input valve 23e serving as an introducing-side three-way valve, according to the virus test system pertaining to the eighth derived-embodiment. Furthermore, the calibration of sensor can be executed, by measuring the multiple responses, after an air including the calibration-purpose virus is ejected to the pseudo-receptor film in the detection vessel 10. Therefore, the difference-integral detection can be executed with a higher precision, as the delay-time responses, for example, from the ball SAW sensor, according to the virus test system pertaining to the eighth derived-embodiment. Then, according to the virus test system pertaining to the eighth derived-embodiment, the difference-integral detection can be executed with the high reliability, compared to the virus test device pertaining to the fundamental embodiment, in an analogous manner with the virus test system pertaining to the seventh derived-embodiment.

By the way, the testing-input valve 23e serving as an introducing-side three-way valve may be omitted, and the virus introducing-tube-A only is connected to the first inlet-canal of the left side of the detection vessel 10 so that the gas piping system can be simplified in the structure illustrated in FIG. 34. In the simplified scheme omitting the introducing-side three-way valve, the calibration of sensor cannot be executed, as the calibration tube- E is omitted. However, the possibility of the contamination of the cleaned moist-air or the cleaned dry-air with the viruses can be eliminated, ascribable to a feature that the purge-gas introducing-tube-D is provided independently from the virus introducing-tube-A. Furthermore, the problem that the purge gas pressure becomes lower can be avoided, by providing the purge-gas introducing-tube-D independent from the virus introducing-tube-A. As the result, the NSA material from the roundtrip path of SAW can be removed efficiently, by using the purge gases from the divided concentrating-mechanisms 34ₙ₁, 34ₙ₂, 34ₙ₃, 34ₙ₄, 34ₙ₅, ........., which serves as a plurality of nozzles. Therefore, the effectiveness that the difference-integral detection with the higher precision is executed can be achieved, even with the virus test device having the simplified structure, in which the introducing-side three-way valve is omitted, because the measurement error due to the NSA material is reduced.

### (OTHER EMBODIMENTS)

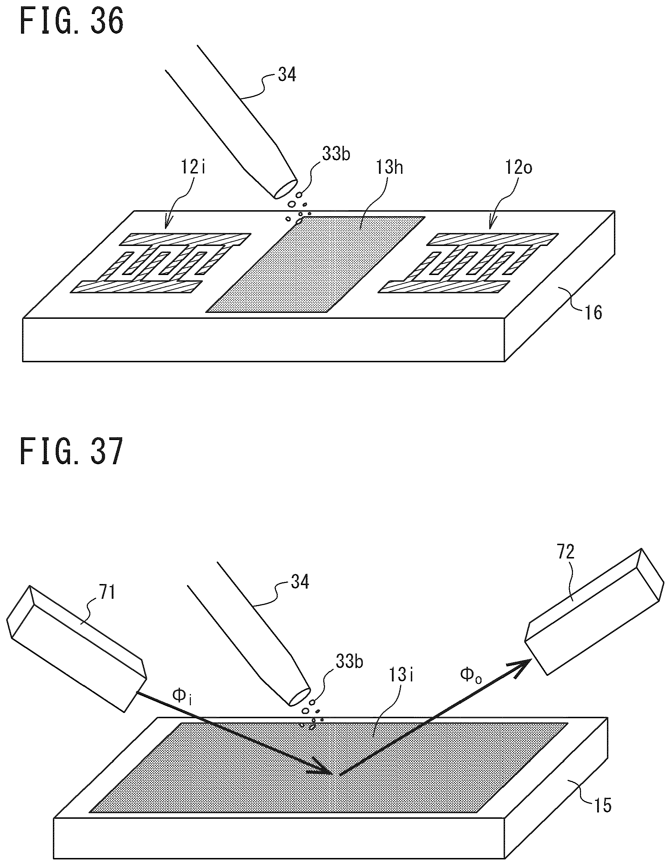

Although the intent and the general concept of the present invention are explained by the above fundamental embodiment and the first to eighth derived-embodiments, the description, and the drawing implementing part of this disclosure, should not be construed as limiting the present invention. The persons skilled in the art can produce the various embodiments, the various modifications, and the various application techniques depending on the above disclosure. Furthermore, although, according to the virus test system pertaining to the fundamental embodiment and the first to eighth derived-embodiments, the case that the signal conditioner which detects the change of the surface states of the pseudo-receptor film physically is the sensor electrode of the ball SAW sensor, is explained exemplify, the signal conditioner is not limited the sensor electrode of the ball SAW sensor, and a signal conditioner may be a set of an input electrode 12ᵢ and an output electrode 12ₒ of a planar SAW sensor as illustrated in FIG. 36.

The planar SAW sensor illustrated in FIG. 36 has the input electrode 12ᵢ, a pseudo-receptor film 13ₕ, and the output electrode 12ₒ, which are provided respectively in the predetermined areas, each of which is defined on the surface of the detector-substrate 16 made of a homogeneous piezoelectric material substrate. As illustrated in FIG. 36, the pseudo-receptor film 13ₕ, which is provided at least on one part of the surface of the detector-substrate 16, is the sensitive film for the SAW. Pseudo-receptors which are scheduled to be specifically bound to the viruses are provided on the surface the pseudo-receptor film 13ₕ. In the state that the pseudo-receptors are arranged on the pseudo-receptor film 13ₕ, the SAW propagates in the pseudo-receptor film 13ₕ. In the configuration illustrated in FIG. 36, the signal conditioner (12ᵢ, 12ₒ) of the present invention encompasses the set of the input electrode 12i and the output electrode 12o. The signal conditioner (12ᵢ, 12ₒ) corresponds to an architecture that the single sensor electrode implements the signal conditioner 12, in the structure of the ball SAW sensor exemplified in FIG. 2. In the planar SAW sensor as illustrated in FIG. 36, the signal conditioner (12ᵢ, 12ₒ) measures variations of the physical signals representing the variations of the physical states of the pseudo-receptor film 13ₕ, due to the specific binding of the pseudo-receptors 14 to the target viruses, by using the electro-acoustic conversion. As the result, the difference-integral detection of the difference of areal densities by weight of the pseudo-receptor film 13ₕ due to the viruses bound to the pseudo-receptors can be executed, as the delay-time response of the planar SAW sensor (16, 12ᵢ, 12ₒ, 13ₕ). And in order to execute the difference-integral detection, a hermetic container which blocks from the outside shall be prepared as a detection vessel, and the viruses contained in the AUT are detected by constructing the hermetically closed space and by placing the AUT in the detection vessel with the planar SAW sensor (16, 12ᵢ, 12ₒ, 13ₕ) illustrated in FIG. 36.

In addition, the signal conditioner of the present invention is not limited to the sensor electrode of the ultrasonic sensor, such as the sensor electrode of the ball SAW sensor, the planar SAW sensor, etc. And the signal conditioner may be a set of an irradiation apparatus 71 and a photodetection apparatus 72 as an optical sensor as illustrated in FIG. 37. The optical sensor illustrated in FIG. 37 has the irradiation apparatus 71 such as a semiconductor laser, etc., which irradiates the detection light to the pseudo-receptor film 13ᵢ provided in the predetermined area on the surface of the detector-substrate 15 made of a homogeneous plate, and the photodetection apparatus 72 such as a photo diode, etc., which photoelectrically detects the detection light reflected at the pseudo-receptor film 13ᵢ. Namely, in the configuration illustrated in FIG. 37, the signal conditioner (71, 72) of the present invention encompasses the set of the irradiation apparatus 71 and the photodetection apparatus 72. The signal conditioner (71, 72) corresponds to the single sensor electrode implementing the signal conditioner 12, in the architecture of the ball SAW sensor exemplified in FIG. 2. In the configuration illustrated in FIG. 37, a detection cell is constructed by a detection vessel (the illustration is omitted), and the planar SAW sensor (16, 12ᵢ, 12ₒ, 13ₕ) and the signal conditioner (71, 72), etc., which are housed in the detection vessel.

In the fundamental embodiment and the first to eighth derived-embodiments, the variations of the areal densities by weight of the pseudo-receptor film 13, ascribable to the specific binding of the pseudo-receptors 14 to the target viruses, is explained as the objective physical signals. However, the descriptions of the fundamental embodiment and the first to eighth derived-embodiments are mere examples. And therefore, "the variations of the physical states of the pseudo-receptor film" intended by the present invention is not limited to the variations of the areal densities by weight, which are exemplified in the fundamental embodiment and the first to eighth derived-embodiments. For example, in the optical sensor as illustrated in FIG. 37, the optical signals representing variations of the physical states of the pseudo-receptor film 13ᵢ, ascribable to the specific binding of the pseudo-receptors 14 to the target viruses, are converted to the corresponding electric signals by the signal conditioner (71, 72). As illustrated in FIG. 37, the pseudo-receptor film 13ᵢ provided at least on a part of the surface of the detector-substrate 15 arranges the pseudo-receptors, which are scheduled to bind specifically to the viruses, on the surface of the detector-substrate 15. In the virus test system pertaining to a still another embodiment illustrated in FIG. 37, the viruses which are bound to the pseudo-receptors contributes to the signals representing variations of the physical states of the pseudo-receptor film 13ᵢ, due to the various changes of a surface morphology, a polarization property, a reflection coefficient, and a scattering characteristic, etc. of the pseudo-receptor film 13ᵢ. Namely, in the architecture of the optical sensor as illustrated in FIG. 37, the optical signals representing the changes of the surface morphology, the polarization property, the reflection coefficient, and the scattering characteristic, etc. of the pseudo-receptor film 13ᵢ, on account of the viruses, are converted to the corresponding electric signals by the signal conditioner (71, 72). And, the viruses bound to the pseudo-receptors can be detected, by executing the arithmetic process of the signal processor, of which the illustration is omitted, utilizing the electric signals transmitted from the signal conditioner (71, 72).

For detecting the changes of the surface morphology, the polarization property, the reflection coefficient, and the scattering characteristic, etc. of the pseudo-receptor film 13ᵢ, a hermetic container which blocks from the outside shall be prepared as the detection vessel, so that the viruses contained in the AUT can be detected, by constructing a hermetically closed space and by placing the AUT in the detection vessel with the detector-substrate 15 illustrated in FIG. 37. In the configuration illustrated in FIG. 37, the detection cell is built by a detection vessel (the illustration is omitted), and the detector-substrate 15, the pseudo-receptor film 13ᵢ, the signal conditioner (71, 72), etc., which are housed in the detection vessel.
If a window penetrating a specific wavelength of the optical signals utilized by the signal conditioner (71, 72) is provided at least a part of a detection vessel, a signal conditioner (71, 72) can be provided at an outside of the detection vessel, which encapsulates the pseudo-receptor film 13ᵢ. If a reflection mirror is provided at an upper portion of the structure represented in FIG. 37, an optical sensor utilizing a multi-reflection can be constructed. Although the exemplification is omitted, the optical sensor may be a transmission-based optical sensor. And if applicating the transmission-based optical sensor, the detector-substrate 15 illustrated in FIG. 37 shall be made of material transparent for the wavelength of the detection light, off course. Enzyme-Linked Immunosorbent Assay (ELISA) method can be classified into the category of the transmission-based optical sensor, because absorbances of the pigments ascribable to the reactions on the surface of the pseudo-receptor film are measured in ELISA method.

If the signal conditioner (71, 72) is implemented by the set of the irradiation apparatus 71 and the photodetection apparatus 72 illustrated in FIG. 37, the light irradiated from the irradiation apparatus 71 can use a light having the wavelength, for example, from far-ultraviolet to far-infrared spectrum. Furthermore, since light is electromagnetic wave, a case that the irradiation apparatus 71 irradiates an electromagnetic wave of terahertz band, the photodetection apparatus 72 receives the electromagnetic wave, and the electromagnetic wave are converted to the corresponding electric signals. The photodetection apparatus 72 implementing the signal conditioner (71, 72) can be deemed an apparatus which executes electro-electro conversion, when the signal of the electromagnetic wave is adjudged as the electric signal. In the case of using the electromagnetic wave of terahertz band, the signal conditioner (71, 72) can be allocated at an outside of the detection vessel which encapsulates the pseudo-receptor film, if the detection vessel has a window, which is transparent for the electromagnetic wave of terahertz band, at least a part of detection vessel.

If the irradiation apparatus 71 irradiates the electromagnetic wave of terahertz band, the measurement of meta-material can be executed, when patterns of the pseudo-receptors 14 having sub-wavelength structure, which is a pattern having dimension shorter than the wavelength of the electromagnetic wave, are delineated by photolithography technique, etc. Concretely, if a variant of the target virus emerges, since the molecular structure of the target virus changes, the vibration frequency peculiar to the molecule of the target virus also changes. If the signal conditioner (71, 72) is implemented by the set of the irradiation apparatus 71 and the photodetection apparatus 72 illustrated in FIG. 37, the variant virus can be specified by identifying the resonance frequency, if the electromagnetic wave having a frequency, which resonates to a frequency peculiar to the molecule vibration of the target variant viruses, is irradiated to the target viruses which are specifically bound to the pseudo-receptors 14, utilizing a wide-band irradiation apparatus 71, which can irradiate with variable frequency.

Namely, the physical changes of the pseudo-receptor film due to the specific bindings of the pseudo-receptors to the target viruses include the changes of the absorption property and the changes of the eigenfrequencies of the pseudo-receptor film. In the electro-electro conversion in which the irradiation apparatus 71 irradiates the electromagnetic wave and the photodetection apparatus 72 is receives the electromagnetic wave, as illustrated in FIG. 37, the electric signals which are received by the photodetection apparatus 72 may be the changes of the impedance. For example, a scattering parameter (S-parameter) measurement system may implement the set of the irradiation apparatus 71 and the photodetection apparatus 72. Therefore, the physical change of the pseudo-receptor film ascribable to the specific binding of the pseudo-receptors to the target viruses includes the changes of the impedance property and the changes of the S-parameter.

The signal conditioners adapted for the ultrasonic sensor, the optical sensor, and the electromagnetic wave sensor, are not limited to the above-described electroacoustic converter, the above-described photoelectric converter, and the above-described electro-electro converter, as described above. For example, the signal conditioners may be the physical-signal vs. electric-signal converter, which is used as a surface plasmon sensor, a quartz crystal microbalance (QCM) sensor, and so on. Furthermore, the signal conditioner may be the physical-signal vs. electric-signal converter, which is used as a field effect transistor (FET)-based biosensor using ion-sensitive FET or a Micro Electro Mechanical Systems (MEMS)-based surface-stress biosensor, etc. Namely, the variations of the surface states of the pseudo-receptor film can be detected by the arithmetic process of the signal processor in which the illustration is omitted, by converting the physical signals detected physically, employing the FET-based biosensor or the MEMS-based surface-stress biosensor, etc., to the corresponding electric signals utilizing the signal conditioner. Even in various cases addressing to miscellaneous apparatuses other than the above-described ultrasonic sensor, the above-described optical sensor, and the above-described electromagnetic sensor, a hermetic container which blocks from the outside shall be prepared as a detection vessel, and a detector-substrate shall be allocated in the detection vessel, under the condition that a pseudo-receptor film is laminated on the detector-substrate.

Furthermore, according to the virus test system pertaining to the fundamental embodiment and the first to eighth derived-embodiment, the cases that the hydrous aerosols are the aerosols-under-test are described, respectively. However, the aerosols-under-test are not always necessary to be the hydrous aerosols. The hydrous rate in the aerosols-under-test may alter depending on whether the main transmission risks owing to the target viruses 60 is airborne infection, droplet infection, or micro-droplets-infection. Furthermore, for example, the main transmission risk is ascribable to the airborne infection, the target viruses 60 may be ejected to the pseudo-receptor film directly with the hydrous rate of zero.

Furthermore, in the virus test system pertaining to the fundamental embodiment and the first to eighth derived-embodiment, the cases of the difference-integral detections which detect whether the target viruses 60 exist or not in the aerosols-under-test 33b are described mainly. However, the technical concept explained in the virus test system pertaining to the fundamental embodiment and the first to eighth derived-embodiment can be applicable to a case that the target viruses 60 are contained in a liquid. Namely, the difference-integral detection can be executed easily for detecting whether the target viruses 60 exist or not in the liquid, by providing a mechanism which changes the liquid to the gas, or the hydrous aerosols in the virus test device pertaining to the fundamental embodiment and the first to eighth derived-embodiment, and by using the air generated by the above gasification process as the aerosols-under-test 33b.

Furthermore, in the description of the virus test device pertaining to the third derived-embodiment of the present invention, although the structure having the double sensors of the first ball SAW sensor (11p, 12a, 13a, 14) and the second ball SAW sensor (11q, 12b, 13b) is presented as recited in FIG. 18. However, the feature of the double sensors may be modified to a structure having triple sensors or more sensors. In the structure having the triple sensors, a third pseudo-receptor film, which addresses to pseudo-receptors scheduled to be bound to different viruses from the viruses bound to the first pseudo-receptor film 13a, is provided at a third ball SAW sensor. Then, attenuation coefficient and delay time of the SAW which propagates on the third pseudo-receptor film can be measured, by providing a third sensor electrode as a third signal conditioner in the third ball SAW sensor, and by converting acoustic signals to corresponding electric signals using the third sensor electrode. Then, SARS-CoV-2 virus and influenza virus can be identified, for example, when applicating an architecture that the first pseudo-receptor film 13a is used to detect the SARS-CoV-2 virus as the target viruses (first target viruses) 60, and the third pseudo-receptor film is used to detect the influenza virus as the second target viruses.

Similarly, in the description of the virus test device pertaining to the sixth derived-embodiment of the present invention, although a scheme that the first detector-substrate 11j, the third detector-substrate 111, and the fifth detector-substrate 11n are prepared as the triple moisture-detection spheres, and the second detector-substrate 11k, the fourth detector-substrate 11m, and the sixth detector-substrate 11o are prepared as the triple virus-testing spheres. In the scheme, the triple moisture-detection spheres and the triple virus-testing spheres are inserted alternately in the set of triple pieces, as exemplified in FIG. 27. However, the SARS-CoV-2 virus and the influenza virus can be identified, when a scheme that the first detector-substrate 11j, the third detector-substrate 111, and the fifth detector-substrate 11n are prepared as triple influenza virus-testing spheres, and the second detector-substrate 11k, the fourth detector-substrate 11m, and the sixth detector-substrate 11o are prepared as triple SARS-CoV-2 virus-testing spheres is employed such that the triple influenza virus-testing spheres and the triple SARS-CoV-2 virus-testing spheres are inserted alternately in the set of triple pieces.

Furthermore, in the structure recited in FIG. 37, a plurality of different species of viruses can be identified, respectively, when the detector-substrate 15 is translationally moved as a belt conveyor system by periodically arranging a plurality of the pseudo-receptor films on the detector-substrate 15 along the one direction on a conveyer belt. Namely, a first pseudo-receptor film being arranged with first pseudo-receptors which is scheduled to be specifically bound to a first virus, a second pseudo-receptor film being arranged with second pseudo-receptors which is scheduled to be specifically bound a second virus, and a third pseudo-receptor film being arranged with third pseudo-receptors which is scheduled to be specifically bound a third virus, ...... are sequentially arranged on the conveyer belt. Or alternatively, a plurality of the pseudo-receptor films may be rotationally moved, by arranging a plurality of the pseudo-receptor films on the circumference surface of the rotary disc periodically.

Furthermore, it is possible to create a new substitutional embodiment by suitably electing a set of technical concepts from the group explained in the fundamental embodiment and the first to eighth derived-embodiments, and combining appropriately the elected technical concepts. For example, calibrated virus-testing spheres which are calibrated by the sensitivity calibration method described in the seventh derived-embodiment, may be used as the virus-testing spheres for the fundamental embodiment and the first to sixth derived-embodiments. Furthermore, in the seventh derived-embodiment, a scheme that the three-dimensional area AR surrounded by the dot-dashed line illustrated in FIG. 29 is integrally molded by the molding apparatus of the 3D printer, etc., is explained. The scheme of miniaturization and integration technique by the integrally molding can be also applicable to the techniques explained in the fundamental embodiment and the first to sixth derived-embodiments. Therefore, a portable virus test system can be realized, by more and more downsizing the virus test system. The downsizing of the virus test system may include miniaturizations of the virus test system, etc., explained in the fundamental embodiment and the first to sixth derived-embodiments.

Furthermore, a ubiquitous virus test system can be realized, which is miniaturized to a size mountable in a portable terminal, etc., by preparing a down-sized suction pump, down-sized piping systems, etc. manufactured by MEMS. Or alternatively, the technical concept of pouring down the purge gases to the detector-substrate in a form of shower from a plurality of divided concentrating-mechanisms 34ₙ₁, 34ₙ₂, 34ₙ₃, 34ₙ₄, 34ₙ₅, ......... described in the eighth derived-embodiment can be used as purging schemes for the virus-testing spheres described in the fundamental embodiment and the first to sixth derived-embodiments, etc. Therefore, various combinations of embodiments are feasible depending on each of the technical concepts recited in the fundamental embodiment and the first to eighth derived-embodiments. As described above, the present invention of course includes various embodiments and modifications and the like which are not detailed in the fundamental embodiment and the first to eighth derived-embodiments. Thus, the technical scopes of the present invention should be determined only by the technical features specifying the invention prescribed by following Claims, conceivable and reasonable from the above explanations.

### [Industrial Applicability]

As stated in the beginning section of the instant specification, according to Infectious Agents Surveillance Report (IASR) which is announced by National Institute of Infectious Diseases (NIID) regularly, multiple pieces of the pathogenic human coronaviruses such as HCoV-OC43, HCoV-229E, HCoV-NL63, HCoV-HKU1, etc., illustrated in the table. 1 have been detected every year from the year of 2019 or before. Among the viruses contributing to causes of the "common cold", the human coronaviruses are recognized to be responsible to about 10 to 15%. Namely, the humans entirely have been immersed deeply in the environment with pathogenic human coronaviruses from before 100 years or more. On July 16, 2021, Dr. Omi Shigeru, Chairman of New Coronavirus Infectious Diseases Control Subcommittee said "I think the times relying only on the movement-restrictions of people are all but over". Furthermore, Dr. Omi also refers to the importance of the science and technology of the epidemiological survey, etc., using Information and Communication Technology (ICT).

Ten to twenty thousand persons testing positive or more were reported every day in Japan, at the phase of August 2021. In the situation of such large number of tested positive persons are reported, the current infectious disease countermeasures, which is relied on PCR test, is approaching the limit. Since the virus test system of the present invention has a feature facilitating the miniaturization, the industrial applicability of the present invention is not limited to the technical scope of the assay equipment used in medical institutions. On account of the portable feature of the virus test system pertaining to the present invention, the present invention can be applied to the technology fields in the manufacturing business, such as manufacturing firms for fabricating business healthcare devices, which are regularly kept in eating and drinking establishments, public transportation systems, the large-scale attractions, etc., and goods-producing companies of the domestic healthcare apparatus, for example. Furthermore, if the miniaturization feasible features are promoted, the virus test system pertaining to the present invention can establish ubiquitous properties which are embeddable in the portable information devices directing to the 5G, 6G, 7G communication technologies, etc., therefore, the present invention can be applied to the technology field of the information and communication. Concretely, if the miniaturization or the integration feasible features are progressed to a level such that the virus test system can be merged in a smart phone, etc., it is possible to inform the result of virus infection to the user, while the user is under telephone conversation. If the resources of cloud computing are leveraged, a real-time data-base representing the state of viral diffusion process can be created by the government office, using the information from the portable information devices, in which the virus test system of the present invention are embedded, respectively. And therefore, effective infectious disease countermeasures utilizing ICT can be realized.

### [Reference Signs List]

A ...virus introducing-tube, 12, 12a, 12b...signal conditioner (sensor electrode), 12i ...signal conditioner (input electrode), 12o ...signal conditioner (output electrode), 13, 13a...pseudo-receptor film (first pseudo-receptor film), 13b... second pseudo-receptor film, 13g, 13h, 13i ...pseudo-receptor film, 14...pseudo-receptors, 31a, 31b...aerosols-under-test (AUT), 33b...AUT, 50...signal processor, 71...irradiation apparatus (signal conditioner), 72...photodetection apparatus (signal conditioner), 503...inspection module (logical circuit)

## Claims

1. A virus test device comprising:
a pseudo-receptor film (13) having a plurality of pseudo-receptors arranged on the pseudo-receptor film, each of the pseudo-receptors (14) mimicking a structure of a host-cell receptor, which binds specifically to a target virus;
a virus introducing-tube (A), configured to suck down an air-under-test containing the target viruses, to compress the air-under-test into a high-speed air-flow of aerosols-under-test, concentrating the target viruses contained in the air-under-test, and to eject the high-speed air-flow to the pseudo-receptor film;
a dry-air bypass-tube (C) configured to generate a cleaned dry-air;
a moist-air introducing-tube (B) configured to generate a cleaned moist-air and supply the cleaned moist-air to the pseudo-receptor film (13);
a ball SAW sensor (1003) including a sensor electrode as a signal conditioner, the signal conditioner, configured to convert physical signals, which represent alterations of physical states of the pseudo-receptor film ascribable to specific bindings of the pseudo-receptors with the target viruses, to electric signals; and
a detection vessel (10) constructing a hermetically closed space for housing the ball SAW sensor (1003),
wherein the physical signals represent variations of areal densities by weight of the pseudo-receptor film ascribable to the specific bindings of the pseudo-receptors with the target viruses, thereby the signal conditioner converts the physical signals to the electric signals,
wherein the pseudo-receptor film (13) is a sensitive film of a surface acoustic wave, the pseudo-receptor film is provided on at least a part in a surface of a piezoelectric-crystal sphere, and the surface acoustic wave propagates in the sensitive film,
wherein the ball SAW sensor (1003) has the piezoelectric-crystal sphere, and the pseudo-receptor film (13) and the sensor electrode which are provided on at least the part in the surface of the piezoelectric-crystal sphere.

2. The virus test device of claim 1, wherein the virus introducing-tube (A) has a filter (30a) disposed at an entrance side thereof and a concentrating-mechanism (34) with a tapered nozzle structure disposed at an exit side thereof,
wherein, a position of a tip of the concentrating-mechanism (34) mates with an inlet-canal, which is cut in an outer wall of the detection vessel, and the high-speed air-flow is ejected to the pseudo-receptor film (13) from the tip.

3. The virus test device of claim 2, wherein the moist-air introducing-tube (B) includes a path of introducing the cleaned moist-air to an inside of the detection vessel (10), and
the dry-air bypass-tube (C) includes a path of introducing the cleaned dry-air to the inside of the detection vessel (10).

4. The virus test device of claim 3, wherein the concentrating-mechanism (34) of the virus introducing-tube (A) is assigned as a dedicated virus concentrating-mechanism (34) for the target viruses, the virus test device further comprising:
a moist-air concentrating-mechanism (34c) with a tapered nozzle structure at an exit side of the moist-air introducing-tube; and
a dry-air concentrating-mechanism (34v) with a tapered nozzle structure at an exit side of the dry-air bypass-tube,
wherein, the detection vessel (10) moves relatively to the virus-concentrating-mechanism, the moist-air concentrating-mechanism (34c) and the dry-air concentrating-mechanism (34v), respectively, so that each of the tips of the virus-concentrating-mechanism, the moist-air concentrating-mechanism (34c) and the dry-air concentrating-mechanism (34v) can mate with the inlet-canal, respectively.

5. The virus test device of claim 3, wherein the concentrating-mechanism (34) serves as a common concentrating-mechanism (34),
an exit side of the moist-air introducing-tube (B) is connected to the virus introducing-tube (A) via a branched tube of the virus introducing-tube (A), the branched tube is assigned at an intermediate position between the common concentrating-mechanism (34) and an on-off valve provided in the virus introducing-tube (A),
an exit side of the dry-air bypass-tube (C) is connected to the moist-air introducing-tube (B) through a humidification-output valve so that the dry-air bypass-tube serves as a branched tube of the moist-air introducing-tube, the humidification-output valve is provided at an intermediate position in the moist-air introducing-tube, and
the target viruses, the cleaned moist air and the cleaned dry-air are sequential ejected to the pseudo-receptor film (13) from the common concentrating-mechanism at different timings, respectively, by switching piping-paths, via operations of the on-off valve and the humidification-output valve.

6. The virus test device of claim 3, further comprising:
a first three-way valve having first, second and third valve-ports;
a second three-way valve having first, second and third valve-ports;
a calibration tube, through which calibration-aerosols containing viruses with a prescribed concentration flow, connected to the first valve-port of the first three-way valve;
wherein, the second valve-port of the first three-way valve is connected to the virus introducing-tube, through which the air-under-test flows,
the third valve-port of the first three-way valve is connected to the detection vessel via the first common concentrating-mechanism,
the calibration-aerosols and the air-under-test are ejected to the pseudo-receptor film through the first common concentrating-mechanism at different timings, respectively, by switching piping-paths via the first three-way valve,
the moist-air introducing-tube (B) is connected to the first valve-port of the second three-way valve, and the dry-air bypass-tube is connected to the second valve-port of the second three-way valve,
the third valve-port of the second three-way valve is connected to a purge-gas introducing-tube (D) which introduces the purge-gas into the detection vessel,
a tip of a second common concentrating-mechanism, which is provided at an exit end of the purge-gas introducing-tube (D), mates with a second inlet-canal, which is cut at another portion of the outer wall of the detection vessel (10),
so that the cleaned moist air, the purge gas and the cleaned dry-air are ejected to the pseudo-receptor film from the second common concentrating-mechanism at different timings, respectively, by switching piping-paths via the second three-way valve.

7. The virus test device of claim 3, further comprising:
a three-way valve having first, second and third valve-ports;
a purge-gas introducing-tube (D) connected to the third valve-port, configured to define a path for introducing a purge gas into the detection vessel (10); and
a plurality of separated concentrating-mechanisms, the separated concentrating-mechanisms are reduced into a single entrance tube connected to an exit end of the purge-gas introducing-tube (D), the separated concentrating-mechanism being disposed at other portions of the outer wall of the detection vessel (10) than a portion where the inlet-canal is cut,
wherein, the moist-air introducing-tube (B) is connected to the first valve-port, and the dry-air bypass-tube is connected to the second valve-port so that the cleaned moist air, the purge gas and the cleaned dry-air are ejected to the pseudo-receptor film (13) from the separated concentrating-mechanisms at different timings, respectively by switching piping-paths by the three-way valve, and
a gas direction, along which purge gases are ejected from the concentrating-mechanisms, is controlled within an angle between 20 degrees or more and 90 degrees or less, measured from a long axis direction of the pseudo-receptor film (13).

8. The virus test device of claim 1, wherein the detection vessel (10) includes:
a body of detection vessel, and
a susceptor (1001) for mounting a subject ball SAW sensor and a replacing ball SAW sensor, a limited part of the susceptor (1001) is detachably inserted upward from a bottom of the body of the detection vessel (10), so that the subject ball SAW sensor (1003) or the replacing ball SAW sensor is selectively inserted in the body of detection vessel (10),
wherein, the replacing ball SAW sensor has same structure and same size as the subject ball SAW sensor (1003), the subject ball SAW sensor is replaced to the replacing ball SAW sensor by moving the susceptor (1001) relatively to the body of the detection vessel (10).

9. A virus test system comprising:
the virus test device of claim 1; and
a signal processor (50), configured to drive the signal conditioner, to execute a difference-integral detection based upon an output data from the signal conditioner for detecting existences of the specific bindings of the pseudo-receptors (14) and the target viruses.

10. A virus test method including:
preparing a pseudo-receptor film (13) having a plurality of pseudo-receptors arranged on the pseudo-receptor film, each of the pseudo-receptors mimicking a structure of a host-cell receptor, configured to bind specifically to a target virus;
after sucking down an air-under-test containing the target viruses, compressing the air-under-test into a high-speed air-flow of aerosols-under-test to concentrate the target viruses contained in the air-under-test, and to eject the high-speed air-flow to the pseudo-receptor film;
converting physical signals representing alterations of physical states of the pseudo-receptor film ascribable to specific bindings of the pseudo-receptors to the target viruses to electric signals, by using a signal conditioner; and
generating a cleaned dry-air;
generating a cleaned moist-air and supplying the cleaned moist-air to the pseudo-receptor film;
executing a difference-integral detection utilizing the electric signals to judge existences of the specific bindings of the pseudo-receptors to the target viruses,
wherein the physical signals represent variations of areal densities by weight of the pseudo-receptor film ascribable to the specific bindings of the pseudo-receptors with the target viruses, thereby the signal conditioner converts the physical signals to the electric signals,
wherein the pseudo-receptor film (13) is a sensitive film of a surface acoustic wave, the pseudo-receptor film is provided on at least a part in a surface of a piezoelectric-crystal sphere, and the surface acoustic wave propagates in the sensitive film,
wherein the signal conditioner is a sensor electrode of a SAW sensor (1003),
wherein the SAW sensor (1003) is a ball SAW sensor having the piezoelectric-crystal sphere, the pseudo-receptor film and the sensor electrode, the pseudo-receptor film (13) and the sensor electrode (12) being provided on at least the part in the surface of the piezoelectric-crystal sphere,
wherein the ball SAW sensor (1003) is housed in a detection vessel (10) constructing a hermetically closed space.

11. A virus test program causing the virus test system of claim 9 to execute a sequence of instructions, the program comprising:
instructions for sucking down an air-under-test containing the target viruses, compressing the air-under-test into a high-speed air-flow of aerosols-under-test to concentrate the target viruses contained in the air-under-test, and to eject the high-speed air-flow to a pseudo-receptor film, which merges a plurality of pseudo-receptors mimicking structures of host-cell receptors scheduled to be bound specifically to target viruses, wherein the pseudo-receptor film (13) is a sensitive film provided on at least a part in a surface of a piezoelectric-crystal sphere of a ball surface acoustic wave sensor;
instructions for causing a signal conditioner , being a sensor electrode of the ball surface acoustic wave sensor, to convert the physical signals, which represent alterations of physical states of the pseudo-receptor film ascribable to specific bindings of the pseudo-receptors to the target viruses, to electric signals, wherein the signals represent variations of areal densities by weight of the pseudo-receptor film ascribable to the specific bindings of the pseudo-receptors with the target viruses;
instructions for generating a cleaned dry-air;
instructions for generating a cleaned moist-air and supplying the cleaned moist-air to the pseudo-receptor film; and
instructions for causing an inspection module to execute a difference-integral detection by an arithmetic and logical operations utilizing the electric signals, for judging existences of the specific bindings of the pseudo-receptors to the target viruses.

## Patentansprüche

1. Virustestvorrichtung, umfassend:
einen Pseudorezeptorfilm (13), der eine Vielzahl von auf dem Pseudorezeptorfilm angeordneten Pseudorezeptoren aufweist, wobei jeder der Pseudorezeptoren (14) eine Struktur eines Wirtszellrezeptors nachahmt, der sich spezifisch an ein Zielvirus bindet;
ein Viruseinführungsrohr (A), das ausgebildet ist, um eine die Zielviren enthaltende Prüfluft nach unten zu saugen, die Prüfluft zu einem Hochgeschwindigkeitsluftstrom von Prüfaerosolen zu komprimieren, wobei die in der Prüfluft enthaltenen Zielviren konzentriert werden, und um den Hochgeschwindigkeitsluftstrom zum Pseudorezeptorfilm auszustoßen;
ein Trockenluft-Bypassrohr (C), das ausgebildet ist, um eine gereinigte Trockenluft zu erzeugen;
ein Feuchtlufteinführungsrohr (B), das ausgebildet ist, um eine gereinigte Feuchtluft zu erzeugen und die gereinigte Feuchtluft dem Pseudorezeptorfilm (13) zuzuführen;
einen Kugel-SAW-Sensor (1003), der eine Sensorelektrode als einen Signalaufbereiter einschließt, wobei der Signalaufbereiter ausgebildet ist, um physikalische Signale, die Änderungen physikalischer Zustände des Pseudorezeptorfilms darstellen, die spezifischen Bindungen der Pseudorezeptoren mit den Zielviren zuzuschreiben sind, in elektrische Signale umzuwandeln; und
ein Detektionsgefäß (10), das einen hermetisch geschlossenen Raum zum Aufnehmen des Kugel-SAW-Sensors (1003) bildet,
wobei die physikalischen Signale Variationen von Flächendichten nach Gewicht des Pseudorezeptorfilms darstellen, die den spezifischen Bindungen der Pseudorezeptoren mit den Zielviren zuzuschreiben sind, wodurch der Signalaufbereiter die physikalischen Signale in die elektrischen Signale umwandelt,
wobei der Pseudorezeptorfilm (13) ein für eine akustische Oberflächenwelle empfindlicher Film ist, der Pseudorezeptorfilm an mindestens einem Teil einer Oberfläche einer piezoelektrischen Kristallkugel bereitgestellt ist, und die akustische Oberflächenwelle sich in dem empfindlichen Film ausbreitet,
wobei der Kugel-SAW-Sensor (1003) die piezoelektrische Kristallkugel und den Pseudorezeptorfilm (13) und die Sensorelektrode aufweist, die an mindestens dem Teil in der Oberfläche der piezoelektrischen Kristallkugel bereitgestellt sind.

2. Virustestvorrichtung nach Anspruch 1, wobei das Viruseinführungsrohr (A) einen an einer Eintrittsseite davon angeordneten Filter (30a) und einen an einer Austrittsseite davon angeordneten Konzentrationsmechanismus (34) mit einer sich verjüngenden Düsenstruktur aufweist,
wobei eine Position einer Spitze des Konzentrationsmechanismus (34) mit einem Einlasskanal zusammenpasst, der in eine Außenwand des Detektionsgefäßes eingeschnitten ist, und der Hochgeschwindigkeitsluftstrom von der Spitze zum Pseudorezeptorfilm (13) ausgestoßen wird.

3. Virustestvorrichtung nach Anspruch 2, wobei das Feuchtlufteinführungsrohr (B) einen Weg zum Einführen der gereinigten Feuchtluft in ein Inneres des Detektionsgefäßes (10) einschließt, und
das Trockenluft-Bypassrohr (C) einen Weg zum Einführen der gereinigten Trockenluft in das Innere des Detektionsgefäßes (10) einschließt.

4. Virustestvorrichtung nach Anspruch 3, wobei der Konzentrationsmechanismus (34) des Viruseinführungsrohres (A) als dedizierter Virus-Konzentrationsmechanismus (34) für die Zielviren zugewiesen ist, wobei die Virustestvorrichtung weiter umfasst:
einen Feuchtluft-Konzentrationsmechanismus (34c) mit einer sich verjüngenden Düsenstruktur an einer Austrittsseite des Feuchtlufteinführungsrohres; und
einen Trockenluft-Konzentrationsmechanismus (34v) mit einer sich verjüngenden Düsenstruktur an einer Austrittsseite des Trockenluft-Bypassrohres,
wobei sich das Detektionsgefäß (10) jeweils relativ zu dem Virus-Konzentrationsmechanismus, dem Feuchtluft-Konzentrationsmechanismus (34c) und dem Trockenluft-Konzentrationsmechanismus (34v) bewegt, sodass jede der Spitzen des Virus-Konzentrationsmechanismus, des Feuchtluft-Konzentrationsmechanismus (34c) und des Trockenluft-Konzentrationsmechanismus (34v) jeweils mit dem Einlasskanal zusammenpassen kann.

5. Virustestvorrichtung nach Anspruch 3, wobei der Konzentrationsmechanismus (34) als ein gemeinsamer Konzentrationsmechanismus (34) dient,
eine Austrittsseite des Feuchtlufteinführungsrohres (B) über ein Verzweigungsrohr des Viruseinführungsrohres (A) mit dem Viruseinführungsrohr (A) verbunden ist, wobei das Verzweigungsrohr an einer Zwischenposition zwischen dem gemeinsamen Konzentrationsmechanismus (34) und einem in dem Viruseinführungsrohr (A) bereitgestellten Ein-Aus-Ventil angeordnet ist,
eine Austrittsseite des Trockenluft-Bypassrohres (C) durch ein Befeuchtungsausgangsventil hindurch mit dem Feuchtlufteinführungsrohr (B) verbunden ist, sodass das Trockenluft-Bypassrohr als ein Verzweigungsrohr des Feuchtlufteinführungsrohres dient, wobei das Befeuchtungsausgangsventil an einer Zwischenposition in dem Feuchtlufteinführungsrohr bereitgestellt ist, und
die Zielviren, die gereinigte Feuchtluft und die gereinigte Trockenluft jeweils nacheinander zu unterschiedlichen Zeitpunkten von dem gemeinsamen Konzentrationsmechanismus zum Pseudorezeptorfilm (13) durch Umschalten der Rohrleitungswege über Betätigungen des Ein-Aus-Ventils und des Befeuchtungsausgangsventils ausgestoßen werden.

6. Virustestvorrichtung nach Anspruch 3, weiter umfassend:
ein erstes Dreiwegeventil, das erste, zweite und dritte Ventilanschlüsse aufweist;
ein zweites Dreiwegeventil, das erste, zweite und dritte Ventilanschlüsse aufweist;
ein Kalibrierungsrohr, durch das Kalibrieraerosole, die Viren mit einer vorgeschriebenen Konzentration enthalten, hindurchfließen, das mit dem ersten Ventilanschluss des ersten Dreiwegeventils verbunden ist;
wobei der zweite Ventilanschluss des ersten Dreiwegeventils mit dem Viruseinführungsrohr, durch das die Prüfluft hindurchfließt, verbunden ist,
der dritte Ventilanschluss des ersten Dreiwegeventils über den ersten gemeinsamen Konzentrationsmechanismus mit dem Detektionsgefäß verbunden ist,
die Kalibrieraerosole und die Prüfluft jeweils zu unterschiedlichen Zeitpunkten durch Umschalten der Rohrleitungswege über das erste Dreiwegeventil durch den ersten gemeinsamen Konzentrationsmechanismus zu dem Pseudorezeptorfilm ausgestoßen werden,
das Feuchtlufteinführungsrohr (B) mit dem ersten Ventilanschluss des zweiten Dreiwegeventils verbunden ist und das Trockenluft-Bypassrohr mit dem zweiten Ventilanschluss des zweiten Dreiwegeventils verbunden ist,
der dritte Ventilanschluss des zweiten Dreiwegeventils mit einem Spülgaseinführungsrohr (D) verbunden ist, welches das Spülgas in das Detektionsgefäß einführt,
eine Spitze eines zweiten gemeinsamen Konzentrationsmechanismus, der an einem Austrittsende des Spülgaseinführungsrohres (D) bereitgestellt ist, mit einem zweiten Einlasskanal zusammenpasst, der an einem anderen Abschnitt der Außenwand des Detektionsgefäßes (10) eingeschnitten ist,
sodass die gereinigte Feuchtluft, das Spülgas und die gereinigte Trockenluft jeweils zu unterschiedlichen Zeitpunkten durch Umschalten der Rohrleitungswege über das zweite Dreiwegeventil von dem zweiten gemeinsamen Konzentrationsmechanismus zum Pseudorezeptorfilm ausgestoßen werden.

7. Virustestvorrichtung nach Anspruch 3, weiter umfassend:
ein Dreiwegeventil, das erste, zweite und dritte Ventilanschlüsse aufweist;
ein Spülgaseinführungsrohr (D), das mit dem dritten Ventilanschluss verbunden ist, das ausgebildet ist, um einen Weg zum Einführen eines Spülgases in das Detektionsgefäß (10) zu definieren; und
eine Vielzahl getrennter Konzentrationsmechanismen, wobei die getrennten Konzentrationsmechanismen in ein einzelnes Eintrittsrohr reduziert sind, das mit einem Austrittsende des Spülgaseinführungsrohres (D) verbunden ist, wobei der getrennte Konzentrationsmechanismus an anderen Abschnitten der Außenwand des Detektionsgefäßes (10) als einem Abschnitt angeordnet ist, an dem der Einlasskanal eingeschnitten ist,
wobei das Feuchtlufteinführungsrohr (B) mit dem ersten Ventilanschluss verbunden ist und das Trockenluft-Bypassrohr mit dem zweiten Ventilanschluss verbunden ist, sodass die gereinigte Feuchtluft, das Spülgas und die gereinigte Trockenluft jeweils zu unterschiedlichen Zeitpunkten durch Umschalten der Rohrleitungswege mittels des Dreiwegeventils von den getrennten Konzentrationsmechanismen zu dem Pseudorezeptorfilm (13) ausgestoßen werden, und
eine Gasrichtung, entlang derer Spülgase von den Konzentrationsmechanismen ausgestoßen werden, innerhalb eines Winkels zwischen 20 Grad oder mehr und 90 Grad oder weniger, von einer Längsachsenrichtung des Pseudorezeptorfilms (13) gemessen, gesteuert wird.

8. Virustestvorrichtung nach Anspruch 1, wobei das Detektionsgefäß (10) einschließt:
einen Körper des Detektionsgefäßes, und
einen Aufnehmer (1001) zum Montieren eines betreffenden Kugel-SAW-Sensors und eines Ersatz-Kugel-SAW-Sensors, wobei ein begrenzter Teil des Aufnehmers (1001) von einem Boden des Körpers des Detektionsgefäßes (10) nach oben lösbar eingesetzt ist, sodass der betreffende Kugel-SAW-Sensor (1003) oder der Ersatz-Kugel-SAW-Sensor selektiv in den Körper des Detektionsgefäßes (10) eingesetzt ist,
wobei der Ersatz-Kugel-SAW-Sensor eine gleiche Struktur und eine gleiche Größe wie der betreffende Kugel-SAW-Sensor (1003) aufweist, wobei der betreffende Kugel-SAW-Sensor durch Bewegen des Aufnehmers (1001) relativ zum Körper des Detektionsgefäßes (10) durch den Ersatz-Kugel-SAW-Sensor ersetzt wird.

9. Virustestsystem, umfassend:
die Virustestvorrichtung nach Anspruch 1; und
einen Signalprozessor (50), der ausgebildet ist, um den Signalaufbereiter anzusteuern, um eine Differenz-Integral-Detektion auf der Grundlage von Ausgabedaten des Signalaufbereiters zum Detektieren der Existenz der spezifischen Bindungen der Pseudorezeptoren (14) und den Zielviren auszuführen.

10. Virustestverfahren, einschließend:
Vorbereiten eines Pseudorezeptorfilms (13), der eine Vielzahl von auf dem Pseudorezeptorfilm angeordneten Pseudorezeptoren aufweist, wobei jeder der Pseudorezeptoren eine Struktur eines Wirtszellrezeptors nachahmt, der ausgeführt ist, um sich spezifisch an ein Zielvirus zu binden;
nach dem Ansaugen der die Zielviren enthaltenden Prüfluft, Komprimieren der Prüfluft zu einem Hochgeschwindigkeitsluftstrom von Prüfaerosolen, um die in der Prüfluft enthaltenen Zielviren zu konzentrieren, und um den Hochgeschwindigkeitsluftstrom zum Pseudorezeptorfilm auszustoßen;
Umwandeln physikalischer Signale, die Änderungen physikalischer Zustände des Pseudorezeptorfilms darstellen, die den spezifischen Bindungen der Pseudorezeptoren an die Zielviren zuzuschreiben sind, in elektrische Signale unter Verwendung eines Signalaufbereiters; und
Erzeugen einer gereinigten Trockenluft;
Erzeugen einer gereinigten Feuchtluft und Zuführen der gereinigten Feuchtluft zu dem Pseudorezeptorfilm;
Ausführen einer Differenz-Integral-Detektion unter Verwendung der elektrischen Signale, um das Existieren der spezifischen Bindungen der Pseudorezeptoren an die Zielviren zu beurteilen,
wobei die physikalischen Signale Variationen von Flächendichten nach Gewicht des Pseudorezeptorfilms darstellen, die den spezifischen Bindungen der Pseudorezeptoren mit den Zielviren zuzuschreiben sind, wodurch der Signalaufbereiter die physikalischen Signale in die elektrischen Signale umwandelt,
wobei der Pseudorezeptorfilm (13) ein für eine akustische Oberflächenwelle empfindlicher Film ist, der Pseudorezeptorfilm an mindestens einem Teil einer Oberfläche einer piezoelektrischen Kristallkugel bereitgestellt ist, und die akustische Oberflächenwelle sich in dem empfindlichen Film ausbreitet,
wobei der Signalaufbereiter eine Sensorelektrode eines SAW-Sensors (1003) ist,
wobei der SAW-Sensor (1003) ein Kugel-SAW-Sensor ist, der die piezoelektrische Kristallkugel, den Pseudorezeptorfilm und die Sensorelektrode aufweist, wobei der Pseudorezeptorfilm (13) und die Sensorelektrode (12) an mindestens dem Teil in der Oberfläche der piezoelektrischen Kristallkugel bereitgestellt sind,
wobei der Kugel-SAW-Sensor (1003) in einem Detektionsgefäß (10) aufgenommen ist, das einen hermetisch geschlossenen Raum bildet.

11. Virustestprogramm, welches das Virustestsystem nach Anspruch 9 veranlasst, eine Folge von Anweisungen auszuführen, wobei das Programm umfasst:
Anweisungen zum Ansaugen einer die Zielviren enthaltenden Prüfluft, zum Komprimieren der Prüfluft zu einem Hochgeschwindigkeitsluftstrom von Prüfaerosolen, um die in der Prüfluft enthaltenen Zielviren zu konzentrieren, und um den Hochgeschwindigkeitsluftstrom zu einem Pseudorezeptorfilm auszustoßen, der eine Vielzahl von Pseudorezeptoren vereinigt, die Strukturen von Wirtszellrezeptoren nachahmen, die vorgesehen sind, sich spezifisch an Zielviren zu binden, wobei der Pseudorezeptorfilm (13) ein empfindlicher Film ist, der an mindestens einem Teil einer Oberfläche einer piezoelektrischen Kristallkugel eines Kugel-SAW-Sensors bereitgestellt ist;
Anweisungen zum Veranlassen eines Signalaufbereiters, der eine Sensorelektrode des Kugel-SAW-Sensors ist, um die physikalischen Signale, die Änderungen physikalischer Zustände des Pseudorezeptorfilms darstellen, die den spezifischen Bindungen der Pseudorezeptoren an die Zielviren zuzuschreiben sind, in elektrische Signale umzuwandeln, wobei die Signale Variationen von Flächendichten nach Gewicht des Pseudorezeptorfilms darstellen, die den spezifischen Bindungen der Pseudorezeptoren mit den Zielviren zuzuschreiben sind;
Anweisungen zum Erzeugen einer gereinigten Trockenluft;
Anweisungen zum Erzeugen einer gereinigten Feuchtluft und Zuführen der gereinigten Feuchtluft zum Pseudorezeptorfilm; und
Anweisungen zum Veranlassen eines Prüfmoduls, eine Differenz-Integral-Detektion durch arithmetische und logische Operationen unter Verwendung der elektrischen Signale zum Beurteilen der Existenz der spezifischen Bindungen der Pseudorezeptoren an die Zielviren auszuführen.

## Revendications

1. Dispositif de test de virus comprenant :
un film (13) pseudo-récepteur présentant une pluralité de pseudo-récepteurs agencés sur le film pseudo-récepteur, chacun des pseudo-récepteurs (14) imitant une structure d'un récepteur de cellule hôte, qui se lie spécifiquement à un virus cible ;
un tube (A) d'introduction de virus, configuré pour aspirer un air à tester contenant les virus cibles, pour comprimer l'air à tester en un flux d'air à grande vitesse d'aérosols à tester, concentrant les virus cibles contenus dans l'air à tester, et pour éjecter le flux d'air à grande vitesse vers le film pseudo-récepteur ;
un tube (C) de dérivation d'air sec configuré pour générer un air sec nettoyé ;
un tube (B) d'introduction d'air humide configuré pour générer un air humide nettoyé et fournir l'air humide nettoyé au film (13) pseudo-récepteur ;
un capteur (1003) SAW à bille incluant une électrode de capteur en tant que conditionneur de signaux, le conditionneur de signaux étant configuré pour convertir des signaux physiques, qui représentent des altérations d'états physiques du film pseudo-récepteur attribuables à des liaisons spécifiques des pseudo-récepteurs avec les virus cibles, en signaux électriques ; et
un récipient (10) de détection construisant un espace hermétiquement fermé permettant de loger le capteur (1003) SAW à bille,
dans lequel les signaux physiques représentent des variations de densités surfaciques en poids du film pseudo-récepteur attribuables aux liaisons spécifiques des pseudo-récepteurs avec les virus cibles, ce par quoi le conditionneur de signaux convertit les signaux physiques en signaux électriques,
dans lequel le film (13) pseudo-récepteur est un film sensible d'une onde acoustique de surface, le film pseudo-récepteur est fourni sur au moins une partie dans une surface d'une sphère à cristaux piézoélectriques, et l'onde acoustique de surface se propage dans le film sensible,
dans lequel le capteur (1003) SAW à bille présente la sphère à cristaux piézoélectriques, et le film (13) pseudo-récepteur et l'électrode de capteur qui sont fournis sur au moins la partie dans la surface de la sphère à cristaux piézoélectriques.

2. Dispositif de test de virus selon la revendication 1, dans lequel le tube (A) d'introduction de virus présente un filtre (30a) disposé au niveau d'un côté d'entrée de celui-ci et un mécanisme (34) de concentration avec une structure de buse conique disposé au niveau d'un côté de sortie de celui-ci,
dans lequel, une position d'une pointe du mécanisme (34) de concentration s'accouple avec un canal d'entrée, qui est découpé dans une paroi extérieure du récipient de détection, et le flux d'air à grande vitesse est éjecté vers le film (13) pseudo-récepteur à partir de la pointe.

3. Dispositif de test de virus selon la revendication 2, dans lequel le tube (B) d'introduction d'air humide inclut un trajet d'introduction de l'air humide nettoyé vers un intérieur du récipient (10) de détection, et
le tube (C) de dérivation d'air sec inclut un trajet d'introduction de l'air sec nettoyé vers l'intérieur du récipient (10) de détection.

4. Dispositif de test de virus selon la revendication 3, dans lequel le mécanisme (34) de concentration du tube (A) d'introduction de virus est affecté en tant que mécanisme (34) de concentration de virus dédié pour les virus cibles, le dispositif de test de virus comprenant en outre :
un mécanisme (34c) de concentration d'air humide avec une structure de buse conique au niveau d'un côté de sortie du tube d'introduction d'air humide ; et
un mécanisme (34v) de concentration d'air sec avec une structure de buse conique au niveau d'un côté de sortie du tube de dérivation d'air sec,
dans lequel, le récipient (10) de détection se déplace relativement au mécanisme de concentration de virus, au mécanisme (34c) de concentration d'air humide et au mécanisme (34v) de concentration d'air sec, respectivement, de sorte que chacune des pointes du mécanisme de concentration de virus, du mécanisme (34c) de concentration d'air humide et du mécanisme (34v) de concentration d'air sec puisse s'accoupler avec le canal d'entrée, respectivement.

5. Dispositif de test de virus selon la revendication 3, dans lequel le mécanisme (34) de concentration sert de mécanisme (34) de concentration commun,
un côté de sortie du tube (B) d'introduction d'air humide est relié au tube (A) d'introduction de virus par l'intermédiaire d'un tube ramifié du tube (A) d'introduction de virus, le tube ramifié étant affecté au niveau d'une position intermédiaire entre le mécanisme (34) de concentration commun et une vanne marche-arrêt fournie dans le tube (A) d'introduction de virus,
un côté de sortie du tube (C) de dérivation d'air sec est relié au tube (B) d'introduction d'air humide à travers une vanne de sortie d'humidification de sorte que le tube de dérivation d'air sec serve de tube ramifié du tube d'introduction d'air humide, la vanne de sortie d'humidification étant fournie au niveau d'une position intermédiaire dans le tube d'introduction d'air humide, et
les virus cibles, l'air humide nettoyé et l'air sec nettoyé sont séquentiellement éjectés vers le film (13) pseudo-récepteur à partir du mécanisme de concentration commun à des moments différents, respectivement, par commutation de trajets de tuyauterie, par l'intermédiaire d'opérations de la vanne marche-arrêt et de la vanne de sortie d'humidification.

6. Dispositif de test de virus selon la revendication 3, comprenant en outre :
une première vanne à trois voies présentant des premier, deuxième et troisième orifices de vanne ;
une deuxième vanne à trois voies présentant des premier, deuxième et troisième orifices de vanne ;
un tube d'étalonnage, à travers lequel des aérosols d'étalonnage contenant des virus avec une concentration prescrite s'écoulent, relié au premier orifice de vanne de la première vanne à trois voies ;
dans lequel, le deuxième orifice de vanne de la première vanne à trois voies est relié au tube d'introduction de virus, à travers lequel l'air à tester s'écoule,
le troisième orifice de vanne de la première vanne à trois voies est relié au récipient de détection par l'intermédiaire du premier mécanisme de concentration commun,
les aérosols d'étalonnage et l'air à tester sont éjectés vers le film pseudo-récepteur à travers le premier mécanisme de concentration commun à des moments différents, respectivement, par commutation de trajets de tuyauterie par l'intermédiaire de la première vanne à trois voies,
le tube (B) d'introduction d'air humide est relié au premier orifice de vanne de la deuxième vanne à trois voies, et le tube de dérivation d'air sec est relié au deuxième orifice de vanne de la deuxième vanne à trois voies,
le troisième orifice de vanne de la deuxième vanne à trois voies est relié à un tube (D) d'introduction de gaz de purge qui introduit le gaz de purge dans le récipient de détection,
une pointe d'un deuxième mécanisme de concentration commun, qui est fourni au niveau d'une extrémité de sortie du tube (D) d'introduction de gaz de purge, s'accouple avec un deuxième canal d'entrée, qui est découpé au niveau d'une autre portion de la paroi extérieure du récipient (10) de détection,
de sorte que l'air humide nettoyé, le gaz de purge et l'air sec nettoyé soient éjectés vers le film pseudo-récepteur à partir du deuxième mécanisme de concentration commun à des moments différents, respectivement, par commutation de trajets de tuyauterie par l'intermédiaire de la deuxième vanne à trois voies.

7. Dispositif de test de virus selon la revendication 3, comprenant en outre :
une vanne à trois voies présentant des premier, deuxième et troisième orifices de vanne ;
un tube (D) d'introduction de gaz de purge relié au troisième orifice de vanne, configuré pour définir un trajet permettant d'introduire un gaz de purge dans le récipient (10) de détection ; et
une pluralité de mécanismes de concentration séparés, les mécanismes de concentration séparés étant réduits en un unique tube d'entrée relié à une extrémité de sortie du tube (D) d'introduction de gaz de purge, le mécanisme de concentration séparé étant disposé au niveau d'autres portions de la paroi extérieure du récipient (10) de détection qu'une portion où le canal d'entrée est découpé,
dans lequel, le tube (B) d'introduction d'air humide est relié au premier orifice de vanne, et le tube de dérivation d'air sec est relié au deuxième orifice de vanne de sorte que l'air humide nettoyé, le gaz de purge et l'air sec nettoyé soient éjectés vers le film (13) pseudo-récepteur à partir des mécanismes de concentration séparés à des moments différents, respectivement par commutation de trajets de tuyauterie par la vanne à trois voies, et
une direction de gaz, le long de laquelle des gaz de purge sont éjectés à partir des mécanismes de concentration, est commandée à l'intérieur d'un angle entre 20 degrés ou plus et 90 degrés ou moins, mesuré à partir d'une direction d'axe long du film (13) pseudo-récepteur.

8. Dispositif de test de virus selon la revendication 1, dans lequel le récipient (10) de détection inclut :
un corps de récipient de détection, et
un suscepteur (1001) permettant de monter un capteur SAW à bille sujet et un capteur SAW à bille remplaçant, une partie limitée du suscepteur (1001) étant insérée de manière détachable vers le haut à partir d'un fond du corps du récipient (10) de détection, de sorte que le capteur (1003) SAW à bille sujet ou le capteur SAW à bille remplaçant soit inséré sélectivement dans le corps du récipient (10) de détection,
dans lequel, le capteur SAW à bille remplaçant présente la même structure et la même taille que le capteur (1003) SAW à bille sujet, le capteur SAW à bille sujet étant remplacé par le capteur SAW à bille remplaçant par déplacement du suscepteur (1001) relativement au corps du récipient (10) de détection.

9. Système de test de virus comprenant :
le dispositif de test de virus selon la revendication 1 ; et
un processeur (50) de signaux, configuré pour entraîner le conditionneur de signaux, pour exécuter une détection d'intégrale différentielle sur la base d'une donnée de sortie provenant du conditionneur de signaux permettant de détecter les existences de liaisons spécifiques des pseudo-récepteurs (14) et des virus cibles.

10. Procédé de test de virus incluant :
la préparation d'un film (13) pseudo-récepteur présentant une pluralité de pseudo-récepteurs agencés sur le film pseudo-récepteur, chacun des pseudo-récepteurs imitant une structure d'un récepteur de cellule hôte, configuré pour se lier spécifiquement à un virus cible ;
après aspiration d'un air à tester contenant les virus cibles, la compression de l'air à tester en un flux d'air à grande vitesse d'aérosols à tester pour concentrer les virus cibles contenus dans l'air à tester, et pour éjecter le flux d'air à grande vitesse vers le film pseudo-récepteur ;
la conversion de signaux physiques représentant des altérations d'états physiques du film pseudo-récepteur attribuables à des liaisons spécifiques des pseudo-récepteurs aux virus cibles en signaux électriques, par utilisation d'un conditionneur de signaux ; et
la génération d'un air sec nettoyé ;
la génération d'un air humide nettoyé et la fourniture de l'air humide nettoyé au film pseudo-récepteur ;
l'exécution d'une détection d'intégrale différentielle utilisant les signaux électriques pour juger des existences des liaisons spécifiques des pseudo-récepteurs aux virus cibles,
dans lequel les signaux physiques représentent des variations de densités surfaciques en poids du film pseudo-récepteur attribuables aux liaisons spécifiques des pseudo-récepteurs avec les virus cibles, ce par quoi le conditionneur de signaux convertit les signaux physiques en signaux électriques,
dans lequel le film (13) pseudo-récepteur est un film sensible d'une onde acoustique de surface, le film pseudo-récepteur est fourni sur au moins une partie dans une surface d'une sphère à cristaux piézoélectriques, et l'onde acoustique de surface se propage dans le film sensible,
dans lequel le conditionneur de signaux est une électrode de capteur d'un capteur (1003) SAW,
dans lequel le capteur (1003) SAW est un capteur SAW à bille présentant la sphère à cristaux piézoélectriques, le film pseudo-récepteur et l'électrode de capteur, le film (13) pseudo-récepteur et l'électrode (12) de capteur étant fournis sur au moins la partie dans la surface de la sphère à cristaux piézoélectriques,
dans lequel le capteur (1003) SAW à bille est logé dans un récipient (10) de détection construisant un espace hermétiquement fermé.

11. Programme de test de virus amenant le système de test de virus selon la revendication 9 à exécuter une séquence d'instructions, le programme comprenant :
des instructions permettant d'aspirer un air à tester contenant les virus cibles, de comprimer l'air à tester en un flux d'air à grande vitesse d'aérosols à tester de manière à concentrer les virus cibles contenus dans l'air à tester, et à éjecter le flux d'air à grande vitesse vers un film pseudo-récepteur, ce qui fusionne une pluralité de pseudo-récepteurs imitant des structures de récepteurs de cellule hôte prévus pour être liés spécifiquement à des virus cibles, dans lequel le film (13) pseudo-récepteur est un film sensible fourni sur au moins une partie dans une surface d'une sphère à cristaux piézoélectriques d'un capteur à onde acoustique de surface à bille ;
des instructions permettant d'amener un conditionneur de signaux, étant une électrode de capteur du capteur à onde acoustique de surface à bille, à convertir les signaux physiques, qui représentent des altérations d'états physiques du film pseudo-récepteur attribuables à des liaisons spécifiques des pseudo-récepteurs aux virus cibles, en signaux électriques, dans lequel les signaux représentent des variations de densités surfaciques en poids du film pseudo-récepteur attribuables aux liaisons spécifiques des pseudo-récepteurs avec les virus cibles ;
des instructions permettant de générer un air sec nettoyé ;
des instructions permettant de générer un air humide nettoyé et de fournir l'air humide nettoyé au film pseudo-récepteur ; et
des instructions permettant d'amener un module d'inspection à exécuter une détection d'intégrale différentielle par des opérations arithmétiques et logiques utilisant les signaux électriques, pour juger des existences des liaisons spécifiques des pseudo-récepteurs aux virus cibles.
